# EUROPEAN PATENT APPLICATION

(11) **EP 4 293 040 A1**
(43) Date of publication of application: **20.12.2023**
(21) Application number: 22179778.0
(22) Date of filing: 19.06.2022
(51) Int. Cl.: C07K 14/715, C12N 15/63, C12N 15/85

(54) **CELL LINE FOR ENGINEERING CYTOKINE RECEPTORS**

(71) Applicant: ETH Zurich, 8092 Zürich (CH)
(72) Inventor: Kucharczyk, Jakub, 8092 Zürich (CH); Reddy, Sai Tota, 8092 Zürich (CH); Vasquez-Lombardi, Rodrigo, 8092 Zürich (CH); Lichtenstein, Tamara Paula, 8092 Zürich (CH); Gao, Beichen, 8092 Zürich (CH)
(74) Representative: Vossius & Partner Patentanwälte Rechtsanwälte mbB

(57) **Abstract**

The present invention relates to a cell line for identifying engineered common gamma chain (y_{c}) receptors, the cell line comprising: (a) a reporter system comprising a polynucleotide encoding a fluorescent marker, wherein the polynucleotide encoding the fluorescent marker is transcriptionally linked to one or more STAT response elements; (b) at least one polynucleotide encoding a STAT protein, wherein the STAT protein activates the STAT response element comprised in the reporter system; and (c) at least one polynucleotide encoding a JAK protein; wherein the endogenous gene encoding the common gamma chain is inactivated in said cell line. Moreover, the present invention relates to methods of using the cell line according to the invention as a screening platform.

## Description

### BACKGROUND INFORMATION

The clinical success of immunotherapies harnessing the specificity and antitumor activity of T cells has recently established them as a new pillar of cancer treatment. In particular, the use of monoclonal antibodies that target the immune checkpoint receptors CTLA-4 and PD-1, and the PD-L1 inhibitory ligand, has catalysed a paradigm shift in the treatment and management of patients suffering from several types of advanced malignancies, such as melanoma, lung and renal cancers; showing unprecedented improvements in objective response rates and overall survival. This therapeutic strategy, also known as immune checkpoint blockade (ICB), leads to systemic T cell activation and ultimately aims to harness the activity of tumour-specific T cells (including circulating and tumour-infiltrating T cells) to mount an antitumour response. There are currently seven approved monoclonal antibody drugs targeting immune checkpoints across more than nineteen cancer indications. Despite their success and now well-established clinical use, there is still a substantial proportion of patients that do not respond to ICB due to several challenges including poor tumour infiltration by T cells, low tumour mutational burden, antigen escape, T cell exhaustion and the presence of immunosuppressive components in the tumour microenvironment. An immunotherapy strategy that aims to circumvent several of these limitations is the use of genetically engineered T cells. In this context, the regulatory approval of five chimeric antigen receptor (CAR)-T cell therapies in the past four years represents a turning point in the field of cancer immunotherapy. These first-in-class drugs rely on the isolation, genetic modification and re-infusion of autologous patient T cells. A CAR is a synthetic receptor with an antibody binding domain engineered to recognize cancer antigens fused to a signalling domain that triggers T cell activation, thus enabling T cells to recognize and destroy malignant cells. Clinical application of CAR-T cells has shown a remarkable efficacy for various leukemias and lymphomas: CAR-T cells targeting CD19 on malignant B cells have resulted in complete remission rates of up to 90% in relapsed and treatment-refractory acute lymphocytic leukaemia (ALL).

While CAR-T cells offer an effective therapeutic strategy for certain cancers, they are still limited in that they require surface expression of a well-defined antigen present only on tumour cells and not on healthy tissue in order to avoid on-target off-tumour toxicity. Furthermore, despite their great success against liquid tumours, clinical evaluation of CAR-T cell therapy against solid tumours has shown disappointing efficacy thus far. By contrast, an alternative T cell therapy strategy relying on the genetic reprogramming of patient T cells with tumour-targeting T cell receptors (i.e., TCR-T cell therapy) has shown clinical efficacy against solid tumours in multiple trials. Notably, TCR-T cell therapies can be directed against not only surface proteins (a requirement for CAR-T cells) but also towards processed intracellular targets displayed by major histocompatibility complexes (MHC), thus vastly increasing the targetable antigen pool. Despite their promise, TCR-T cells suffer from their own set of challenges including a predisposition for TCR cross-reactivity; as well as overall response rates well below 50% for most types of tumours.

Significant efforts are being directed at improving the overall safety and efficacy of both CAR-T and TCR-T approaches. In addition to their own particular safety challenges, both approaches can be limited in their application by the common occurrence of cytokine release syndrome (CRS) post-infusion, an adverse event resulting from excessive activation of engineered T cells that can lead to life-threatening complications. As such, several strategies aiming to improve engineered T cell safety are currently under development, including the use of engineered safety switches, conditional T cell activation systems and synthetic cytokine signalling systems. In terms of efficacy, strategies for enhancing the potency of both TCR-T and CAR-T cells against solid tumours include the use of engineered chimeric switch receptors, the development of antigen-induced cytokine secretion systems, overexpression of activating co-receptors and the suppression of inhibitory receptors. Furthermore, manufacturing approaches that aim to shift the phenotypes of engineered T cell products away from effector memory phenotypes (TEM) and towards longer-lived stem-cell memory (TSCM) and central memory (TCM) phenotypes are actively being developed in order to promote increased T cell persistence post-infusion, as recently reported in the context of CAR-T cell therapy. Modulation of cytokine signalling has emerged as a key strategy for addressing important aspects of engineered T cell safety, efficacy and manufacturing, thus highlighting the crucial role of cytokines in T cell biology and the potential for cytokine and cytokine receptor engineering for the development of next-generation T cell therapies with enhanced therapeutic properties.

Cytokines are a group of small proteins primarily secreted by immune cells that play a central role in regulating the immune system and pathophysiological processes. Due to their small molecular size, cytokines are characterised by a short serum half-life. Furthermore, most cytokines display pleiotropic effects, meaning that a single cytokine can act on several different cell types. Cytokines are crucial for the homeostatic proliferation and survival of immune cells, as well as for orchestrating immune responses during infection, inflammation and tumorigenesis. Cytokines act by binding to their cognate cell surface receptors to induce receptor dimerization and cell membrane reorganisation which in turn trigger signalling events that drive key cellular processes including activation, proliferation, differentiation and apoptosis. Due to their central role in regulating immunity, a number of cytokines have been approved for therapeutic use, namely interleukin-2 (IL-2), interleukin-11 (IL-11), interferon alpha (IFN-α), interferon beta (IFN-β), interferon gamma (IFN-γ), tumour necrosis factor alpha (TNF-α), granulocyte-macrophage colony-stimulating factor (GM-CSF) and granulocyte colony-stimulating factor (G-CSF). Notably, the short-serum half-life and pleiotropy of unmodified cytokines limit their therapeutic potential, a prominent example being the common occurrence of vascular leak syndrome (VLS) following high-dose IL-2 treatment in cancer. As such, molecular engineering approaches have been applied to improve the therapeutic properties of cytokines, most notably for half-life prolongation by covalent linkage to polyethylene glycol (PEG) as is the case for the regulatory approved PEG-IFN-α (peginterferon alfa-2a), PEG-IFN-β (peginterferon beta-la) and PEG-G-CSF (pegfilgastrim). In addition to half-life prolongation, molecular engineering has been applied to address cytokine pleiotropy, as is the case for the multiple engineered IL-2 variants that have biassed specificity towards cytotoxic immune cells and are currently undergoing clinical development for cancer indications.

Common gamma chain (γ_{c}) cytokines are of particular importance for the activation and proliferation of T cells and other cytotoxic cell types (e.g., natural killer (NK) cells) playing crucial roles in antitumour immunity. The γ_{c} family of cytokines is composed of six members (i.e., IL-2, IL-4, IL-7, IL-9, IL-15 and IL-21) that share the γ_{c} subunit as part of their receptors for signalling. In addition to the γ_{c}, each of these cytokines incorporate additional signalling subunits into their receptors, namely IL-2Rβ (shared by IL-2 and IL-15), IL-7Rα, IL-4R, IL-9R and IL-21R, and in some cases a third high-affinity receptor subunit. The major signalling pathway of γ_{c} cytokines is the JAK-STAT pathway, in which binding of γ_{c} cytokines to non-γ_{c} receptor subunits triggers the recruitment of γ_{c}, leading to receptor assembly. Subsequently, Janus kinase (JAK) 1 and JAK3, which are associated with the intracellular regions of non-γ_{c} subunits and γ_{c}, respectively, come into close proximity and become activated via trans-phosphorylation. Activated JAK1/JAK3 in turn phosphorylate specific residues within the intracellular regions of both non-γ_{c} and γ_{c} subunits, thus creating docking sites for signal transducer and activator of transcription (STAT) factors. Notably, specific non-γ_{c} subunits favour the docking of particular STAT proteins, for example IL-2Rβ and IL-7Rα favouring STATS and IL-21R favouring STAT3 recruitment. After docking, STAT proteins are phosphorylated by JAK1/JAK3 at key tyrosine residues, triggering STAT dimerisation and translocation into the nucleus for transcriptional modulation of target genes regulating key cellular processes including differentiation, proliferation and apoptosis, with examples being STATS-driven transcriptional activation of genes encoding cyclin D2 (proliferation), IL-2Rα (positive feedback loop) and Fas ligand (pro-apoptotic) following IL-2 stimulation in T cells. Furthermore, cross-talk between the JAK-STAT signalling pathway and additional signalling pathways, most notably the MAPK and PI3K/AKT pathways, can further modulate the multiple cell differentiation, proliferation and survival cues elicited by specific γ_{c} cytokines.

Among the γ_{c} family of cytokines, IL-2, IL-7, IL-15 and IL-21 play distinct and sometimes overlapping roles in T cell biology that can be exploited for therapeutic purposes:
Interleukin 2. IL-2 is primarily produced by CD4+ T cells under steady-state conditions, and by both CD4+ and CD8+ T cells following stimulation by antigen. Activated dendritic cells (DCs) and mast cells can also secrete IL-2, though in lower quantities. The IL-2 receptor (IL-2R) can exist in a dimeric or trimeric form. The dimeric IL-2R is composed of IL-2Rβ and γ_{c} and shows intermediate affinity for IL-2 (K_{d} ~ 10⁻⁹ M). The trimeric high-affinity IL-2R is formed by IL-2Rα, IL-2Rβ and γ_{c} (trimeric receptor affinity: K_{d} ~ 10⁻¹¹ M). Notably, individual IL-2 receptor subunits can also bind IL-2 in isolation with a wide range of affinities, namely K_{d} ~ 10 nM for the high-affinity IL-2Rα and K_{d} ~ 450 nM for the moderate affinity IL-2Rβ and weak to undetectable affinity (K_{d} > 50 µM) for γ_{c}. The IL-2Rα does not possess an intracellular signalling domain, and thus does not contribute to signal transduction. Of note, trimeric IL-2 receptors are constitutively expressed on regulatory T cells (Tregs) and are only transiently expressed by activated conventional T cells, while the dimeric receptor is expressed on NK cells and memory CD8+ T cells. IL-2 has both immunostimulatory and immunosuppressive effects. On the one hand, it promotes expansion and survival of activated T cells and their differentiation into defined effector T cell subsets as well as differentiation of naive T cells. It also stimulates growth and differentiation of activated B cells, thereby facilitating immunoglobulin synthesis, as well as proliferation and function of natural killer (NK) cells. On the other hand, IL-2 stimulates the differentiation, growth, and survival of regulatory T cells (Tregs), and acts synergistically with TCR signalling to promote T cell apoptosis following antigen encounter through activation-induced cell death (AICD). Furthermore, IL-2 promotes CD8+ T cell exhaustion during chronic viral infection and cancer, which has motivated the recent development of IL-2 partial agonists as a strategy to maintain proliferative capacity while avoiding terminal exhaustion.
Interleukin-7. IL-7 is produced by non-hematopoietic mesenchymal and epithelial cells in secondary lymphoid organs and fulfils a key role in the homeostatic proliferation of naive and memory T cells. In addition to T cells, IL-7 exerts additional functions across several immune cell types including B cells, NK cells, monocytes/macrophages and innate lymphoid cells. In the context of engineered T cell manufacturing, it has been demonstrated that it is possible to differentiate naive precursors into TSCM following stimulation with antigen and culturing with a combination of IL-7 and IL-15. In addition to its crucial role in T cell homeostasis and application for promoting TSCM expansion in vitro, hyperactive IL-7 signalling has been implicated in the development and maintenance of haematological cancers, including B- and T-ALL. In this context, gain-of-function mutations in the L-7Rα gene leading to constitutive ligand-independent signalling or hypersensitive signalling, particularly in T-ALL, have been proposed as candidate mechanisms for malignant transformation. Indeed, while a recent approach using constitutively-active IL-7Rα homodimers successfully potentiated CAR-T efficacy in the preclinical setting, careful engineering of the IL-7/IL-7R axis is required to prevent uncontrolled proliferation of engineered T cell products (e.g., through non-constitutive attenuated signalling or incorporation of safety switch mechanisms).
Interleukin-15. IL-15 is produced primarily by innate immune cell subsets (e.g., activated DCs, monocytes) and shares functional and structural characteristics with IL-2. IL-2 and IL-15 share the IL-2Rβ and the γ_{c} receptor subunits, while having private alpha receptor subunits. Different to IL-2, however, IL-15 preferentially expands cytotoxic CD8+ T cells and NK cells over Tregs, protects T cells from AICD, and promotes CD8+ memory T cell persistence. These properties have motivated the clinical development of IL-15 and IL-15-IL-15Rα fusion proteins for cancer immunotherapy applications. In terms of engineered T cell manufacturing, the combination of IL-7 and IL-15 for ex vivo expansion has received particular attention in recent years, due to its ability to yield a higher proportion of TSCM compared to expansion in the presence of IL-2.
Interleukin-21. IL-21 is primarily produced by natural killer T (NKT) cells, T follicular helper cells (TFH) and TH17 cells. IL-21 exerts a range of pleiotropic effects on different immune cell subsets, including the enhancement of CD8+ cytotoxic T cell function in chronic infection and cancer, differentiation of CD4+ Th17 cells and their involvement in autoimmunity and the differentiation of germinal centre B cells required for mounting of effective humoral immune responses. Notably, IL-21 signalling in CD8+ T cells leads to contrasting effects to those of IL-2 signalling. While IL-2 promotes differentiation of memory CD8+ T cells into effector T cells, IL-21 inhibits this process and favours the expansion of TSCM phenotypes with an enhanced capability for self-renewal and multipotency leading to prolonged persistence and increased antitumor activity in mouse tumour models.

Optimisation of therapeutic cytokine pharmacokinetics and pharmacodynamics has been achieved through molecular engineering, with several engineered cytokines (most notably interleukin-2, IL-2) rapidly making their way into the clinic. Much like with soluble cytokines, engineering of cytokine receptors that display increased sensitivity, are fine-tuned for enhanced T cell activity or that respond only to specific ligands are all attractive strategies for the development of T cell products with enhanced function, potency and safety. In this regard, the development of synthetic orthogonal cytokine-receptor pairs has emerged as a potential strategy to exert control over infused engineered T cells co-expressing a targeting receptor (e.g., a transgenic CAR or TCR) and an IL-2Rβ receptor subunit mutant that is unable to be activated by wild-type IL-2, but that recognizes an engineered IL-2 variant (WO2017/044464, WO2021/146487). Furthermore, the development of chimeric switch receptors composed of an inhibitory ectodomain (e.g., PD-1, Fas) and intracellular domains based on co-stimulatory or cytokine receptors (e.g., 4-1BB) have been designed with the aim of converting T cell inhibitory signals into stimulatory ones in order to increase the potency of engineered T cell products (WO2020/180664, WO2018/170475). In addition to these strategies, the engineering of cytokine receptors with increased responsiveness and tuned signalling to T cell activating cytokines, such as IL-2, IL-7, IL-15 and IL-21, would be highly desirable as a means to enhance both the ex vivo manufacturing and in vivo antitumour activity of CAR-T and TCR-T cell products. In terms of manufacturing, enhanced responsiveness and tuned signalling to key cytokines could achieve a T cell product with a higher proportion of T cells with a TSCM phenotype, which can lead to a better persistence post-infusion and, by extension, increased efficacy. In terms of antitumor activity, preferential capture of key cytokines within the tumour microenvironment has the potential to both enhance tumour infiltration and to counteract the activity of immunosuppressive immune cell subsets by competition for available cytokines, a prominent example being IL-2 stimulation of Tregs.

Despite the advancements described above, there is still a need in the art for engineered γ_{c} cytokine receptors.

Thus, it is one objective of the present invention to provide a cellular platform for a functional screening of engineered γ_{c} cytokine receptors.

It is another objective of the present invention to provide methods for identifying engineered γ_{c} cytokine receptors having altered responsiveness to one or more cytokines.

Another objective of the present invention is to provide methods for identifying engineered γ_{c} cytokine receptors that are responsive to bio-orthogonal cytokines.

Furthermore, it is an objective of the present invention to provide methods for identifying chimeric receptors comprising a cytoplasmic portion of a common gamma chain receptor.

It is an additional objective of the present invention to provide lymphocytes comprising an engineered common gamma chain receptor.

These and other objectives are achieved by the independent claims of the present invention. The dependent claims are related to specific embodiments.

### SUMMARY OF THE INVENTION

The present invention is characterized in the herein provided embodiments and claims. In particular, the present invention relates, *inter alia,* to the following embodiments:
1. A cell line for identifying engineered common gamma chain (γ_{c}) receptors, the cell line comprising:
   a) a reporter system comprising a polynucleotide encoding a fluorescent marker, wherein the polynucleotide encoding the fluorescent marker is transcriptionally linked to one or more STAT response elements;
   b) at least one polynucleotide encoding a STAT protein, wherein the STAT protein activates the STAT response element comprised in the reporter system; and
   c) at least one polynucleotide encoding a JAK protein;
   wherein said cell line does not encode a functional common gamma chain.
2. The cell line according to embodiment 1, wherein the cell line further comprises one or more polynucleotides encoding IL2Rα and IL2Rβ.
3. The cell line according to embodiment 1 or 2, wherein the cell line further comprises a polynucleotide encoding IL7Rα.
4. The cell line according to any one of embodiments 1 to 3, wherein the cell line further comprises one or more polynucleotides encoding IL4Rα, IL9Rα, IL15Rα and/or IL21Rα.
5. The cell line according to any one of embodiments 1 to 4, wherein the polynucleotide encoding the fluorescent marker is transcriptionally linked to at least 2, 3, 4, or 5 STAT response elements.
6. The cell line according to any one of embodiments 1 to 5, wherein the cell line further comprises a polynucleotide encoding a sequence-specific endonuclease, in particular wherein the sequence-specific endonuclease is a CRISPR-associated (Cas) protein, in particular wherein the CRISPR-associated (Cas) protein is Cas9.
7. The cell line according to any one of embodiments 1 to 6, wherein the cell line is a mammalian cell line, in particular wherein the mammalian cell line is a human cell line, in particular wherein the human cell line is derived from HEK-293.
8. A method for obtaining an engineered common gamma chain (γ_{c}) variant, the method comprising the steps of:
   a) introducing a plurality of polynucleotides encoding common gamma chain (γ_{c}) variants into the cell line according to any one of embodiments 1 to 7;
   b) contacting the cells obtained in step (a) with a cytokine;
   c) isolating cell(s) expressing a polynucleotide encoding an engineered common gamma chain (γ_{c}) variant based on the expression level of the polynucleotide encoding the fluorescent marker; and
   d) obtaining the engineered common gamma chain (γ_{c}) variant encoded by the cell(s) isolated in step (c).
9. The method according to embodiment 8, wherein obtaining the engineered common gamma chain (γ_{c}) variant comprises the steps of:
   i) sequencing the polynucleotides encoding the engineered common gamma chain (γ_{c}) variants of the cells isolated in step (c); and
   ii) identifying a nucleotide sequence encoding an engineered common gamma chain (γ_{c}) variant based on the sequencing results obtained in step (i) and/or identifying one or more mutations in an engineered common gamma chain (γ_{c}) variant based on the sequencing results obtained in step (i).
10. The method according to embodiment 9, wherein the nucleotide sequence encoding the engineered common gamma chain (γ_{c}) variant and/or the one or more mutations in an engineered common gamma chain (γ_{c}) variant is/are identified with a classification algorithm.
11. A method for obtaining an engineered common gamma chain (γ_{c}) variant having enhanced cytokine responsiveness, the method comprising the steps of:
   a) introducing a polynucleotide encoding a common gamma chain (γ_{c}) variant into a first cell of a cell line according to any one of embodiments 1 to 7; and introducing a polynucleotide encoding a reference common gamma chain (γ_{c}) into a second cell of a cell line according to any one of embodiments 1 to 7;
   b) contacting the cells obtained in step (a) with a cytokine;
   c) identifying the common gamma chain (γ_{c}) variant as an engineered common gamma chain (γ_{c}) variant having enhanced responsiveness to the cytokine, if the expression of the polynucleotide encoding the fluorescent marker is higher in the first cell encoding said engineered common gamma chain (γ_{c}) variant compared to the second cell encoding the reference common gamma chain (γ_{c}); and
   d) obtaining an engineered common gamma chain (γ_{c}) variant having enhanced cytokine responsiveness based on the identification in step (c).
12. The method according to embodiment 11, wherein the polynucleotide encoding the engineered common gamma chain (γ_{c}) variant, and optionally also the polynucleotide encoding the reference common gamma chain (γ_{c}), is comprised in a library of polynucleotides encoding a plurality of common gamma chain (γ_{c}) variants.
13. The method according to embodiment 12, wherein the library of polynucleotides encoding a plurality of common gamma chain (γ_{c}) variants is obtained by site-directed mutagenesis of a template polynucleotide encoding a common gamma chain (γ_{c}).
14. The method according to embodiment 13, wherein the template polynucleotide encodes a common gamma chain (γ_{c}) having an amino acid sequence as set forth in SEQ ID NO:1.
15. The method according to embodiment 14, wherein at least one amino residue in position His66 to His214 of SEQ ID NO:1 has been subjected to site directed mutagenesis, in particular wherein at least one amino acid residue in position Tyr103 to Cys160 and/or Arg204 to His214 of SEQ ID NO:1 has been subjected to site directed mutagenesis.
16. The method according to embodiment 14 or 15, wherein at least one amino residue in position Arg263 to Lys272, Thr279 to Trp288, Leu299 to Cys308 and/or Ser333 to Leu343 of SEQ ID NO:1 is subjected to site directed mutagenesis.
17. The method according to any one of embodiments 11 to 16, wherein the polynucleotide encoding the engineered common gamma chain (γ_{c}) variant and the polynucleotide encoding the reference common gamma chain (γ_{c}) are integrated into the genomes of the first and second cell, respectively, by means of genome editing.
18. The method according to embodiment 17, wherein genome editing involves a sequence-specific endonuclease, in particular wherein the sequence-specific endonuclease is a CRISPR-associated (Cas) protein, in particular wherein the CRISPR-associated (Cas) protein is Cas9.
19. The method according to any one of embodiments 11 to 18, wherein the cytokine is IL-2, IL-4, IL-7, IL-9, IL-15 or IL-21, in particular wherein the cytokine is IL-2, IL-15 or IL-7.
20. The method according to any one of embodiments 11 to 19, wherein the reference common gamma chain (γ_{c}) comprises an amino acid sequence as set forth in SEQ ID NO:1.
21. The method according to any one of embodiments 11 to 20, wherein expression of the polynucleotide encoding the fluorescent marker is detected and/or quantified by flow cytometry.
22. The method according to any one of embodiments 11 to 21, wherein obtaining an engineered common gamma chain (γ_{c}) variant having enhanced cytokine responsiveness comprises a step of isolating cells encoding an engineered common gamma chain (γ_{c}) variant by fluorescence-activated cell sorting (FACS) based on the expression of the polynucleotide encoding the fluorescent marker.
23. The method according to embodiment 22, wherein the isolated cells encoding an engineered common gamma chain (γ_{c}) variant are subjected to sequencing, in particular deep sequencing.
24. The method according to embodiment 23, wherein a classification algorithm is used to identify mutations contributing to the phenotype of an engineered common gamma chain (γ_{c}) variant from the obtained sequencing results.
25. A method for obtaining an engineered common gamma chain (γ_{c}) variant having reduced cytokine responsiveness, the method comprising the steps of:
   a) introducing a polynucleotide encoding a common gamma chain (γ_{c}) variant into a first cell of a cell line according to any one of embodiments 1 to 7; and introducing a polynucleotide encoding a reference common gamma chain (γ_{c}) into a second cell of a cell line according to any one of embodiments 1 to 7;
   b) contacting the cells obtained in step (a) with a cytokine;
   c) identifying the common gamma chain (γ_{c}) variant as an engineered common gamma chain (γ_{c}) variant having reduced responsiveness to the cytokine, if the expression of the polynucleotide encoding the fluorescent marker is lower in the first cell encoding said engineered common gamma chain (γ_{c}) variant compared to the second cell encoding the reference common gamma chain (γ_{c}); and
   d) obtaining an engineered common gamma chain (γ_{c}) variant having reduced cytokine responsiveness based on the identification in step (c).
26. The method according to embodiment 25, wherein the polynucleotide encoding the engineered common gamma chain (γ_{c}) variant, and optionally also the polynucleotide encoding the reference common gamma chain (γ_{c}), is comprised in a library of polynucleotides encoding a plurality of common gamma chain (γ_{c}) variants.
27. The method according to embodiment 26, wherein the library of polynucleotides encoding a plurality of common gamma chain (γ_{c}) variants is obtained by site-directed mutagenesis of a template polynucleotide encoding a common gamma chain (γ_{c}).
28. The method according to embodiment 27, wherein the template polynucleotide encodes a common gamma chain (γ_{c}) having an amino acid sequence as set forth in SEQ ID NO:1.
29. The method according to embodiment 28, wherein at least one amino residue in position His66 to His214 of SEQ ID NO:1 has been subjected to site directed mutagenesis, in particular wherein at least one amino acid residue in position Tyr103 to Cys160 and/or Arg204 to His214 of SEQ ID NO:1 has been subjected to site directed mutagenesis.
30. The method according to embodiment 28 or 29, wherein at least one amino residue in position Arg263 to Lys272, Thr279 to Trp288, Leu299 to Cys308 and/or Ser333 to Leu343 of SEQ ID NO:1 is subjected to site directed mutagenesis.
31. The method according to any one of embodiments 25 to 30, wherein the polynucleotide encoding the engineered common gamma chain (γ_{c}) variant and the polynucleotide encoding the reference common gamma chain (γ_{c}) are integrated into the genomes of the first and second cell, respectively, by means of genome editing.
32. The method according to embodiment 31, wherein genome editing involves a sequence-specific endonuclease, in particular wherein the sequence-specific endonuclease is a CRISPR-associated (Cas) protein, in particular wherein the CRISPR-associated (Cas) protein is Cas9.
33. The method according to any one of embodiments 25 to 32, wherein the cytokine is IL-2, IL-4, IL-7, IL-9, IL-15 or IL-21, in particular wherein the suitable cytokine is IL-2, IL-15 or IL-7.
34. The method according to any one of embodiments 25 to 33, wherein the reference common gamma chain (γ_{c}) comprises an amino acid sequence as set forth in SEQ ID NO:1.
35. The method according to any one of embodiments 25 to 34, wherein the expression of the polynucleotide encoding the fluorescent marker is detected and/or quantified by flow cytometry.
36. The method according to any one of embodiments 25 to 35, wherein obtaining an engineered common gamma chain (γ_{c}) variant having reduced cytokine responsiveness comprises a step of isolating cells encoding an engineered common gamma chain (γ_{c}) variant having reduced cytokine responsiveness by fluorescence-activated cell sorting (FACS) based on the expression of the polynucleotide encoding the fluorescent marker.
37. The method according to embodiment 36, wherein the isolated cells encoding an engineered common gamma chain (γ_{c}) variant are subjected to sequencing, in particular deep sequencing.
38. The method according to embodiment 37, wherein a classification algorithm is used to identify mutations contributing to the phenotype of an engineered common gamma chain (γ_{c}) variant from the obtained sequencing results.
39. A method for obtaining an engineered common gamma chain (γ_{c}) variant for selective cytokine activation, the method comprising the steps of:
   a) introducing a plurality of polynucleotides encoding common gamma chain (γ_{c}) variants into a plurality of cells according to any one of embodiments 1 to 7;
   b) contacting the cells obtained in step (a) with a first cytokine;
   c) isolating cells based on the expression of the polynucleotide encoding the fluorescent marker in response to the first cytokine;
   d) contacting the cells isolated in step (c) with a second cytokine;
   e) isolating cells expressing based on the expression of the polynucleotide encoding the fluorescent marker in response to the second cytokine; and
   f) obtaining an engineered common gamma chain (γ_{c}) variant for selective cytokine activation from the cells isolated in step (e).
40. The method according to embodiment 39, wherein the plurality of polynucleotides encoding common gamma chain (γ_{c}) variants, and optionally the polynucleotide encoding the reference common gamma chain (γ_{c}), are part of a library of polynucleotides encoding common gamma chain (γ_{c}) variants.
41. The method according to embodiment 40, wherein the library of polynucleotides encoding a plurality of common gamma chain (γ_{c}) variants is obtained by site-directed mutagenesis of a template polynucleotide encoding a common gamma chain (γ_{c}).
42. The method according to embodiment 41, wherein the template polynucleotide encodes a common gamma chain (γ_{c}) having an amino acid sequence as set forth in SEQ ID NO:1.
43. The method according to embodiment 42, wherein at least one amino residue in position His66 to His214 of SEQ ID NO:1 has been subjected to site directed mutagenesis, in particular wherein at least one amino acid residue in position Tyr103 to Cys160 and/or Arg204 to His214 of SEQ ID NO:1 has been subjected to site directed mutagenesis.
44. The method according to embodiment 42 or 43, wherein at least one amino residue in position Arg263 to Lys272, Thr279 to Trp288, Leu299 to Cys308 and/or Ser333 to Leu343 of SEQ ID NO:1 is subjected to site directed mutagenesis.
45. The method according to any one of embodiments 39 to 44, wherein the polynucleotide encoding the engineered common gamma chain (γ_{c}) variant and, optionally, the polynucleotide encoding the reference common gamma chain (γ_{c}), are integrated into the genomes of the first and second cell, respectively, by means of genome editing.
46. The method according to embodiment 45, wherein genome editing involves a sequence-specific endonuclease, in particular wherein the sequence-specific endonuclease is a CRISPR-associated (Cas) protein, in particular wherein the CRISPR-associated (Cas) protein is Cas9.
47. The method according to any one of embodiments 39 to 46, wherein the first and second cytokine differ in their amino acid sequences.
48. The method according to embodiment 47, wherein the first and second cytokine are selected from the group consisting of: IL-2, IL-4, IL-7, IL-9, IL-15 and IL-21.
49. The method according to embodiment 47 or 48, wherein the first cytokine is an inactive variant of a cytokine and wherein the second cytokine is an active variant of the first cytokine.
50. The method according to embodiment 49, wherein the first cytokine is an inactivated IL-2 variant and wherein the second cytokine is IL-2 comprising an amino acid sequence as set forth in SEQ ID NO:2.
51. The method according to embodiment 50, wherein the inactivated IL-2 variant comprises a mutation at position Leu18, Gln22, Arg38, Lys43, Glu61, Asn88, Val91, Thr123, Gln126, Ser127 and/or Ser130 of SEQ ID NO:2.
52. The method according to any one of embodiments 39 to 51, wherein the reference common gamma chain (γ_{c}) comprises an amino acid sequence as set forth in SEQ ID NO:1.
53. The method according to any one of embodiments 39 to 52, wherein the cells in steps (c) and (e) are isolated by fluorescence-activated cell sorting (FACS) based on the expression level of the polynucleotide encoding the fluorescent marker.
54. The method according to embodiment 53, wherein the cells isolated in step (e) are subjected to sequencing, in particular to deep sequencing.
55. The method according to embodiment 54, wherein a classification algorithm is used to identify mutations contributing to the phenotype of the engineered common gamma chain (γ_{c}) variant from the obtained sequencing results.
56. A method for obtaining a chimeric common gamma chain (γ_{c}) receptor comprising a cytoplasmic domain of a common gamma chain (γ_{c}) receptor and an extracellular domain of a receptor protein, the method comprising the steps of:
   a) introducing a plurality of polynucleotides encoding chimeric common gamma chain (γ_{c}) receptor candidates into a plurality of cells according to any one of embodiments 1 to 7;
   b) contacting the cells obtained in step (a) with a ligand of the receptor protein;
   c) isolating cells expressing the polynucleotide encoding the fluorescent marker in response to the ligand of the receptor protein; and
   d) obtaining a chimeric common gamma chain (γ_{c}) receptor from the cells isolated in the (c).
57. The method according to embodiment 56, wherein the chimeric common gamma chain (γ_{c}) receptor candidates comprise amino acid residues Glu 262 to Thr 347 of SEQ ID NO:1.
58. The method according to embodiment 57, wherein at least one amino acid residue in position Arg263 to Lys272, Thr279 to Trp288, Leu299 to Cys308 and/or Ser333 to Leu343 of SEQ ID NO:1 is subjected to site directed mutagenesis.
59. The method according to any one of embodiments 56 to 58, wherein the synthetic common gamma chain (γ_{c}) receptor comprises an extracellular domain of a Fas receptor.
60. The method according to any one of embodiments 56 to 59, wherein the polynucleotide encoding the synthetic common gamma chain (γ_{c}) receptor is integrated into the genome of the cell by means of genome editing.
61. The method according to embodiment 60, wherein genome editing involves a sequence-specific endonuclease, in particular wherein the sequence-specific endonuclease is a CRISPR-associated (Cas) protein, in particular wherein the CRISPR-associated (Cas) protein is Cas9.
62. The method according to any one of embodiments 56 to 61, wherein the cells in step (c) are isolated by fluorescence-activated cell sorting (FACS) based on the expression level of the polynucleotide encoding the fluorescent marker.
63. The method according to embodiment 62, wherein the cells isolated in step (c) are subjected to sequencing, in particular to deep sequencing.
64. The method according to embodiment 63, wherein a classification algorithm is used to identify mutations contributing to the phenotype of the engineered common gamma chain (γ_{c}) variant from the obtained sequencing results.

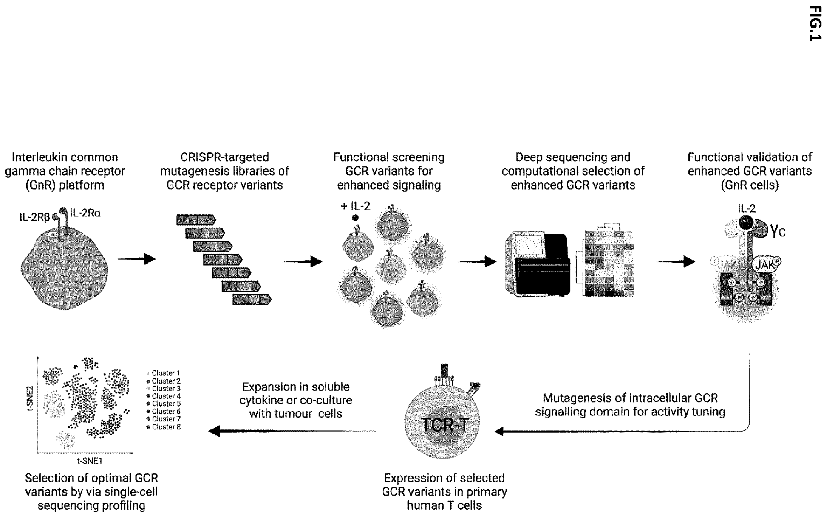

Herein, the inventors disclose a technology platform and data generation system enabling the molecular engineering and functional high-throughput screening of the common gamma chain receptor (γ_{c} or GCR for simplicity). This process, which is termed GCR-Engine, combines mammalian cellular display, CRISPR-targeted mutagenesis, functional screening, computational analysis and single-cell transcriptomic profiling for the identification and prediction of GCR variants with enhanced sensitivity and tuned signalling strength in response to specific γ_{c} cytokines (Fig. 1). GCR-Engine incorporates a functional dimension to cytokine receptor engineering, an important advance in the field given the very weak binding affinity that the GCR has for its cytokine ligands, a feature that complicates its engineering using traditional affinity-based approaches (e.g., phage and yeast display). A core component of GCR-Engine is the Gamma chain Reporter (GnR) engineered cell line. GnR cells are a derivative of HEK293 cells generated by multistep CRISPR-Cas9 genome editing in order to facilitate the display of GCR libraries and the detection of GCR signalling through a fluorescence STAT reporter, thus providing a phenotypic marker for high-throughput screening and selection by means of fluorescence activated cell sorting (FACS). The applications of the GCR-Engine workflow are many-fold, including but not limited to the following: (i) the engineering of GCR variants with enhanced responsiveness to key γ_{c} cytokines in T cell biology, (ii) tuning of GCR signalling output to promote T cell stemness, (iii) and engineering of GCRs with enhanced selectivity to individual or multiple γ_{c} cytokines. Thus, GCR-Engine is a unique tool for the discovery of GCR variants that can enhance the therapeutic efficacy of engineered T cells and other engineered cellular products (e.g., NK cells, NKT cells and macrophages).

Accordingly, in a particular embodiment, the invention relates to a cell line for identifying engineered common gamma chain (γ_{c}) receptors, the cell line comprising: (a) a reporter system comprising a polynucleotide encoding a fluorescent marker, wherein the polynucleotide encoding the fluorescent marker is transcriptionally linked to one or more STAT response elements; (b) at least one polynucleotide encoding a STAT protein, wherein the STAT protein activates the STAT response element comprised in the reporter system; and (c) at least one polynucleotide encoding a JAK protein; wherein said cell line does not encode a functional common gamma chain.

That is, the cell line according to the invention is a cell line that comprises a functional JAK/STAT signaling pathway but lacks a functional common gamma chain receptor. Furthermore, the cell line according to the invention encodes a fluorescent marker that is under transcriptional control of the JAK/STAT signaling pathway. Complementation of the cell line with a functional common gamma chain receptor results in expression of the fluorescent marker in the presence of a suitable cytokine.

Hence, the cell line according to the invention provides a platform for identifying engineered common gamma chain receptor variants. It has been shown herein that the cell line according to the invention can be used for identifying common gamma chain receptor variants that confer enhanced, reduced, or selective responsiveness to specific cytokines.

It is preferred herein that the endogenous gene encoding the common gamma chain is inactivated in the cell line according to the invention. The skilled person is aware of various strategies to inactivate an endogenous gene in a cell. For example, the endogenous gene encoding the common gamma chain may be fully or partially deleted or replaced with another nucleic acid. It is important to note that the term *"endogenous gene"* is not limited to the coding sequence of the gene but also relates to regulatory sequences, such as promoters and other regulatory sequences. Thus, an endogenous gene may be inactivated through modifications in one of more regulatory sequences. In other words, the cell line according to the invention is a cell line that is unable to express, at least under certain conditions, a functional common gamma chain receptor.

Methods for inactivating an endogenous gene include, without limitation, CRISPR/Cas-mediated genome editing, as described in Example 3 of the present invention. In human cells, the common gamma chain receptor is encoded by the gene *IL2RG.* The human *IL2RG* gene is located on the long (q) arm of the X chromosome at position 13.1.

In certain embodiments, the cell line according to the invention may be derived from a parental cell line that comprises a heterologous polynucleotide, or transgene, encoding a functional common gamma chain that can form a cytokine receptor complex. In such embodiments, it is required that the heterologous polynucleotide encoding the common gamma chain is inactivated as disclosed above for endogenous polynucleotides encoding a common gamma chain.

It is preferred herein that the cell line according to the invention is a cell line that comprises the JAK/STAT signaling pathway. That is, the cell line according to the invention comprises at least one nucleotide encoding a Janus kinase (JAK) protein family member and at least one nucleotide encoding a signal transducer and activator of transcription (STAT) protein family member. It is preferred herein that the cell line according to the invention encodes at least JAK1 and/or JAK3. However, more preferably, the cell line according to the invention encodes JAK1 and JAK3. Specific combinations of cytokine receptors and STAT family members will be disclosed herein below.

The cell line according to the invention further comprises a polynucleotide encoding a fluorescent protein to enable the identification and/or isolation of cells by flow cytometry based on the expression level of the fluorescent marker.

It is preferred herein that the polynucleotide encoding the fluorescent marker is functionally linked to a cytokine receptor via the JAK/STAT signaling pathway. That is, the polynucleotide encoding the fluorescent marker preferably comprises one or more STAT response elements to enable expression of the fluorescent marker in the presence of a phosphorylated STAT dimer. Accordingly, a *"STAT response element"* is a regulatory DNA element that can interact with a phosphorylated STAT dimer. Thus, it is preferred herein that the polynucleotide encoding the fluorescent marker is under control of a STAT-inducible promoter.

It is to be understood that each STAT protein binds to a specific STAT response element. Preferred combinations of STAT proteins and STAT response elements will be disclosed herein below.

The term *"fluorescent marker"* as used herein refers to a protein emitting light when irradiated with excitation light. The skilled person is aware of a wide range of fluorescent proteins. That is, the fluorescent protein may be, without limitation, a green fluorescent protein (GFP), a yellow fluorescent protein (YFP), a red fluorescent protein (RFP), an orange fluorescent protein (OFP), a cyan fluorescent protein (CFP), a blue fluorescent protein (BFP), or a far-red fluorescent protein. Herein, the green fluorescent protein may be enhanced green fluorescent protein (EGFP), Emerald, Superfolder GFP (sfGFP), Azami Green, TagGFP, TurboGFP, ZsGreen or T-Sapphire, the yellow fluorescent protein may be an enhanced yellow fluorescent protein (EYFP), Topaz, Venus, mCitrine, Ypet, TagYFP, PhiYFP, ZsYellow1 or mBanana, the red fluorescent protein may be mRuby, mRuby2 mApple, mStrawberry, AsRed2 or mRFP, the orange fluorescent protein may be Kusabira Orange, Kusabira Orange2, mOrange, mOrange2, dTomato, dTomato-Tandem, TagRFP, TagRFP-T, DsRed, DsRed2, DsRed-Express, DsRed-Monomer or mTangerine, the cyan fluorescent protein may be enhanced cyan fluorescent protein (ECFP), mECFP, mCerulean, CyPet AmCyan1, Midori-Ishi Cyan, TagCFP or mTFP1, the blue fluorescent protein may be enhanced blue fluorescent protein (EBFP), EBFP2, Azurite or mTagBFP, and the far red fluorescent protein may be mPlum, mCherry, dKeima-Tandem.

The common gamma chain receptor is a cytokine receptor sub-unit that is common to the receptor complexes for at least six different interleukin receptors: IL-2, IL-4, IL-7, IL-9, IL-15 and IL-21 receptor. Thus, the cell line according to the invention may be used for identifying common gamma chain receptors that confer altered or novel functionalities to any one of these cytokine receptors. For that, it is preferred that the cell line according to the invention comprises a particular combination of structural elements, i.e. a particular combination of cytokine receptor subunits, JAK proteins, STAT protein and STAT response elements.

In certain embodiments, the cell line according to the invention may be used for identifying common gamma chain receptor variants that confer altered or novel functionalities to an IL-2 receptor.

Thus, in a particular embodiment, the invention relates to the cell line according to the invention, wherein the cell line further comprises a polynucleotide encoding IL2Rβ.

In a particular embodiment, the invention relates to the cell line according to the invention, wherein the cell line further comprises one or more polynucleotides encoding IL2Rα and IL2Rβ.

In such embodiments, it is preferred that the cell line according to the invention comprises a polynucleotide encoding at least an IL-2 receptor beta chain (IL2Rβ). More preferably, the cell line according to the invention comprises one or more polynucleotides encoding an IL-2 receptor alpha (IL2Rα) and an IL-2 receptor beta chain (IL2Rβ). The IL-2 receptor subunits are associated with JAK1 and JAK3. Thus, in certain embodiments, the cell line according to the invention preferably comprises one or more polynucleotides encoding JAK1 and/or JAK3. Binding of a cytokine to the IL-2 receptor results in the transfer of phosphate groups to STAT5 proteins. Thus, in certain embodiments, the cell line according to the invention preferably comprises a polynucleotide encoding STAT5. To enable expression of the fluorescent marker protein, it is preferred that the polynucleotide encoding the fluorescent protein is transcriptionally linked to one or more STAT5 response elements.

Thus, in certain embodiments, the invention relates to the cell line according to the invention, wherein the cell line comprises one or more polynucleotides encoding IL2Rβ, JAK1, JAK3 and STAT5 and wherein the polynucleotide encoding the fluorescent protein is transcriptionally linked to one or more STAT5 response elements.

In certain embodiments, the invention relates to the cell line according to the invention, wherein the cell line comprises one or more polynucleotides encoding IL2Rα, IL2Rβ, JAK1, JAK3 and STAT5 and wherein the polynucleotide encoding the fluorescent protein is transcriptionally linked to one or more STAT5 response elements.

It is to be understood that the polynucleotides encoding IL2Rα, IL2Rβ, JAK1, JAK3 and/or STAT5 may be the endogenous genes encoding these proteins or may be polynucleotides that have been introduced into the cell line according to the invention.

In certain embodiments, the cell line according to the invention may be used for identifying common gamma chain receptor variants that confer altered or novel functionalities to an IL-4 receptor.

Thus, in a particular embodiment, the invention relates to the cell line according to the invention, wherein the cell line further comprises a polynucleotide encoding IL4Rα.

In such embodiments, it is preferred that the cell line according to the invention comprises a polynucleotide encoding at least an IL-4 receptor alpha chain (IL4Rα). The IL-4 receptor subunits are associated with JAK1 and JAK3. Thus, in certain embodiments, the cell line according to the invention preferably comprises one or more polynucleotides encoding JAK1 and/or JAK3. Binding of a cytokine to the IL-4 receptor results in the transfer of phosphate groups to STAT6 proteins. Thus, in certain embodiments, the cell line according to the invention preferably comprises a polynucleotide encoding STAT6. To enable expression of the fluorescent marker protein, it is preferred that the polynucleotide encoding the fluorescent protein is transcriptionally linked to one or more STAT6 response elements.

In certain embodiments, the invention relates to the cell line according to the invention, wherein the cell line comprises one or more polynucleotides encoding IL4Rα, JAK1, JAK3 and STAT6 and wherein the polynucleotide encoding the fluorescent protein is transcriptionally linked to one or more STAT6 response elements.

It is to be understood that the polynucleotides encoding IL4Rα, JAK1, JAK3 and/or STAT6 may be the endogenous genes encoding these proteins or may be polynucleotides that have been introduced into the cell line according to the invention.

In certain embodiments, the cell line according to the invention may be used for identifying common gamma chain receptor variants that confer altered or novel functionalities to an IL-7 receptor.

Thus, in a particular embodiment, the invention relates to the cell line according to the invention, wherein the cell line further comprises a polynucleotide encoding IL7Rα.

In such embodiments, it is preferred that the cell line according to the invention comprises a polynucleotide encoding at least an IL-7 receptor alpha chain (IL7Rα). The IL-7 receptor subunits are associated with JAK1 and JAK3. Thus, in certain embodiments, the cell line according to the invention preferably comprises one or more polynucleotides encoding JAK1 and/or JAK3. Binding of a cytokine to the IL-7 receptor results in the transfer of phosphate groups to STAT5 proteins. Thus, in certain embodiments, the cell line according to the invention preferably comprises a polynucleotide encoding STAT5. To enable expression of the fluorescent marker protein, it is preferred that the polynucleotide encoding the fluorescent protein is transcriptionally linked to one or more STAT5 response elements.

In certain embodiments, the invention relates to the cell line according to the invention, wherein the cell line comprises one or more polynucleotides encoding IL7Rα, JAK1, JAK3 and STAT5 and wherein the polynucleotide encoding the fluorescent protein is transcriptionally linked to one or more STAT5 response elements.

It is to be understood that the polynucleotides encoding IL7Rα, JAK1, JAK3 and/or STAT5 may be the endogenous genes encoding these proteins or may be polynucleotides that have been introduced into the cell line according to the invention.

In certain embodiments, the cell line according to the invention may be used for identifying common gamma chain receptor variants that confer altered or novel functionalities to an IL-9 receptor.

Thus, in a particular embodiment, the invention relates to the cell line according to the invention, wherein the cell line further comprises a polynucleotide encoding IL9Rα.

In such embodiments, it is preferred that the cell line according to the invention comprises a polynucleotide encoding at least an IL-9 receptor alpha chain (IL9Rα). The IL-9 receptor subunits are associated with JAK1 and JAK3. Thus, in certain embodiments, the cell line according to the invention preferably comprises one or more polynucleotides encoding JAK1 and/or JAK3. Binding of a cytokine to the IL-9 receptor results in the transfer of phosphate groups to STAT5 proteins. Thus, in certain embodiments, the cell line according to the invention preferably comprises a polynucleotide encoding STAT5. To enable expression of the fluorescent marker protein, it is preferred that the polynucleotide encoding the fluorescent protein is transcriptionally linked to one or more STAT5 response elements.

In certain embodiments, the invention relates to the cell line according to the invention, wherein the cell line comprises one or more polynucleotides encoding IL9Rα, JAK1, JAK3 and STAT5 and wherein the polynucleotide encoding the fluorescent protein is transcriptionally linked to one or more STAT5 response elements.

It is to be understood that the polynucleotides encoding IL9Rα, JAK1, JAK3 and/or STAT5 may be the endogenous genes encoding these proteins or may be polynucleotides that have been introduced into the cell line according to the invention.

In certain embodiments, the cell line according to the invention may be used for identifying common gamma chain receptor variants that confer altered or novel functionalities to an IL-15 receptor.

Thus, in a particular embodiment, the invention relates to the cell line according to the invention, wherein the cell line further comprises a polynucleotide encoding IL15Rα.

In a particular embodiment, the invention relates to the cell line according to the invention, wherein the cell line further comprises one or more polynucleotide encoding IL15Rα and IL2Rβ.

In such embodiments, it is preferred that the cell line according to the invention comprises a polynucleotide encoding at least an IL-15 receptor alpha chain (IL9Rα) and, preferably, an IL-2 receptor beta chain (IL2Rβ). The IL-15 receptor subunits are associated with JAK1 and JAK3. Thus, in certain embodiments, the cell line according to the invention preferably comprises one or more polynucleotides encoding JAK1 and/or JAK3. Binding of a cytokine to the IL-15 receptor results in the transfer of phosphate groups to STAT5 proteins. Thus, in certain embodiments, the cell line according to the invention preferably comprises a polynucleotide encoding STAT5. To enable expression of the fluorescent marker protein, it is preferred that the polynucleotide encoding the fluorescent protein is transcriptionally linked to one or more STAT5 response elements.

In certain embodiments, the invention relates to the cell line according to the invention, wherein the cell line comprises one or more polynucleotides encoding IL15Rα, JAK1, JAK3 and STAT5 and wherein the polynucleotide encoding the fluorescent protein is transcriptionally linked to one or more STAT5 response elements.

In certain embodiments, the invention relates to the cell line according to the invention, wherein the cell line comprises one or more polynucleotides encoding IL15Rα, IL2Rβ,JAK1, JAK3 and STAT5 and wherein the polynucleotide encoding the fluorescent protein is transcriptionally linked to one or more STAT5 response elements.

It is to be understood that the polynucleotides encoding IL15Rα, IL2Rβ,JAK1, JAK3 and/or STAT5 may be the endogenous genes encoding these proteins or may be polynucleotides that have been introduced into the cell line according to the invention.

In certain embodiments, the cell line according to the invention may be used for identifying common gamma chain receptor variants that confer altered or novel functionalities to an IL-21 receptor.

Thus, in a particular embodiment, the invention relates to the cell line according to the invention, wherein the cell line further comprises a polynucleotide encoding IL21Rα.

In such embodiments, it is preferred that the cell line according to the invention comprises a polynucleotide encoding at least an IL-21 receptor alpha chain (IL21Rα). The IL-21 receptor subunits are associated with JAK1 and JAK3. Thus, in certain embodiments, the cell line according to the invention preferably comprises one or more polynucleotides encoding JAK1 and/or JAK3. Binding of a cytokine to the IL-21 receptor results in the transfer of phosphate groups to STAT3 proteins. Thus, in certain embodiments, the cell line according to the invention preferably comprises a polynucleotide encoding STAT3. To enable expression of the fluorescent marker protein, it is preferred that the polynucleotide encoding the fluorescent protein is transcriptionally linked to one or more STAT3 response elements.

In certain embodiments, the invention relates to the cell line according to the invention, wherein the cell line comprises one or more polynucleotides encoding IL21Rα, JAK1, JAK3 and STAT3 and wherein the polynucleotide encoding the fluorescent protein is transcriptionally linked to one or more STAT5 response elements.

It is to be understood that the polynucleotides encoding IL21Rα, JAK1, JAK3 and/or STAT3 may be the endogenous genes encoding these proteins or may be polynucleotides that have been introduced into the cell line according to the invention.

In a particular embodiment, the invention relates to the cell line according to the invention, wherein the cell line further comprises one or more polynucleotides encoding IL4Rα, IL9Rα, IL15Rα and/or IL21Rα.

In a particular embodiment, the invention relates to the cell line according to the invention, wherein the polynucleotide encoding the fluorescent marker is transcriptionally linked to at least 2, 3, 4, or 5 STAT response elements.

That is, in certain embodiments, the reporter system comprising the polynucleotide encoding the fluorescent marker comprises at least 1, 2, 3, 4 or 5 STAT response elements. In certain embodiments, the reporter system comprising the polynucleotide encoding the fluorescent marker comprises at least 1, 2, 3, 4, 5, 6, 7, 8, 9 or 10 STAT response elements. In certain embodiments, the reporter system comprising the polynucleotide encoding the fluorescent marker comprises 5 STAT response elements.

The STAT response elements comprised in the reporter system may have identical nucleotide sequences. That, it each STAT response element may be bound by the same STAT protein. However, the reporter system may also comprise two or more distinct STAT response elements, i.e. STAT response elements that are bound by different STAT protein. For example, in certain embodiments, the reporter system may comprise one or more STAT3 response elements and one or more STAT5 response elements. This is of particular interest in a cell line that is intended for engineering cytokine receptors that phosphorylate both STAT3 and STAT5, such as the IL-21 receptor.

In certain embodiments, the reporter system according to the invention comprises one or more STAT5 response element. Preferably, the STAT5 response element can be bound by human STAT5 and comprises the nucleotide sequence GGTTTTCCTGGAAAGTT (SEQ ID NO:6), TTCCTGGAA (SEQ ID NO:7), AGTTCTGAGAAAAGT (SEQ ID NO:8) or TTCTGAGAA (SEQ ID NO:9).

In certain embodiments, the reporter system according to the invention comprises one or more STAT6 response element. Preferably, the STAT6 response element can be bound by human STAT6 and comprises a nucleotide sequence AGTTCCGAGGAAAAGT (SEQ ID NO:10) or TTCCGAGGAA (SEQ ID NO:11).

In certain embodiments, the reporter system according to the invention comprises one or more STAT3 response element. Preferably, the STAT3 response element can be bound by human STAT3 and comprises a nucleotide sequence AGCTTCATTTCCCGTAAATCGTCGA (SEQ ID NO:12) or TTCCCGTAA (SEQ ID NO:13).

The skilled person is aware of suitable methods with which the cell line according to the invention may be obtained. In particular, the skilled person is aware of molecular biology methods and methods of molecular cloning to disrupt endogenous genes in a cell and/or to introduce heterologous polynucleotides into the genome of a cell.

Within the present invention, the subunits of the cytokine receptors (i.e. IL2Rα, IL2Rβ, IL4Rα, IL7Rα, IL9Rα, IL15Rα and IL21Rα), the JAK proteins and/or the STAT proteins may be encoded by endogenous genes of the cell line according to the invention. However, heterologous polynucleotides encoding the aforementioned proteins may also be integrated into the genome of the cell line according to the invention by methods of molecular cloning.

Further, the endogenous gene encoding the common gamma chain receptor or a transgene encoding the common gamma chain receptor may be deleted, disrupted or replaced by molecular biology methods known in the art.

The reporter system comprising the polynucleotide encoding the fluorescent marker and the STAT response elements is preferably encoded by a heterologous polynucleotide. Methods for integrating said heterologous polynucleotide into the genome of a cell are known to the person skilled in the art.

It is preferred herein that the disruption of endogenous genes or transgenes (i.e. the IL2RG gene) or the integration of heterologous polynucleotides into the genome is achieved by genome editing, in particular with the CRISPR/Cas method as known in the art. However, it is to be understood that the cell line according to the invention may have been obtained by any other method known in the art. For example, heterologous polynucleotides may be integrated into the genome of a cell by viral transduction as known in the art.

To facilitate CRISPR/Cas-mediated genome editing, it is preferred herein that the cell line according to the invention encodes a sequence-specific endonuclease, in particular a CRISPR-associated protein.

Thus, in a particular embodiment, the invention relates to the cell line according to the invention, wherein the cell line further comprises a polynucleotide encoding a sequence-specific endonuclease, in particular wherein the sequence-specific endonuclease is a CRISPR-associated (Cas) protein, in particular wherein the CRISPR-associated (Cas) protein is Cas9.

Cell lines encoding a CRISPR-associated protein have the advantage that it is no longer necessary to introduce a purified CRISPR-associated protein together with the tracrRNA and the crRNA in every genome editing step.

The polynucleotide encoding the CRISPR-associated protein may be introduced into the cell line according to the invention by any method known in the art. For example, the polynucleotide encoding the CRISPR-associated protein may be integrated into the genome of the cell line according to the invention by CRISPR/Cas-mediated genome editing as disclosed in Example 1. Alternatively, the polynucleotide encoding the CRISPR-associated protein may be integrated into the genome of the cell line according to the invention by viral transduction.

The term *"CRISPR-associated protein"* refers to RNA-guided endonucleases that are part of a CRISPR (Clustered Regularly Interspaced Short Palindromic Repeats) system (and their homologs, variants, fragments or derivatives), which is used by prokaryotes to confer adaptive immunity against foreign DNA elements. CRISPR-associated proteins include, without limitation, Cas9, Cpf1 (Cas12), C2c1, C2c3, C2c2, Cas13, CasX and CasY.

As used herein, the term *"CRISPR-associated protein"* includes wild-type proteins as well as homologs, variants, fragments and derivatives thereof. Therefore, when referring to artificial nucleic acid molecules encoding Cas9, Cpf1 (Cas12), C2c1, C2c3, and C2c2, Cas13, CasX and CasY, said artificial nucleic acid molecules may encode the respective wild-type proteins, or homologs, variants, fragments and derivatives thereof.

It is important to note that the presence of a polynucleotide encoding a CRISPR-associated protein is not an essential feature of the cell line according to the invention. That is, the cell line according to the invention may have been obtained without using the CRISPR/Cas method or, where the cell line according to the invention has been obtained using the CRISPR/Cas method, the polynucleotide encoding the CRISPR-associated protein may have been removed from the genome of the cell line subsequently.

The cell line according to the invention may be derived from any suitable cell line. In certain embodiments, the cell line according to the invention is derived from a eukaryotic cell, in particular a mammalian cell. In certain embodiments, the cell line according to the invention is derived from a human cell. It is preferred herein that all elements disclosed herein (the cytokine receptors, the common gamma chain, the JAK protein and the STAT proteins) have the same origin as the cell line according to the invention. In particular, it is preferred that all elements disclosed herein and the cell line have a human origin.

In a particular embodiment, the invention relates to the cell line according to the invention, wherein the cell line is a mammalian cell line, in particular wherein the mammalian cell line is a human cell line, in particular wherein the human cell line is derived from HEK-293.

The term *"cell line",* as used herein, refers to a cell culture comprising a single cell type that can be serially propagated in culture for prolonged periods. Human embryonic kidney 293 cells, also often referred to as HEK 293, HEK-293, 293 cells, or less precisely as HEK cells, are a specific immortalized cell line derived from a spontaneously miscarried or aborted fetus or human embryonic kidney cells grown in tissue culture taken from a female fetus in 1973. HEK 293 cells have been widely used in cell biology research for many years, because of their reliable growth and propensity for transfection. They are also used by the biotechnology industry to produce therapeutic proteins and viruses for gene therapy as well as safety testing for a vast array of chemicals.

Within the present invention, it is preferred that the cell line is a stable cell line, more preferably a stable human cell line. That is, the polynucleotides disclosed herein are preferably integrated into the genome of the cell line.

In further embodiments, the invention relates to the use of the cell line according to the invention for obtaining engineered common gamma chain variants.

That is, in a particular embodiment, the invention relates to a method for obtaining an engineered common gamma chain (γ_{c}) variant, the method comprising the steps of: (a) introducing a plurality of polynucleotides encoding common gamma chain (γ_{c}) variants into the cell line according to the invention; (b) contacting the cells obtained in step (a) with a cytokine; (c) isolating cell(s) expressing a polynucleotide encoding an engineered common gamma chain (γ_{c}) variant based on the expression level of the polynucleotide encoding the fluorescent marker; and (d) obtaining the engineered common gamma chain (γ_{c}) variant encoded by the cell(s) isolated in step (c).

That is, the cell line according to the invention may be used for identifying engineered common gamma chain variants from a library of common gamma chain variants, wherein the engineered common gamma chain variants are identified based on the expression level of a reporter gene. That is, the cell line according to the invention may be used to identify engineered common gamma chain variants that can modulate (enhance or reduce) the responsiveness of a cytokine receptor to a specific cytokine or cytokine variant.

As disclosed in more detail above, the cell line according to the invention lacks a functional common gamma chain receptor and comprises a reporter system that is under control of the JAK/STAT signaling pathway. Accordingly, the cell line according to the invention may be used as a screening platform to identify engineered common gamma chain variants that can complement cytokine receptors and/or confer new functionalities to cytokine receptors. In particular, the cell line according to the invention may be used to identify engineered common gamma chain variants that can complement and confer new functionalities to IL-2, IL-4, IL-7, IL-9, IL-15 and/or IL-21 cytokine receptors, as these cytokine receptors share the common gamma chain.

For that, the cell line according to the invention may be contacted with a plurality of polynucleotides encoding one or more common gamma chain receptor variant(s). In one embodiment, the cell line according to the invention is contacted with a plurality of polynucleotides encoding the same common gamma chain receptor variant. The variant may be a wild type variant or a modified variant.

However, it is preferred herein that the plurality of polynucleotides encoding the common gamma chain receptor variants is a library of polynucleotides, wherein at least two polynucleotides encode a different common gamma chain receptor variant. That is, the cell line according to the invention may be contacted with a plurality of polynucleotides encoding at least 2, at least 5, at least 10, at least 25, at least 50, at least 100, at least 150, at least 200, at least 250, at least 300, at least 350, at least 400, at least 500, at least 10³, at least 10⁴, at least 10⁵, at least 10⁶, or at least 10⁷ different common gamma chain receptor variants.

A library of polynucleotides encoding common gamma chain receptor variants may be obtained by methods known in the art. For example, a library of polynucleotides encoding common gamma chain receptor variants may be obtained by introducing mutations in a template polynucleotide encoding a common gamma chain receptor. Various PCR methods for introducing random or site-specific mutations into DNA templates are known in the art. Alternatively, libraries of polynucleotides encoding common gamma chain receptor variants may be designed computationally and then synthesized chemically. Commercial suppliers of computationally designed libraries are known to the person skilled in the art.

Several positions in the human common gamma chain receptor have been disclosed as either being involved in the interaction with other cytokine receptor subunits or as being involved in signal transduction via the JAK/STAT pathway. These residues are of particular interest when generating libraries comprising polynucleotides that encode common gamma chain receptor variants.

For example, several amino acid residues at positions His66 - His214 of the human common gamma chain (SEQ ID NO:1) have been disclosed as being involved in the interaction with other cytokine receptor subunits. That is, in certain embodiments, the plurality of polynucleotides encoding common gamma chain receptor variants may be obtained by introducing one or more mutations at any one of positions His66, Tyr67, Trp68, Tyr69, Lys70, Asn71, Ser72, Asp73, Asn74, Asp75, Lys76, Val77, Gln78, Lys79, Cys80, Ser81, His82, Tyr83, Leu84, Phe85, Ser86, Glu87, Glu88, Ile89, Thr90, Ser91, Gly92, Cys93, Gln94, Leu95, Gln96, Lys97, Lys98, Glu99, Ile100, His101, Leu102, Tyr103, Gln104, Thr105, Phe106, Val107, Val108, Gln109, Leu110, Gln111, Asp112, Pro113, Arg114, Glu115, Pro116, Arg117, Arg118, Gln119, Ala120, Thr121 Gln122, Met123, Leu124, Lys125, Leu126, Gln127, Asn128, Leu129, Val130, Ile131, Pro132, Trp133, Ala134, Pro135, Glu136, Asn137, Leu138, Thr139, Leu140, His141, Lys142, Leu143, Ser144, Glu145, Ser146, Gln147, Leu148, Glu149, Leu150, Asn151, Trp152, Asn153, Asn154, Arg155, Phe156, Leu157, Asn158, His159, Cys160, Leu161, Glu162, His163, Leu164, Val165, Gln166, Tyr167, Arg168, Thr169, Asp170, Trp171, Asp172, His173, Ser174, Trp175, Thr176, Glu177, Gln178, Ser179, Val180, Asp181, Tyr182, Arg183, His184, Lys185, Phe186, Ser187, Leu188, Pro189, Ser190, Val191, Asp192, Gly193, Gln194, Lys195, Arg196, Tyr197, Thr198, Phe199, Arg200, Val201, Arg202, Ser203, Arg204, Phe205, Asn206, Pro207, Leu208, Cys209, Gly210, Ser211, Ala212, Gln213 and/or His214.

In a particular embodiment, the plurality of polynucleotides encoding common gamma chain receptor variants may be obtained by introducing one or more mutations at any one of positions Lys98, Glu99, Ile100, His101, Leu102, Tyr103, Gln104, Thr105, Phe106, Val107, Val108, Gln109, Leu110, Gln111, Asp112, Pro113, Arg114, Glu115, Pro116, Arg117, Arg118, Gln119, Ala120, Thr121 Gln122, Met123, Leu124, Lys125, Leu126, Gln127, Asn128, Leu129, Val130, Ile131, Pro132, Trp133, Ala134, Pro135, Glu136, Asn137, Leu138, Thr139, Leu140, His141, Lys142, Leu143, Ser144, Glu145, Ser146, Gln147, Leu148, Glu149, Leu150, Asn151, Trp152, Asn153, Asn154, Arg155, Phe156, Leu157, Asn158, His159, Cys160, Leu161, Glu162, His163, Leu164, Val165, Arg204, Phe205, Asn206, Pro207, Leu208, Cys209, Gly210, Ser211, Ala212, Gln213 and/or His214.

In a more particular embodiment, the plurality of polynucleotides encoding common gamma chain receptor variants may be obtained by introducing one or more mutations at any one of positions Lys98, Glu99, Ile100, His101, Leu102, Tyr103, Gln104, Thr105, Phe106, Val107, Val108, Arg155, Phe156, Leu157, Asn158, His159, Cys160, Leu161, Glu162, His163, Leu164, Val165, Arg204, Phe205, Asn206, Pro207, Leu208, Cys209, Gly210, Ser211, Ala212, Gln213 and/or His214.

In a more particular embodiment, the plurality of polynucleotides encoding common gamma chain receptor variants may be obtained by introducing one or more mutations at any one of positions Arg204, Phe205, Asn206, Pro207, Leu208, Cys209, Gly210, Ser211, Ala212, Gln213 and/or His214.

Several amino acid residues at positions Arg263 - Lys272, Thr279 - Trp288, Leu299 - Cys308 and Ser333 - Leu343 of the human common gamma chain (SEQ ID NO:1) have been disclosed as being involved in JAK/STAT-mediated signal transduction. That is, the plurality of polynucleotides encoding common gamma chain receptor variants may be obtained by introducing one or more mutations at any one of positions Arg263, Thr264, Met265, Pro266, Arg267, Ile268, Pro269, Thr270, Leu271, Lys272, Thr279, Glu280, Tyr281, His282, Gly283, Asn284, Phe285, Ser286, Ala287. Trp288, Leu299, Gln300, Pro301, Asp302, Tyr303, Ser304, Glu305, Arg306, Leu307, Cys308, Ser333, Pro334, Tyr335, Trp336, Ala337, Pro338, Pro339, Cys340, Tyr341, Thr342 and/or Leu343.

In certain embodiments, the plurality of polynucleotides encoding the common gamma chain receptor variants may be obtained by site-saturation mutagenesis of one or more of the aforementioned amino acid residues. For that, a codon encoding any one of the aforementioned amino acid residues may be replaced with a degenerated NNK codon to obtain variants having all possible amino acid substitutions in the respective position. Preferably, site-saturation mutagenesis is performed as described by Mason et al., Nucleic Acids Research, 2018, 46(14), 7436-7449.

The plurality of polynucleotides encoding the common gamma chain receptor variants may be a plurality of linear or circular DNA molecules, wherein each DNA molecule encodes a common gamma chain receptor variant. In a preferred embodiment, the plurality of polynucleotides encoding the common gamma chain receptor variants is a plurality (or a library) of plasmids.

The skilled person is aware of methods to introduce a DNA molecule into a cell line. For example, the plurality of polynucleotides encoding the common gamma chain receptor variants may be introduced into the cell line according to the invention by transfection, as disclosed in the appended examples.

The term *"transfection"* as used herein refers to the introduction of foreign DNA into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics. Preferably, the plurality of polynucleotides encoding the common gamma chain receptor variants are transfected into the cell line according to the invention by electroporation, as disclosed in the appended examples.

Within the present invention, it is preferred that the polynucleotides encoding the common gamma chain receptor variants are integrated into the genome of the cell line according to the invention. For that, each polynucleotide encoding a common gamma chain receptor variant preferably comprises a complementary region that allows site specific integration into a pre-determined location in the genome of the cell line. Preferred target locations in the genome of the host cell, also referred to as safe harbours, are known to the person skilled in the art and include, without limitation, the AAVS1 locus and the CCR5 locus.

To allow site-specific integration of a polynucleotide encoding a common gamma chain receptor variant into the genome of the cell line according to the invention, the polynucleotide encoding the common gamma chain receptor variant is preferably introduced into the cell line according to the invention together with a gRNA that introduces a double strand break at the respective target location in the genome. Therefore, it is preferred that the cell line according to the invention encodes a CRISPR-associated protein.

In certain embodiments, the plurality of polynucleotides encoding the common gamma chain receptor variants is a DNA plasmid library that is introduced into the cell line according to the invention together with a gRNA that introduces a double strand break at the integration site of the plasmids. In certain embodiments, the plasmids and the gRNA are designed such that the polynucleotide encoding the common gamma chain variant can be integrated into the AAVS1 locus of the cell line according to the invention.

The first step of the method according to the invention results in a plurality of cells, wherein each cell has been complemented with a polynucleotide encoding a common gamma chain receptor variant. Cells that have been successfully complemented with a polynucleotide encoding a common gamma chain receptor variant can be identified and/or isolated by flow cytometry. For example, a commercial anti-GCR antibody may be used to identify and/or isolate cells that express a common gamma chain receptor variant on their cell surface. Accordingly, in certain embodiments, the method according to the invention comprises an additional step of identifying and/or isolating cells that express a common gamma chain receptor on their cell surface. That is, the method according to the invention may comprise a first round of selection, where cells are selected that express a common gamma chain receptor (variant) on their cell surface and a second round of selection, where the responsiveness of the cell to one or more cytokines is assessed.

Accordingly, in a next step, cells expressing a common gamma chain receptor on their cell surface are contacted with a cytokine. The cytokine is preferably a cytokine that can be recognized by a cytokine receptor that comprises a common gamma chain. That is, the cytokine is preferably IL-2, IL-4, IL-7, IL-9, IL-15 or IL-21. Further, the cytokine is preferably a cytokine that can be recognized by the cell line according to the invention (suitable combination of cytokine receptor subunits, JAK and STAT protein).

However, the cytokine may also be a modified variant of any of the aforementioned cytokines. For example, the cytokine may be a variant that has been engineered for increased stability. Further, the cytokine may be an inactive variant, i.e. a variant that cannot be bound by a wild-type cytokine receptor to activate downstream signaling. In such embodiments, the cell line according to the invention may be used to identify engineered common gamma chain variants that can confer binding to the inactivated variant, but no longer to the active variant. The cell line according to the invention may thus be used to identify orthogonal cytokine - cytokine receptor pairs, as described in more detail below.

The cytokine is preferably contacted with the cells in a liquid, i.e. in a buffer or a cell culture medium. That is, a suspension comprising the cells is preferably mixed with a solution comprising the cytokine. The cytokine may be added to the cells at any suitable concentration. That is, the cytokine may be added to the cells at a concentration ranging from 0.1 pM to 1µM, from 1 pM to 100 nM, from 5 pM to 50 nM, from 25 pM to 40 nM, from 100 pM to 30 nM, from 0.5 nM to 20 nM, from 1 nM to 15 nM or from 5 nM to 10 nM. When screening for engineered common gamma chain variants that attenuate cytokine binding, higher concentrations of cytokines may be added to the cells. For example, in such embodiments, the cytokine may be added to the cells at a concentration ranging from 1 nM to 500 nM, from 5 nM to 200 nM or from 10 nM to 100 nM. However, it is to be noted that the exact concentration of the cytokine depends on the aim of the experiment (identifying more/less responsive cytokine receptors, identifying orthogonal cytokine receptors, etc.). The skilled person is capable of identifying suitable cytokine concentrations for a particular application. Further, the skilled person is capable of identifying a suitable number of cells to be contacted with the cytokine. In a non-limiting embodiment, 10⁵ - 10⁶ cells/mL may be contacted with 5 nM of a cytokine.

The cells may be contacted with the cytokine for at least 1, 2, 3, 4, 5, 6, 7, 8, 9, 10, 12, 16, 20, 24, 36, 48, 72, 96 or 120 hours. In a non-limiting embodiment, the cells are contacted with the cytokine for 24 hours. Preferably, the contacting takes place in cell culture medium, for example in DMEM medium with 10 % FBS. However, any suitable cell culture medium may be used. The skilled person is capable of identifying suitable cell culture media based on the origin of the cell line according to the invention.

After the contacting step, cell(s) expressing a polynucleotide encoding an engineered common gamma chain (γ_{c}) variant may be isolated based on the expression level of the polynucleotide encoding the fluorescent marker. Since the polynucleotide encoding the fluorescent marker is under control of the JAK/STAT signaling pathway, the fluorescent marker will only be expressed when the cytokine receptor comprising the common gamma chain variant is activated by a suitable cytokine. Thus, only cells that express a responsive cytokine receptor comprising a functional common gamma chain variant will become fluorescent. Fluorescent cells can readily be separated from non-fluorescent cells, for example by fluorescence-activated cell sorting (FACS). Methods and devices for separating fluorescent cells from non-fluorescent cells are well known in the art. Furthermore, the skilled person is capable of isolating sub-populations of fluorescent cells, *i.e.* a highly fluorescent sub-population of cells.

It is important to note that the method according to the invention may not only be used for identifying engineered common gamma chain receptor variants that render the cell more responsive to a cytokine. Instead, the method according to the invention may also be used for identifying engineered common gamma chain receptors that no longer respond to one or more cytokine or show a weaker response to one or more cytokine. In these embodiments, cells can be isolated that do not express or only weakly express the fluorescent marker in the presence of a particular cytokine (but still express the common gamma chain receptor on the cell surface).

In certain embodiments, steps (b) and (c), i.e. contacting cells with a cytokine and isolating cells based on the expression of the fluorescent marker, may be repeated multiple times. That is, steps (b) and (c) may be repeated 2, 3, 4, 5, 6, 7, 8, 9 or 10 times. Further, steps (b) and (c) may be repeated with different cytokines or different concentrations of the same cytokine. For example, in a first found, cells may be isolated that are more responsive to IL-15 (high fluorescence) and in a second round cells having reduced responsiveness to IL-2 (no fluorescence) may be isolated. Thus, engineered common gamma chain receptor variants may be isolated which can confer altered cytokine responsiveness to a cell.

Once cells with a desired response to one or more cytokines have been isolated in step (c), an engineered common gamma chain (γc) variant may be obtained from these cells. Within the present invention, obtaining an engineered common gamma chain variant preferably comprises a step of obtaining a nucleic acid sequence encoding said engineered common gamma chain variant. For that, DNA may be isolated from the cells that have been isolated in step (c). The obtained DNA may then be amplified and sequenced by any suitable method known in the art. For example, DNA obtained from a single cell may be sequenced by Sanger sequencing as known in the art. However, DNA may also be obtained from an entire population of cells (for example the fluorescent population) and subjected to deep sequencing.

The skilled person is capable of translating an obtained nucleic acid sequence into a protein sequence. Thus, by sequencing the DNA encoding the engineered common gamma chain receptor, the skilled person can obtain the engineered common gamma chain receptor.

Thus, in a particular embodiment, the invention relates to the method according to the invention, wherein obtaining the engineered common gamma chain (γ_{c}) variant comprises the steps of: (i) sequencing the polynucleotides encoding the engineered common gamma chain (γ_{c}) variants of the cells isolated in step (c); and (ii) identifying a nucleotide sequence encoding an engineered common gamma chain (γ_{c}) variant based on the sequencing results obtained in step (i) and/or identifying one or more mutations in an engineered common gamma chain (γ_{c}) variant based on the sequencing results obtained in step (i).

As mentioned above, a nucleic acid sequence encoding an engineered common gamma chain receptor may be obtained from a single cell or from a population of cells.

In certain embodiments, a nucleic acid encoding an engineered common gamma chain receptor may be amplified from a single cell or a population of monoclonal cells and subjected to Sanger sequencing. As such, the nucleic acid sequence of the entire engineered common gamma chain receptor may be directly obtained and translated into an amino acid sequence.

In a preferred embodiment, multiple nucleic acid sequences encoding engineered common gamma chain receptors may be obtained by deep sequencing of a population of cells that has been isolated based on a specific response to one or more cytokines. The term *"deep sequencing"* as used herein generally refers to next- or higher-generation sequencing known to a skilled artisan. In particular, the term *"deep sequencing"* as used herein indicates that the total number of reads is many times larger than the length of the sequence under study.

The method according to the invention preferably comprises a step of deep mutational scanning involving a combination of multi-site saturation mutagenesis (preferably of one or more of the positions disclosed above), phenotypic screening (preferably by FACS) and deep sequencing.

Reads obtained by deep or next-generation sequencing are typically short and do not cover the entire polynucleotide encoding the engineered common gamma chain receptor. Thus, deep or next generation sequencing is preferably used to identify one or more mutations that contribute to a specific phenotype. Thus, *"identifying a nucleotide sequence encoding an engineered common gamma chain (γ_{c}) variant"* in the broadest sense encompasses the identification of one or more mutations that contribute to a specific phenotype. Thus, in certain embodiments, an engineered common gamma chain receptor can be obtained by introducing one or more mutations that have been identified with the method according to the invention into a common gamma chain receptor

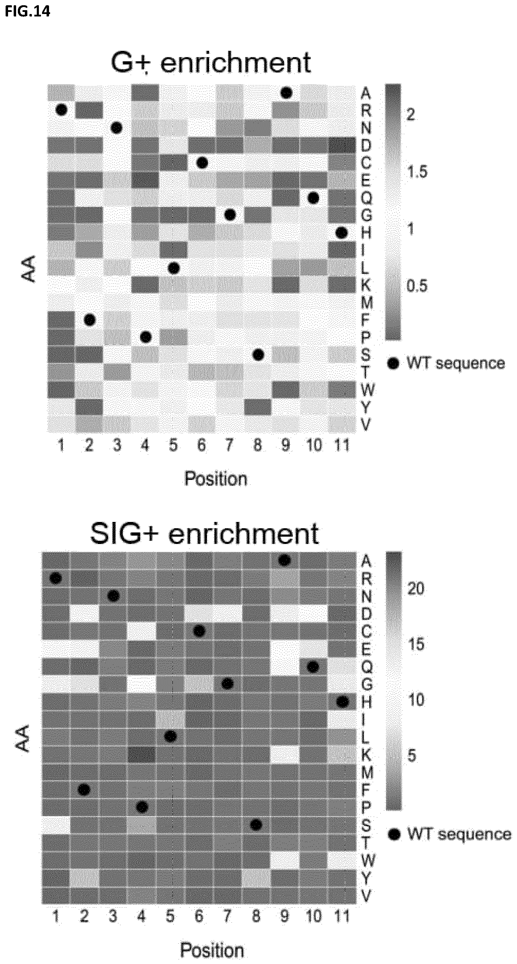

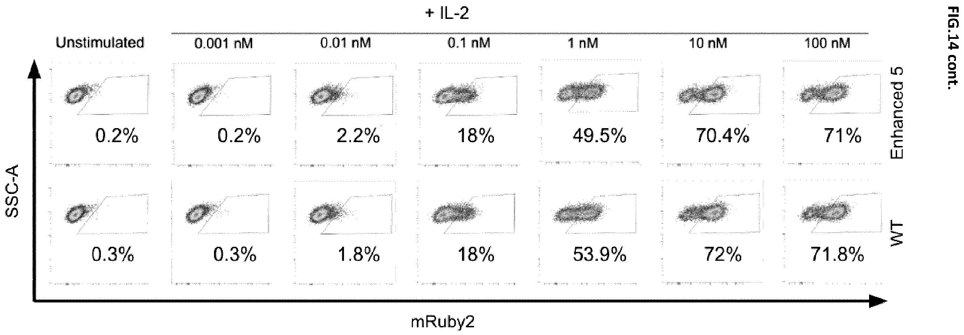

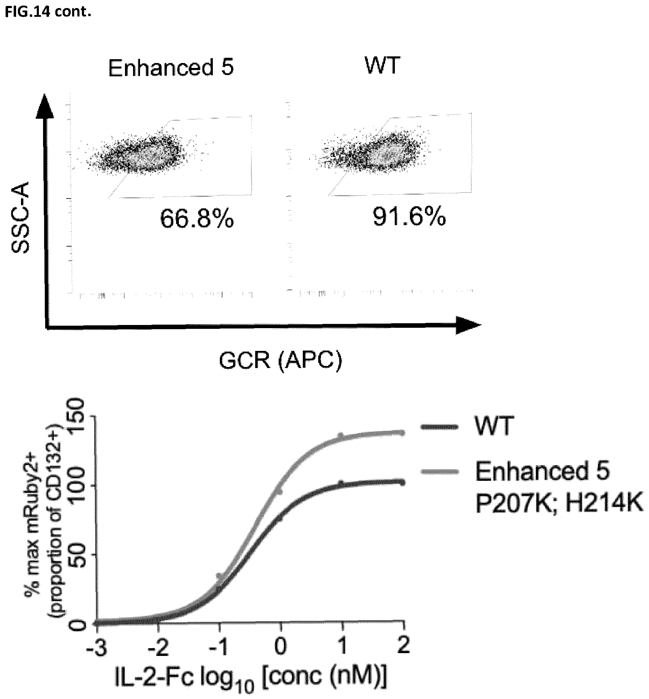

The skilled person is aware of bioinformatic methods that can be used to process the data obtained by deep sequencing. In particular, bioinformatic methods may be used to identify mutations that are enriched in a cell population. Methods for read frequency enrichment are known to the person skilled in the art. For example, read frequency enrichment is applied in Fig.14 to identify mutations P207K and H214K that result in increased responsiveness to IL-2.

To facilitate the identification of mutations that contribute to a specific phenotype, a classification algorithm may be used. Thus, in a particular embodiment, the invention relates to the method according to the invention, wherein the nucleotide sequence encoding the engineered common gamma chain (γ_{c}) variant and/or the one or more mutations in an engineered common gamma chain (γ_{c}) variant is/are identified with a classification algorithm.

The classification algorithm is preferably trained with deep sequencing data sets. For example, the classification algorithm may be trained with a first data set that has been obtained by sequencing of a cell population that expressed the fluorescent marker in response to a specific cytokine and with a second data set that has been obtained by sequencing a cell population that did not express the fluorescent marker in response to the same cytokine. For that, the deep sequencing data sets may be converted into a form that can be provided to a classification algorithm. Conversion of the data sets may be achieved, without limitation, by one-hot encoding, as known in the art.

Various classification algorithms can be used for identifying mutations that contribute to a specific phenotype. The term *"classification algorithm"* refers to a supervised learning technique that is used to identify the category of new observations on the basis of training data. In classification, a program learns from the given dataset or observations and then classifies new observation into a number of classes or groups. For example, a classification algorithm may be used within the present invention to decide whether a mutation in a common gamma chain variant contributes to a specific phenotype or not. The classification algorithms may be, without limitation, a support vector machine (SVM) algorithm, a random forest algorithm, or an artificial neural network.

It is to be understood that the method according to the invention aims at obtaining an engineered common gamma chain receptor. However, *"obtaining an engineered common gamma chain receptor"* also encompasses obtaining the genetic information of an engineered common gamma chain receptor variant. The genetic information of an engineered common gamma chain receptor variant may be a nucleic acid sequence encoding an entire engineered common gamma chain receptor variant. However, the genetic information of an engineered common gamma chain receptor variant may also be restricted to one or more key mutations that contribute to a specific phenotype of an engineered common gamma chain receptor.

Thus, in a particular embodiment, the invention relates to a method for identifying one or more mutations in an engineered common gamma chain (γ_{c}) variant, the method comprising the steps of: (a) introducing a plurality of polynucleotides encoding common gamma chain (γ_{c}) variants into the cell line according to the invention; (b) contacting the cells obtained in step (a) with a cytokine; (c) isolating cell(s) expressing a polynucleotide encoding an engineered common gamma chain (γ_{c}) variant based on the expression level of the polynucleotide encoding the fluorescent marker; and (d) identifying one or more mutations in the engineered common gamma chain (γ_{c}) variant encoded by the cell(s) isolated in step (c).

It is to be understood that the one or more mutations are preferably mutations that are enriched in a particular cell population. After the one or more mutations have been identified using the method according to the invention, their impact may be verified experimentally. For that, the mutations that have been identified using the method according to the invention may be introduced into a wild-type common gamma chain receptor either alone or in combination. The impact of these mutations may then be verified by testing the responsiveness of cells expressing the resulting common gamma chain receptors to one or more suitable cytokines. Verification of the phenotype may be performed *in vitro* or *in vivo.*

In a particular embodiment, the method as disclosed herein above may be used for obtaining an engineered common gamma chain receptor variant having enhanced responsiveness to a specific cytokine.

Thus, in a particular embodiment, the invention relates to a method for obtaining an engineered common gamma chain (γ_{c}) variant having enhanced cytokine responsiveness, the method comprising the steps of: (a) introducing a polynucleotide encoding an common gamma chain (γ_{c}) variant into a first cell of a cell line according to the invention; and introducing a polynucleotide encoding a reference common gamma chain (γ_{c}) into a second cell of a cell line according to the invention; (b) contacting the cells obtained in step (a) with a cytokine; (c) identifying a common gamma chain (γ_{c}) variant as an engineered common gamma chain (γ_{c}) variant having enhanced responsiveness to the cytokine, if the expression of the polynucleotide encoding the fluorescent marker is higher in the first cell encoding said engineered common gamma chain (γ_{c}) variant compared to the second cell encoding the reference common gamma chain (γ_{c}); and (d) obtaining an engineered common gamma chain (γ_{c}) variant having enhanced cytokine responsiveness based on the identification in step (c).

In Example 7, the inventors have shown that the method according to the invention can be used to identify engineered common gamma chain receptor variants having enhanced responsiveness to IL-2. That is, deep mutational scanning has been performed at pre-defined positions of the common gamma chain receptor to identify mutations that are associated with an enhanced responsiveness to IL-2. The identified mutations (P207K and H214K) were then re-introduced into the common gamma chain receptor to verify the phenotype.

To identify an engineered common gamma chain (γ_{c}) variant having enhanced cytokine responsiveness to a specific cytokine, the phenotype of a cell encoding said engineered common gamma chain (γ_{c}) variant has to be compared to the phenotype of a cell encoding a reference common gamma chain (γ_{c}).

The reference common gamma chain (γ_{c}) may be a wild-type human common gamma chain (γ_{c}) having an amino acid sequence as set forth in SEQ ID NO:1. Thus, in a particular embodiment, the invention relates to the method according to the invention, wherein the reference common gamma chain (γ_{c}) comprises an amino acid sequence as set forth in SEQ ID NO:1. However, the reference common gamma chain (γ_{c}) may be a common gamma chain (γ_{c}) variant itself. That is, the reference common gamma chain (γ_{c}) may be a common gamma chain (γ_{c}) variant that is encoded in the same library as the engineered common gamma chain (γ_{c}) variant.

The cell encoding the engineered common gamma chain (γ_{c}) variant and the cell encoding the reference common gamma chain (γ_{c}) are preferably obtained by introducing a plurality of polynucleotides encoding common gamma chain (γ_{c}) variants into the cell line according to the invention, as described herein. Thus, in a particular embodiment, the invention relates to the method according to the invention, wherein the polynucleotide encoding the engineered common gamma chain (γ_{c}) variant, and optionally also the polynucleotide encoding the reference common gamma chain (γ_{c}), is comprised in a library of polynucleotides encoding a plurality of common gamma chain (γ_{c}) variants.

In certain embodiments, the library of polynucleotides is a deep mutational scanning library that has been obtained by site-saturation mutagenesis (at one or multiple positions) of a template polynucleotide encoding a common gamma chain (γ_{c}). Thus, in a particular embodiment, the invention relates to the method according to the invention, wherein the library of polynucleotides encoding a plurality of common gamma chain (γ_{c}) variants is obtained by site-directed mutagenesis of a template polynucleotide encoding a common gamma chain (γ_{c}).

That is, one or more codons of the template polynucleotide encoding the common gamma chain (γ_{c}) may be replaced with a degenerate codon (i.e. the degenerate codon NNK). The degenerate codon NNK has the benefit that it encodes all 20 canonical amino acids but encodes only a single stop codon. Thus, using the degenerate codon NNK allows replacing a particular amino acid residue of a common gamma chain (γ_{c}) with all other 19 amino acid residues, while having a low frequency of non-functional variants.

The library of polynucleotides may be obtained by applying site-directed mutagenesis to one or more codons of a template polynucleotide encoding a common gamma chain (γ_{c}). Preferably, site-directed mutagenesis is applied to one or more codons encoding amino acid residues of a human common gamma chain (γ_{c}), in particular the amino acid residues defined herein above.

That is, in a particular embodiment, the invention relates to the method according to the invention, wherein the template polynucleotide encodes a common gamma chain (γ_{c}) having an amino acid sequence as set forth in SEQ ID NO:1.

The mutations may be introduced into regions of the human common gamma chain (γ_{c}) that are responsible for interacting with other cytokine receptor subunits. Thus, in a particular embodiment, the invention relates to the method according to the invention, wherein at least one amino acid residue in position His66 to His214 of SEQ ID NO:1 is subjected to site directed mutagenesis, in particular wherein at least one amino acid residue in position Tyr103 to Cys160 and/or Arg204 to His214 of SEQ ID NO:1 is subjected to site directed mutagenesis.

Alternatively or in addition, the mutations may be introduced into regions of the human common gamma chain (γ_{c}) that are involved in JAK/STAT-mediated signal transduction. Thus, in another embodiment, the invention relates to the method according to the invention, wherein at least one amino acid residue in position Arg263 to Lys272, Thr279 to Trp288, Leu299 to Cys308 and/or Ser333 to Leu343 of SEQ ID NO:1 is subjected to site directed mutagenesis.

The library of polynucleotides may be introduced into the cell line according to the invention by any method known in the art. However, it is preferred that the polynucleotide encoding the common gamma chain (γ_{c}) variant is integrated into the genome of the cell line according to the invention. Preferably, the polynucleotide encoding the common gamma chain (γ_{c}) variant is integrated into the genome of the cell line according to the invention at a pre-defined site. Site-directed integration of a polynucleotide into the genome of a cell may be achieved by genome editing, as disclosed herein above. Thus, in a particular embodiment, the invention relates to the method according to the invention, wherein the polynucleotide encoding the engineered common gamma chain (γ_{c}) variant and the polynucleotide encoding the reference common gamma chain (γ_{c}) are integrated into the genomes of the first and second cell, respectively, by means of genome editing.

In a particular embodiment, the invention relates to the method according to the invention, wherein genome editing involves a sequence-specific endonuclease, in particular wherein the sequence-specific endonuclease is a CRISPR-associated (Cas) protein, in particular wherein the CRISPR-associated (Cas) protein is Cas9.

The obtained cells are then contacted with the cytokine to which the responsiveness is to be enhanced. For that, the cells may be contacted with the cytokine at a fixed or at varying concentrations, i.e., where multiple rounds of contacting with varying cytokine concentrations are performed. For example, by contacting cells with varying, i.e. decreasing, cytokine concentrations, engineered common gamma chain (γ_{c}) variants may be obtained that confer higher sensitivity to cytokine receptors.

In certain embodiments, the method according to the invention is used to increase responsiveness to one or more naturally occurring cytokine(s), in particular cytokine(s) that are recognized by a cytokine receptor comprising a common gamma chain. Thus, in a particular embodiment, the invention relates to the method according to the invention, wherein the cytokine is IL-2, IL-4, IL-7, IL-9, IL-15 or IL-21, in particular wherein the suitable cytokine is IL-2, IL-15 or IL-7. Contacting the cells with the cytokine may be performed as disclosed elsewhere herein.

The method according to the present invention comprises a step of comparing the expression of a fluorescent marker between a first cell and a second cell. When comparing the expression of the fluorescent marker between two cells, the cell expressing higher levels of the fluorescent protein can be determined to express an engineered common gamma chain (γ_{c}) variant having enhanced cytokine responsiveness, whereas the cell expressing lower levels of the fluorescent protein can determined to express the reference common gamma chain (γ_{c}). That is, it is not necessary to determine the first cell and the second cell prior to the identification in step (c). In particular, the first and second cell may be identified by flow cytometry.

Engineered common gamma chain (γ_{c}) variants that confer enhanced responsiveness to a cytokine receptor will result in increased expression of the fluorescent marker, in particular at low cytokine concentrations. Thus, engineered common gamma chain (γ_{c}) variants that confer enhanced responsiveness to a cytokine receptor are preferably identified in cells that express high levels of the fluorescent marker in the presence of a cytokine and, in particular, at low cytokine concentrations.

The expression level of the fluorescent marker is preferably determined by flow cytometry. Thus, in a particular embodiment, the invention relates to the method according to the invention, wherein the expression of the polynucleotide encoding the fluorescent marker is detected and/or quantified by flow cytometry.

Cells expressing an engineered common gamma chain (γ_{c}) variant having enhanced responsiveness to a cytokine are preferably isolated by FACS based on the expression level of the fluorescent marker. Thus, in a particular embodiment, the invention relates to the method according to the invention, wherein obtaining an engineered common gamma chain (γ_{c}) variant having enhanced cytokine responsiveness comprises a step of isolating cells encoding an engineered common gamma chain (γ_{c}) variant having enhanced cytokine responsiveness by fluorescence-activated cell sorting (FACS) based on the expression of the polynucleotide encoding the fluorescent marker.

To obtain an engineered common gamma chain (γ_{c}) variant having enhanced cytokine responsiveness, the cells may be subjected to various rounds of screening, involving contacting the cells with different cytokine concentrations and subsequent isolation of fluorescent cells. In particular, the cytokine concentration may be decreased in each round to enrich cells that respond even to residual amounts of the cytokine.

The isolated cells may then be subjected to sequencing as disclosed herein above. In a preferred embodiment, a population of fluorescent cells may be isolated and subjected to deep sequencing. Thus, in a particular embodiment, the invention relates to the method according to the invention, wherein the isolated cells encoding an engineered common gamma chain (γ_{c}) variant are subjected to sequencing, in particular deep sequencing.

Mutations that contribute to an enhanced cytokine responsiveness may be identified by feeding the obtained sequencing data into a trained classification algorithm as disclosed herein above. Thus, in a particular embodiment, the invention relates to the method according to the invention, wherein a classification algorithm is used to identify a nucleotide sequence encoding an engineered common gamma chain (γ_{c}) variant having enhanced cytokine responsiveness from the obtained sequencing results.

In a particular embodiment, the invention relates to the method according to the invention, wherein a classification algorithm is used to identify mutations contributing to the phenotype of an engineered common gamma chain (γ_{c}) variant from the obtained sequencing results.

In a particular embodiment, the invention relates to a cytokine receptor comprising an engineered common gamma chain variant.

In certain embodiments, the invention relates to a cytokine receptor comprising an engineered common gamma chain variant, wherein the engineered common gamma chain variant confers enhanced responsiveness to IL-2, IL-4, IL-7, IL-9, IL-15 and/or IL-21.

In certain embodiments, the invention relates to a cytokine receptor comprising an engineered common gamma chain variant, wherein the engineered common gamma chain variant confers enhanced responsiveness to IL-2.

In certain embodiments, the invention relates to a cytokine receptor comprising an engineered common gamma chain variant, wherein the engineered common gamma chain variant confers enhanced responsiveness to IL-2, and wherein the engineered common gamma chain variant is derived from a human common gamma chain (SEQ ID NO:1) and comprises one or more mutations at any one of positions Arg204, Phe205, Asn206, Pro207, Leu208, Cys209, Gly210, Ser211, Ala212, Gln213 and/or His214.

In certain embodiments, the invention relates to a cytokine receptor comprising an engineered common gamma chain variant, wherein the engineered common gamma chain variant confers enhanced responsiveness to IL-2, and wherein the engineered common gamma chain variant is derived from a human common gamma chain (SEQ ID NO:1) and comprises mutations at positions P207 and/or H214.

In certain embodiments, the invention relates to a cytokine receptor comprising an engineered common gamma chain variant, wherein the engineered common gamma chain confers enhanced responsiveness to IL-2, and wherein the engineered common gamma variant chain variant is derived from a human common gamma chain (SEQ ID NO:1) and comprises mutations P207K and/or H214K

In certain embodiments, the invention relates to a cytokine receptor comprising an engineered common gamma chain variant, wherein the engineered common gamma chain variant confers enhanced responsiveness to IL-4.

In certain embodiments, the invention relates to a cytokine receptor comprising an engineered common gamma chain variant, wherein the engineered common gamma chain variant confers enhanced responsiveness to IL-7.

In certain embodiments, the invention relates to a cytokine receptor comprising an engineered common gamma chain variant, wherein the engineered common gamma chain variant confers enhanced responsiveness to IL-9.

In certain embodiments, the invention relates to a cytokine receptor comprising an engineered common gamma chain variant, wherein the engineered common gamma chain variant confers enhanced responsiveness to IL-15.

In certain embodiments, the invention relates to a cytokine receptor comprising an engineered common gamma chain variant, wherein the engineered common gamma chain variant confers enhanced responsiveness to IL-21.

The method according to the invention may be used in a similar way to identify engineered common gamma chain (γ_{c}) variant having reduced cytokine responsiveness.

That is, in a particular embodiment, the invention relates to a method for obtaining an engineered common gamma chain (γ_{c}) variant having reduced cytokine responsiveness, the method comprising the steps of: (a) introducing a polynucleotide encoding a common gamma chain (γ_{c}) variant into a first cell of a cell line according to the invention; and introducing a polynucleotide encoding a reference common gamma chain (γ_{c}) into a second cell of a cell line according to the invention; (b) contacting the cells obtained in step (a) with a cytokine; (c) identifying a common gamma chain (γ_{c}) variant as an engineered common gamma chain (γ_{c}) variant having reduced responsiveness to the cytokine, if the expression of the polynucleotide encoding the fluorescent marker is lower in the first cell encoding said engineered common gamma chain (γ_{c}) variant compared to the second cell encoding the reference common gamma chain (γ_{c}); and (d) obtaining an engineered common gamma chain (γ_{c}) variant having reduced cytokine responsiveness based on the identification in step (c).

Engineered common gamma chain (γ_{c}) variants having reduced cytokine responsiveness may be obtained by isolating cells that express lower levels of the fluorescent marker than cells expressing a reference common gamma chain (γ_{c}) when contacted with a cytokine. To be able to discriminate between engineered common gamma chain (γ_{c}) variants having reduced cytokine responsiveness and non-functional common gamma chain (γ_{c}) variants, it is preferred that multiple rounds of positive and negative selection are performed.

For example, engineered common gamma chain (γ_{c}) variants having reduced cytokine responsiveness may be obtained by introducing a plurality of polynucleotides encoding common gamma chain (γ_{c}) variants into the cell line according to the invention and contacting the obtained cells with a cytokine at high cytokine concentrations. After the contacting step, cells that express the fluorescent marker may be isolated and contacted again with the same cytokine, but this time at a lower concentration. After the second contacting step, only the cells that do not express the fluorescent marker are isolated. The cells isolated in the second round will be cells that only respond to a cytokine when it is added at high concentrations.

As described herein above, the first and second cell are preferably obtained by introducing a library of polynucleotides encoding a plurality of common gamma chain (γ_{c}) variants into the cell line according to the invention. Thus, in a particular embodiment, the invention relates to the method according to the invention, wherein the polynucleotide encoding the engineered common gamma chain (γ_{c}) variant, and optionally also the polynucleotide encoding the reference common gamma chain (γ_{c}), is part of a library of polynucleotides encoding a plurality of common gamma chain (γ_{c}) variants.

In a particular embodiment, the invention relates to the method according to the invention, wherein the library of polynucleotides encoding a plurality of common gamma chain (γ_{c}) variants is obtained by site-directed mutagenesis of a template polynucleotide encoding a common gamma chain (γ_{c}).

In a particular embodiment, the invention relates to the method according to the invention, wherein the common gamma chain (γ_{c}) encoded by the template polynucleotide comprises an amino acid sequence as set forth in SEQ ID NO:1.

In a particular embodiment, the invention relates to the method according to the invention, wherein at least one amino acid residue in position His66 to His214 of SEQ ID NO:1 is subjected to site directed mutagenesis, in particular wherein at least one amino acid residue in position Tyr103 to Cys160 and/or Arg204 to His214 of SEQ ID NO:1 is subjected to site directed mutagenesis.

In a particular embodiment, the invention relates to the method according to the invention, wherein at least one amino acid residue in position Arg263 to Lys272, Thr279 to Trp288, Leu299 to Cys308 and/or Ser333 to Leu343 of SEQ ID NO:1 is subjected to site directed mutagenesis.

Preferably, the polynucleotides encoding the common gamma chain (γ_{c}) variants have been designed in a way such that they can be integrated into the genome after they have been introduced into the cell line according to the invention in a site-specific manner.

Thus, in a particular embodiment, the invention relates to the method according to the invention, wherein the polynucleotide encoding the engineered common gamma chain (γ_{c}) variant and the polynucleotide encoding the reference common gamma chain (γ_{c}) are integrated into the genome of the cell by means of genome editing.

In a particular embodiment, the invention relates to the method according to the invention, wherein genome editing involves a sequence-specific endonuclease, in particular wherein the sequence-specific endonuclease is a CRISPR-associated (Cas) protein, in particular wherein the CRISPR-associated (Cas) protein is Cas9.

In a particular embodiment, the invention relates to the method according to the invention, wherein the suitable cytokine is IL-2, IL-4, IL-7, IL-9, IL-15 or IL-21, in particular wherein the cytokine is IL-2, IL-15 or IL-7.

In a particular embodiment, the invention relates to the method according to the invention, wherein the reference common gamma chain (γ_{c}) comprises an amino acid sequence as set forth in SEQ ID NO:1.

In a particular embodiment, the invention relates to the method according to the invention, wherein the expression of the polynucleotide encoding the fluorescent marker is detected and/or quantified by flow cytometry.

In a particular embodiment, the invention relates to the method according to the invention, wherein obtaining an engineered common gamma chain (γ_{c}) variant having reduced cytokine responsiveness comprises a step of isolating cells encoding an engineered common gamma chain (γ_{c}) variant having reduced cytokine responsiveness by fluorescence-activated cell sorting (FACS) based on the expression of the polynucleotide encoding the fluorescent marker.

In a particular embodiment, the invention relates to the method according to the invention, wherein the isolated cells encoding an engineered common gamma chain (γ_{c}) variant are subjected to sequencing, in particular deep sequencing.

In a particular embodiment, the invention relates to the method according to the invention, wherein a classification algorithm is used to identify a nucleotide sequence encoding an engineered common gamma chain (γ_{c}) variant having reduced cytokine responsiveness from the obtained sequencing results.

In a particular embodiment, the invention relates to the method according to the invention, wherein a classification algorithm is used to identify mutations contributing to the phenotype of an engineered common gamma chain (γ_{c}) variant from the obtained sequencing results

In a particular embodiment, the invention relates to the method according to the invention, wherein a classification algorithm is used to identify mutations contributing to the phenotype of an engineered common gamma chain (γ_{c}) variant from the obtained sequencing results.

In certain embodiments, the invention relates to a cytokine receptor comprising an engineered common gamma chain variant, wherein the engineered common gamma chain variant confers reduced responsiveness to IL-2, IL-4, IL-7, IL-9, IL-15 and/or IL-21.

In certain embodiments, the invention relates to a cytokine receptor comprising an engineered common gamma chain variant, wherein the engineered common gamma chain variant confers reduced responsiveness to IL-2.

In certain embodiments, the invention relates to a cytokine receptor comprising an engineered common gamma chain variant, wherein the engineered common gamma chain variant confers reduced responsiveness to IL-2, and wherein the engineered common gamma chain variant is derived from a human common gamma chain (SEQ ID NO:1) and comprises one or more mutations at any one of positions Arg204, Phe205, Asn206, Pro207, Leu208, Cys209, Gly210, Ser211, Ala212, Gln213 and/or His214.

In certain embodiments, the invention relates to a cytokine receptor comprising an engineered common gamma chain variant, wherein the engineered common gamma chain variant confers reduced responsiveness to IL-2, and wherein the engineered common gamma chain variant is derived from a human common gamma chain (SEQ ID NO:1) and comprises mutations at positions P207 and/or H214.

In certain embodiments, the invention relates to a cytokine receptor comprising an engineered common gamma chain variant, wherein the engineered common gamma chain variant confers reduced responsiveness to IL-4.

In certain embodiments, the invention relates to a cytokine receptor comprising an engineered common gamma chain variant, wherein the engineered common gamma chain variant confers reduced responsiveness to IL-7.

In certain embodiments, the invention relates to a cytokine receptor comprising an engineered common gamma chain variant, wherein the engineered common gamma chain variant confers reduced responsiveness to IL-9.

In certain embodiments, the invention relates to a cytokine receptor comprising an engineered common gamma chain variant, wherein the engineered common gamma chain variant confers reduced responsiveness to IL-15.

In certain embodiments, the invention relates to a cytokine receptor comprising an engineered common gamma chain variant, wherein the engineered common gamma chain variant confers reduced responsiveness to IL-21.

Instead of enhancing or reducing the responsiveness to a single cytokine, the method according to the invention may also be used for obtaining engineered common gamma chain (γ_{c}) variant that result in a selective cytokine response. For example, the method according to the invention may be used to enhance or maintain the responsiveness to a first cytokine and to simultaneously reduce the responsiveness to a second cytokine. Furthermore, the method according to the invention may be used to identify an engineered common gamma chain (γ_{c}) variant that confers responsiveness to an inactive cytokine variant but abolishes or reduces responsiveness to the active counterpart.

Thus, in a particular embodiment, the invention relates to a method for obtaining an engineered common gamma chain (γ_{c}) variant for selective cytokine activation, the method comprising the steps of: (a) introducing a plurality of polynucleotides encoding common gamma chain (γ_{c}) variants into a plurality of cells according to the invention; (b) contacting the cells obtained in step (a) with a first cytokine; (c) isolating cells based on the expression of the polynucleotide encoding the fluorescent marker in response to the first cytokine; (d) contacting the cells isolated in step (c) with a second cytokine; (e) isolating cells based on the expression of the polynucleotide encoding the fluorescent marker in response to the second cytokine; and (f) obtaining an engineered common gamma chain (γ_{c}) variant for selective cytokine activation from the cells isolated in step (e).

First, a plurality of polynucleotides encoding common gamma chain (γ_{c}) variants may be introduced into the cell line according to the invention as described herein. The plurality of polynucleotides may be obtained as disclosed elsewhere herein, in particular by site-saturation mutagenesis, preferably as part of a deep-mutational scanning workflow.

Thus, in a particular embodiment, the invention relates to the method according to the invention, wherein the plurality of polynucleotides encoding common gamma chain (γ_{c}) variants, and optionally the polynucleotide encoding the reference common gamma chain (γ_{c}), are part of a library of polynucleotides encoding common gamma chain (γ_{c}) variants.

In a particular embodiment, the invention relates to the method according to the invention, wherein the library of polynucleotides encoding a plurality of common gamma chain (γ_{c}) variants is obtained by site-directed mutagenesis of a template polynucleotide encoding a common gamma chain (γ_{c}).

In a particular embodiment, the invention relates to the method according to the invention, wherein the common gamma chain (γ_{c}) encoded by the template polynucleotide comprises an amino acid sequence as set forth in SEQ ID NO:1.

In a particular embodiment, the invention relates to the method according to the invention, wherein at least one amino acid residue in position His66 to His214 of SEQ ID NO:1 is subjected to site directed mutagenesis, in particular wherein at least one amino acid residue in position Tyr103 to Cys160 and/or Arg204 to His214 of SEQ ID NO:1 is subjected to site directed mutagenesis.

In a particular embodiment, the invention relates to the method according to the invention, at least one amino acid residue in position Arg263 to Lys272, Thr279 to Trp288, Leu299 to Cys308 and/or Ser333 to Leu343 of SEQ ID NO:1 is subjected to site directed mutagenesis.

Preferably, the polynucleotides encoding the common gamma chain (γ_{c}) variants are designed in a way such that they can be integrated into the genome after they have been introduced into the cell line according to the invention.

Thus, in a particular embodiment, the invention relates to the method according to the invention, wherein the polynucleotide encoding the engineered common gamma chain (γ_{c}) variant and, optionally, the polynucleotide encoding the reference common gamma chain (γ_{c}), are integrated into the genome of the cell by means of genome editing.

In a particular embodiment, the invention relates to the method according to the invention, wherein genome editing involves a sequence-specific endonuclease, in particular wherein the sequence-specific endonuclease is a CRISPR-associated (Cas) protein, in particular wherein the CRISPR-associated (Cas) protein is Cas9.

Obtaining engineered common gamma chain (γ_{c}) variants for selective cytokine activation typically requires multiple rounds of selection with two or more cytokines, preferably including rounds of positive and negative selection. Thus, in a particular embodiment, the invention relates to the method according to the invention, wherein the first and second cytokine differ in their amino acid sequences.

The first and second cytokine may be naturally occurring cytokines. That is, in a particular embodiment, the invention relates to the method according to the invention, wherein the first and second cytokine are selected from the group consisting of: IL-2, IL-4, IL-7, IL-9, IL-15 and IL-21.

In certain embodiments, the method according to the invention may be used to obtain engineered common gamma chain (γ_{c}) variants that confer enhanced responsiveness to two different cytokines. For example, the method according to the invention may be used to obtain engineered common gamma chain (γ_{c}) variants that confer enhanced responsiveness to two different cytokines selected from the group consisting of: IL-2, IL-4, IL-7, IL-9, IL-15 and IL-21. In certain embodiments, the method according to the invention may be used to obtain engineered common gamma chain (γ_{c}) variants that confer enhanced responsiveness to IL-7 and IL-15.

Thus, in certain embodiments, the invention relates to a method for obtaining an engineered common gamma chain (γ_{c}) variant for selective cytokine activation, the method comprising the steps of: (a) introducing a plurality of polynucleotides encoding common gamma chain (γ_{c}) variants into a plurality of cells according to the invention; (b) contacting the cells obtained in step (a) with a first cytokine; (c) isolating cells expressing the polynucleotide encoding the fluorescent marker in response to the first cytokine; (d) contacting the cells isolated in step (c) with a second cytokine; (e) isolating cells expressing the polynucleotide encoding the fluorescent marker in response to the second cytokine; and (f) obtaining an engineered common gamma chain (γ_{c}) variant for selective cytokine activation from the cells isolated in step (e).

Identifying engineered common gamma chain (γ_{c}) variants conferring enhanced responsiveness to two or more cytokines may require two or more rounds of positive selection. Preferably, multiple rounds of selection at different cytokine concentrations are performed for each cytokine.

In certain embodiments, the method according to the invention may be used to obtain engineered common gamma chain (γ_{c}) variants that confer enhanced responsiveness to a first cytokine and reduced responsiveness to a second cytokine. For example, the method according to the invention may be used to obtain engineered common gamma chain (γ_{c}) variants that confer enhanced responsiveness to a cytokine selected from the group consisting of: IL-2, IL-4, IL-7, IL-9, IL-15 and IL-21 and reduced responsiveness to another cytokine selected from the same group. In certain embodiments, the method according to the invention may be used to obtain engineered common gamma chain (γ_{c}) variants that confer enhanced responsiveness to IL-15 and reduced responsiveness to IL-2. In certain embodiments, the method according to the invention may be used to obtain engineered common gamma chain (γ_{c}) variants that confer enhanced responsiveness to IL-7 and reduced responsiveness to IL-2. In certain embodiments, the method according to the invention may be used to obtain engineered common gamma chain (γ_{c}) variants that confer enhanced responsiveness to IL-21 and reduced responsiveness to IL-2.

Thus, in certain embodiments, the invention relates to a method for obtaining an engineered common gamma chain (γ_{c}) variant for selective cytokine activation, the method comprising the steps of: (a) introducing a plurality of polynucleotides encoding common gamma chain (γ_{c}) variants into a plurality of cells according to the invention; (b) contacting the cells obtained in step (a) with a first cytokine; (c) isolating cells expressing the polynucleotide encoding the fluorescent marker in response to the first cytokine; (d) contacting the cells isolated in step (c) with a second cytokine; (e) isolating cells that (i) do not express the polynucleotide encoding the fluorescent marker in response to the second cytokine; or (ii) show reduced expression of the polynucleotide encoding the fluorescent marker in response to the second cytokine in comparison to a cell comprising a polynucleotide encoding a reference common gamma chain (γ_{c}); and (f) obtaining an engineered common gamma chain (γ_{c}) variant for selective cytokine activation from the cells isolated in step (e).

Identifying engineered common gamma chain (γ_{c}) variants conferring enhanced responsiveness to a first cytokine and reduced responsiveness to a second cytokine may require at least one round of positive and one round of negative selection. Preferably, multiple rounds of selection at different cytokine concentrations are performed for each cytokine.

Instead of naturally occurring cytokines, the method according to the invention may also be used for identifying orthogonal cytokine - cytokine receptor pairs. For example, the cells may first be contacted with an inactive cytokine variant. Inactive cytokine variants are mutated variants of naturally occurring cytokines that can no longer bind to a cytokine receptor to induce a cellular response. The cell line according to the invention may be used to identify engineered common gamma chain (γ_{c}) variants that can confer responsiveness to an inactive cytokine variant, i.e. by isolating cells that express the fluorescent marker in the presence of the inactive cytokine variant. Cells that respond to the inactive cytokine variant may then be contacted in a second round with the active counterpart of the inactive cytokine variant. In this second round, cells that do not respond to the active counterpart can be selected (i.e. cells that do not express the fluorescent marker). As such, cells can be obtained that only respond to the inactive cytokine variant but not to the active counterpart.

Thus, in a particular embodiment, the invention relates to the method according to the invention, wherein the first cytokine is an inactive variant of a cytokine and wherein the second cytokine is an active variant of the first cytokine.

That is, in certain embodiments, the invention relates to a method for obtaining an engineered common gamma chain (γ_{c}) variant for selective cytokine activation, the method comprising the steps of: (a) introducing a plurality of polynucleotides encoding common gamma chain (γ_{c}) variants into a plurality of cells according to the invention; (b) contacting the cells obtained in step (a) with an inactive cytokine variant; (c) isolating cells expressing the polynucleotide encoding the fluorescent marker in response to the inactive cytokine variant; (d) contacting the cells isolated in step (c) with the active counterpart of said inactive cytokine variant; (e) isolating cells that (i) do not express the polynucleotide encoding the fluorescent marker in response to the active counterpart of said inactive cytokine variant; or (ii) show reduced expression of the polynucleotide encoding the fluorescent marker in response to the active counterpart of said inactive cytokine variant in comparison to a cell comprising a polynucleotide encoding a reference common gamma chain (γ_{c}); and (f) obtaining an engineered common gamma chain (γ_{c}) variant for selective cytokine activation from the cells isolated in step (e).

The inactive cytokine variant may be an inactive variant of IL-2, IL-4, IL-7, IL-9, IL-15 or IL-21. In certain embodiments, the method according to the invention may be used to identify an engineered common gamma chain (γ_{c}) variant that confers responsiveness to an inactive IL-2 variant and simultaneously abolishes or reduces responsiveness to IL-2. Thus, in a particular embodiment, the invention relates to the method according to the invention, wherein the first cytokine is an inactivated IL-2 variant and wherein the second cytokine is IL-2 comprising an amino acid sequence as set forth in SEQ ID NO:2.

Various inactive IL-2 variants are known in the art. In a particular embodiment, the invention relates to the method according to the invention, wherein the inactivated IL-2 variant comprises a mutation at position Leu18, Gln22, Arg38, Lys43, Glu61, Asn88, Val91, Thr123, Gln126, Ser127 and/or Ser130 of SEQ ID NO:2.

In a particular embodiment, the invention relates to the method according to the invention, wherein the reference common gamma chain (γ_{c}) comprises an amino acid sequence as set forth in SEQ ID NO:1.

Cells obtained in the final round of selection may be isolated and sequenced as described elsewhere herein. In particular, the cells obtained in the final round of selection may be subjected to deep sequencing and mutations that confer selective cytokine activation may be identified by bioinformatic methods, including the use of classification algorithms.

In a particular embodiment, the invention relates to the method according to the invention, wherein the cells in steps (c) and (e) are isolated by fluorescence-activated cell sorting (FACS) based on the expression level of the polynucleotide encoding the fluorescent marker.

In a particular embodiment, the invention relates to the method according to the invention, wherein the cells isolated in step (e) are subjected to sequencing, in particular to deep sequencing.

In a particular embodiment, the invention relates to the method according to the invention, wherein a classification algorithm is used to identify a nucleotide sequence encoding an engineered common gamma chain (γ_{c}) variant for selective cytokine activation from the sequencing results.

In a particular embodiment, the invention relates to the method according to the invention, wherein a classification algorithm is used to identify mutations contributing to the phenotype of the engineered common gamma chain (γ_{c}) variant for selective cytokine activation from the sequencing results.

In certain embodiments, the invention relates to a cytokine receptor comprising an engineered common gamma chain variant, wherein the engineered common gamma chain variant confers selective cytokine responsiveness to the cytokine receptor.

In certain embodiments, the invention relates to a cytokine receptor comprising an engineered common gamma chain variant, wherein the engineered common gamma chain variant confers enhanced responsiveness to IL-7 and IL-15.

In certain embodiments, the invention relates to a cytokine receptor comprising an engineered common gamma chain variant, wherein the engineered common gamma chain variant confers enhanced responsiveness to IL-15 and reduced responsiveness to IL-2.

In certain embodiments, the invention relates to a cytokine receptor comprising an engineered common gamma chain variant, wherein the engineered common gamma chain variant confers enhanced responsiveness to IL-7 and reduced responsiveness to IL-2.

In certain embodiments, the invention relates to a cytokine receptor comprising an engineered common gamma chain variant, wherein the engineered common gamma chain variant confers enhanced responsiveness to IL-21 and reduced responsiveness to IL-2.

In certain embodiments, the invention relates to a cytokine receptor comprising an engineered common gamma chain variant, wherein the engineered common gamma chain variant confers enhanced responsiveness to an inactivated IL-2 variant and reduced responsiveness to IL-2.

The cell line according to the invention may not only be used for identifying and obtaining engineered common gamma chain (γ_{c}) variants that alter the responsiveness of cytokine receptors, but also for identifying chimeric receptors comprising a cytoplasmic domain of a common gamma chain (γ_{c}). That is, the cytoplasmic portion of the common gamma chain (γ_{c}), which comprises the elements for JAK/STAT-mediated signal transduction, may be fused to the extracellular part of another receptor. Binding of the extracellular part of the chimeric receptor by a suitable ligand will activate JAK/STAT-mediated signaling, resulting in the expression of the fluorescent marker in the cell line according to the invention. As such, the cell line according to the invention may be used to identify functional chimeric receptors in a high-throughput manner.

That is, in a particular embodiment, the invention relates to a method for obtaining a chimeric common gamma chain (γ_{c}) receptor comprising a cytoplasmic portion of a common gamma chain (γ_{c}) receptor and an extracellular portion of a receptor protein, the method comprising the steps of: (a) introducing a plurality of polynucleotides encoding chimeric common gamma chain (γ_{c}) receptor candidates into a plurality of cells according to the invention; (b) contacting the cells obtained in step (a) with a ligand of the receptor protein; (c) isolating cells expressing the polynucleotide encoding the fluorescent marker in response to the ligand of the receptor protein; and (d) obtaining a chimeric common gamma chain (γ_{c}) receptor from the cells isolated in the (c).

Within the present invention, a *"chimeric common gamma chain (γ_{c}) receptor"* is an integral membrane protein comprising a cytoplasmic portion of a common gamma chain (γ_{c}) and an extracellular portion of another receptor.

The cytoplasmic portion of the common gamma chain (γ_{c}) may be the entire cytoplasmic domain of the common gamma chain (γ_{c}) or may be a part of the cytoplasmic domain of the common gamma chain (γ_{c}). In embodiments where the cytoplasmic portion of the common gamma chain (γ_{c}) is only a portion of the cytoplasmic domain of the common gamma chain (γ_{c}), it is preferred that the portion of the cytoplasmic domain of the common gamma chain (γ_{c}) retains the ability to induce JAK-STAT mediated signaling.

That is, the cytoplasmic portion of the chimeric common gamma chain (γ_{c}) receptor preferably comprises amino acid residues Arg263 to Lys272, Thr279 to Trp288, Leu299 to Cys308 and/or Ser333 to Leu343 of SEQ ID NO:1. In a more preferred embodiment, the cytoplasmic portion of the chimeric common gamma chain (γ_{c}) receptor comprises the entire cytoplasmic domain of a common gamma chain (γ_{c}). Thus, in a particular embodiment, the invention relates to the method according to the invention, wherein the chimeric common gamma chain (γ_{c}) receptor comprises amino acid residues Glu 262 to Thr 347 of SEQ ID NO:1 (corresponding to SEQ ID NO:3).

In a particular embodiment, the chimeric common gamma chain (γ_{c}) receptor comprises the cytoplasmic and the transmembrane domain of a common gamma chain (γ_{c}). Thus, in a particular embodiment, the invention relates to the method according to the invention, wherein the chimeric common gamma chain (γ_{c}) receptor comprises amino acid residues Val 241 to Thr 347 of SEQ ID NO:1 (corresponding to SEQ ID NO:4).

In certain embodiments, the cytoplasmic domain of the chimeric common gamma chain (γ_{c}) receptor candidate may comprise mutations to fine tune JAK/STAT-mediated signal transduction. That is, in certain embodiments, the chimeric common gamma chain (γ_{c}) receptor candidate may comprise an amino acid sequence having at least 70%, 75%, 80%, 85%, 90% or 95% sequence identity to SEQ ID NO:3 (cytoplasmic domain of human γ_{c}). In certain embodiments, the chimeric common gamma chain (γ_{c}) receptor candidate may comprise an amino acid sequence having at least 70%, 75%, 80%, 85%, 90% or 95% sequence identity to SEQ ID NO:4 (cytoplasmic and transmembrane domain of human γ_{c}).

The term "*sequence identity*" as used herein refers to the percentage of sequence identity between two polypeptide sequences or two nucleic acid sequences. To determine the percent identity of two amino acid sequences or of two nucleic acid sequences, the sequences are aligned for optimal comparison purposes (e.g. gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e. % identity=number of identical overlapping positions/total number of positions × 100%). Methods and tools for calculating sequence identity are well known in the art.

As described herein above, residues Arg263 - Lys272, Thr279 - Trp288, Leu299 - Cys308 and Ser 333 - Leu 343 of SEQ ID NO:1 have been described to be involved in JAK/STAT-mediated signaling. In certain embodiments, one or more of these residues may be mutated in order to fine tune signal transduction of the chimeric common gamma chain (γ_{c}) receptor. Thus, in a particular embodiment, the invention relates to the method according to the invention, wherein at least one amino acid residue in position Arg263 to Lys272, Thr 279 to Trp 288, Leu 299 to Cys 308 and/or Ser 333 to Leu 343 of SEQ ID NO:1 is mutated.

The extracellular portion of the chimeric common gamma chain (γ_{c}) receptor is preferably an extracellular portion of a receptor that retains the ability to bind to its cognate ligand. In certain embodiments, the extracellular portion of the chimeric common gamma chain (γ_{c}) receptor comprises the entire extracellular domain of a receptor.

In a preferred embodiment, the chimeric common gamma chain (γ_{c}) receptor comprises a cytoplasmic portion of a human common gamma chain (γ_{c}), wherein the cytoplasmic portion of the human common gamma chain (γ_{c}) retains the ability to induce JAK/STAT-mediated signaling, and an extracellular portion of another human receptor, wherein the extracellular portion of the other human receptor retains the ability to bind to its cognate ligand.

In certain embodiments, the chimeric common gamma chain (γ_{c}) receptor comprises an extracellular portion of a human Fas receptor. In certain embodiments, the chimeric common gamma chain (γ_{c}) receptor comprises an extracellular portion of a human Fas receptor, wherein the extracellular portion of the human Fas receptor retains the ability to bind to the Fas ligand.

In certain embodiments, the chimeric common gamma chain (γ_{c}) receptor comprises the extracellular domain of the human Fas receptor. Thus, in a particular embodiment, the invention relates to the method according to the invention, wherein the chimeric common gamma chain (γ_{c}) receptor comprises an extracellular domain of a Fas receptor, or a portion thereof that retains the ability to bind to the Fas ligand.

In certain embodiments, the extracellular domain of the chimeric common gamma chain (γ_{c}) receptor comprises an amino acid sequence as set forth in SEQ ID NO:5 (extracellular domain of FasR). In certain embodiments, the extracellular domain of the chimeric common gamma chain (γ_{c}) receptor comprises an amino acid sequence having at least 70%, 75%, 80%, 85%, 90% or 95% sequence identity to SEQ ID NO:5 (extracellular domain of FasR).

The transmembrane domain of the chimeric common gamma chain (γ_{c}) receptor may be any suitable transmembrane domain known in the art. That is, the transmembrane domain does not necessarily need to be of the same origin as the cytoplasmic domain and/or the extracellular domain. However, in certain embodiments, the transmembrane domain is of the same origin as the cytoplasmic domain. That is, in certain embodiments, the transmembrane domain is the transmembrane domain of a common gamma chain (γ_{c}). In certain embodiments, the transmembrane domain is of the same origin as the extracellular domain. In certain embodiments, the transmembrane domain is the transmembrane domain of a Fas receptor.

The skilled person is aware of methods to obtain a polynucleotide encoding a chimeric common gamma chain (γ_{c}) receptor candidate. For example, a polynucleotide encoding a chimeric common gamma chain (γ_{c}) receptor candidate may be designed computationally by combining nucleotide sequences encoding an extracellular portion of a receptor, a cytoplasmic portion of a common gamma chain (γ_{c}) and a suitable transmembrane domain. The computationally designed polynucleotide may then be synthesized chemically.

Alternatively or in addition, libraries of polynucleotides encoding chimeric common gamma chain (γ_{c}) receptor candidates may be designed computationally, for example by combining extracellular and cytoplasmic portions of different sizes and/or origin.

Further, libraries of polynucleotides encoding chimeric common gamma chain (γ_{c}) receptor candidates may be obtained by introducing mutations into a template polynucleotide encoding a chimeric common gamma chain (γ_{c}) receptor candidate as described elsewhere herein.

Using libraries of polynucleotides encoding chimeric common gamma chain (γ_{c}) receptor candidates has the advantage that high numbers of candidates can be tested in a single experiment.

Alternatively, polynucleotides encoding chimeric common gamma chain (γ_{c}) receptor candidates or libraries of polynucleotides encoding a chimeric common gamma chain (γ_{c}) receptor candidates may be obtained by conventional cloning as known in the art.

The polynucleotide encoding a chimeric common gamma chain (γ_{c}) receptor candidate may be integrated into the genome of the cell line according to the invention as described herein above. Thus, in a particular embodiment, the invention relates to the method according to the invention, wherein the polynucleotide encoding the synthetic common gamma chain (γ_{c}) receptor is integrated into the genome of the cell by means of genome editing.

In a particular embodiment, the invention relates to the method according to the invention, wherein genome editing involves a sequence-specific endonuclease, in particular wherein the sequence-specific endonuclease is a CRISPR-associated (Cas) protein, in particular wherein the CRISPR-associated (Cas) protein is Cas9.

Cells comprising a polynucleotide encoding a chimeric common gamma chain (γ_{c}) receptor candidate are then contacted with a suitable ligand. Preferably, the ligand is a cognate ligand of the receptor from which the extracellular portion has been obtained. In certain embodiments, the ligand is a Fas ligand.

Cell expressing a functional chimeric common gamma chain (γ_{c}) receptor can be identified and or isolated based on the expression level of the fluorescent marker as described herein. Thus, in a particular embodiment, the invention relates to the method according to the invention, wherein the cells in step (c) are isolated by fluorescence-activated cell sorting (FACS) based on the expression level of the polynucleotide encoding the fluorescent marker.

Further, functional chimeric common gamma chain (γ_{c}) receptors or specific mutations that result in an optimized chimeric common gamma chain (γ_{c}) receptor may be identified by sequencing, in particular deep sequencing. Thus, in a particular embodiment, the invention relates to the method according to the invention, wherein the cells isolated in step (c) are subjected to sequencing, in particular to deep sequencing.

That is, cells expressing a chimeric common gamma chain (γ_{c}) receptor may be isolated and subjected to sequencing. For example, a polynucleotide encoding an entire chimeric common gamma chain (γ_{c}) receptor may be sequenced by Sanger sequencing.

In certain embodiments, a chimeric common gamma chain (γ_{c}) receptor may be optimized by deep mutational scanning, for example by performing site-saturation mutagenesis of one or more of the residues of the cytoplasmic domain of the common gamma chain that have been disclosed herein. In such embodiments, a plurality of optimized chimeric common gamma chain (γ_{c}) receptors resulting in the same phenotype may be isolated by flow cytometry.

To identify specific chimeric common gamma chain (γ_{c}) receptor from this plurality of optimized chimeric common gamma chain (γ_{c}) receptors, single cells may be further isolated and subjected to sequencing in order to identify the entire sequence of the chimeric common gamma chain (γ_{c}) receptor encoded in the isolated cell.

However, it is more preferred that the entire population of cells encoding optimized chimeric common gamma chain (γ_{c}) receptors is subjected to deep sequencing. Subsequently, specific mutations that result in a desired phenotype may be identified by read frequency enrichment and/or with a classification algorithm as disclosed elsewhere herein.

In a particular embodiment, the invention relates to the method according to the invention, wherein a classification algorithm is used to identify a synthetic common gamma chain (γ_{c}) receptor from the obtained sequencing results.

In a particular embodiment, the invention relates to the method according to the invention, wherein a classification algorithm is used to identify mutations contributing to the phenotype of the chimeric common gamma chain (γ_{c}) receptor from the obtained sequencing results.

Another aspect of the invention relates to a chimeric common gamma chain (γ_{c}) receptor, in particular a chimeric common gamma chain (γ_{c}) receptor that has been obtained with the method according to the invention.

In certain embodiments, the cytoplasmic domain of the chimeric common gamma chain (γ_{c}) receptor may comprise an amino acid sequence having at least 70%, 75%, 80%, 85%, 90% or 95% sequence identity to SEQ ID NO:3 (cytoplasmic domain of human γ_{c}). In certain embodiments, the cytoplasmic domain of the chimeric common gamma chain (γ_{c}) receptor may comprise an amino acid sequence having at least 70%, 75%, 80%, 85%, 90% or 95% sequence identity to SEQ ID NO:4 (cytoplasmic and transmembrane domain of human γ_{c}). In certain embodiments, the cytoplasmic domain of the chimeric common gamma chain (γ_{c}) receptor may comprise amino acid residues Ser 241 to Thr 347 of SEQ ID NO:1.

In certain embodiments, the cytoplasmic domain of the chimeric common gamma chain (γ_{c}) receptor may comprise amino acid residues Ser 241 to Thr 347 of SEQ ID NO:1, wherein at least one amino acid residue at position Arg263 to Lys272, Thr 279 to Trp 288, Leu 299 to Cys 308 and/or Ser 333 to Leu 343 of SEQ ID NO:1 is mutated.

In a particular embodiment, the invention relates to a chimeric receptor comprising a cytoplasmic portion of a common gamma chain (γ_{c}) and an extracellular portion of a Fas receptor.

That is, in certain embodiments, the extracellular domain of the chimeric common gamma chain (γ_{c}) receptor comprises an amino acid sequence as set forth in SEQ ID NO:5 (extracellular domain of FasR). In certain embodiments, the extracellular domain of the chimeric common gamma chain (γ_{c}) receptor comprises an amino acid sequence having at least 70%, 75%, 80%, 85%, 90% or 95% sequence identity to SEQ ID NO:5 (extracellular domain of FasR).

Another aspect of the invention relates to an isolated cell comprising one or more cytokine receptors, wherein the one or more cytokine receptors comprise an engineered common gamma chain (γ_{c}) variant, and wherein the engineered common gamma chain (γ_{c}) variant alters the responsiveness of at least one cytokine receptor towards its cognate cytokine.

That is, the invention relates to a cell encoding an engineered common gamma chain (γ_{c}) variant, in particular an engineered common gamma chain (γ_{c}) variant that has been identified with the method according to the invention.

In certain embodiments, the engineered common gamma chain (γ_{c}) variant has been derived from a human common gamma chain (γ_{c}) having an amino acid sequence as set forth in SEQ ID NO:1. Preferably, the engineered common gamma chain (γ_{c}) variant comprises one or more mutations in any one of the positions of SEQ ID NO:1 that have been disclosed herein.

The cell that comprises the engineered common gamma chain (γ_{c}) variant may be any cell. In certain embodiments, the cell is a T cell. As such, by replacing the common gamma chain (γ_{c}) of a T cell with an engineered common gamma chain (γ_{c}) variant, T cells having a modified cytokine responsiveness can be obtained.

### BRIEF DESCRIPTION OF THE DRAWING

**Figure 1****.** Overview of the GCR-Engine pipeline. GnR cells enable CRISPR-targeted mutagenesis and high-throughput functional screening of the common gamma chain cytokine receptor (GCR). Highly-diverse GCR libraries are displayed on GnR cells and subjected to stimulation with selected cytokines (e.g.; IL-2). GnR-GCR cells with desired signalling properties are next isolated by FACS and their transgenic GCR locus is amplified by PCR for deep sequencing. Computational analysis of read frequency enrichment is applied to identify GCR variants with predicted enhanced responsiveness to a particular cytokine. Selected GCR variants are expressed in primary human T cells which are next stimulated with the cytokine of interest and single-cell sequencing is applied in order to identify GCR variants promoting favourable T-cell phenotypes.

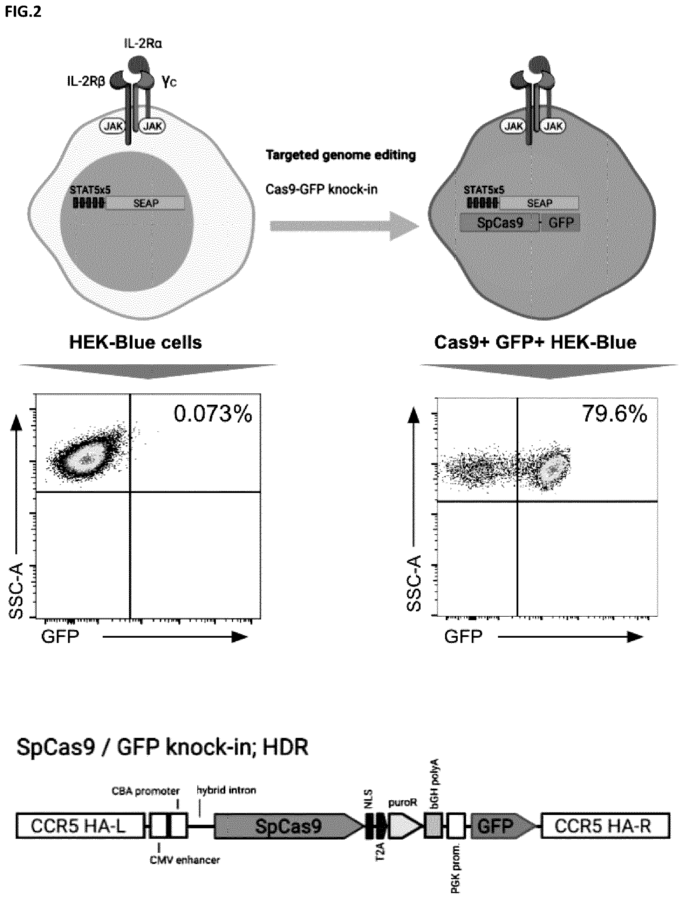

**Figure 2****.** CRISPR-targeted integration of a Cas9 transgene into HEK-Blue cells. HEK-Blue cells, expressing the complete trimeric IL-2 receptor, were transfected with a gRNA targeting the CCR5 locus as well as a double-stranded DNA homology-directed repair (HDR) template mapping to the CCR5 locus. Next, a single-cell clone expressing GFP (an integration marker) was isolated by FACS.

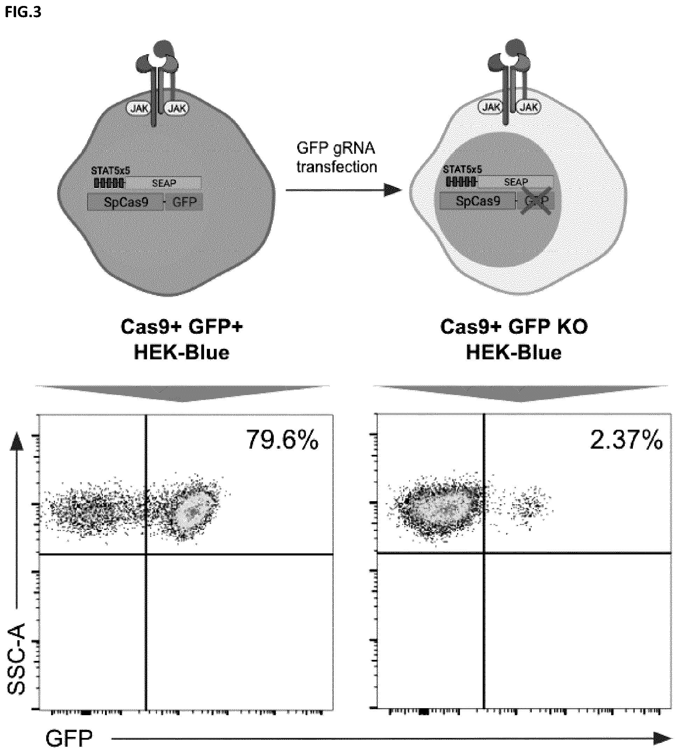

**Figure 3****.** Validation of transgenic Cas9 activity by CRISPR-targeted GFP knockout. Cas9+. GFP+ HEK-Blue cells were electroporated with a gRNA targeting the GFP transgene in the absence of exogenous Cas9. Flow cytometry plots of control electroporation (bottom left) and GFP gRNA electroporation (bottom right) on day 5 post-transfection are shown.

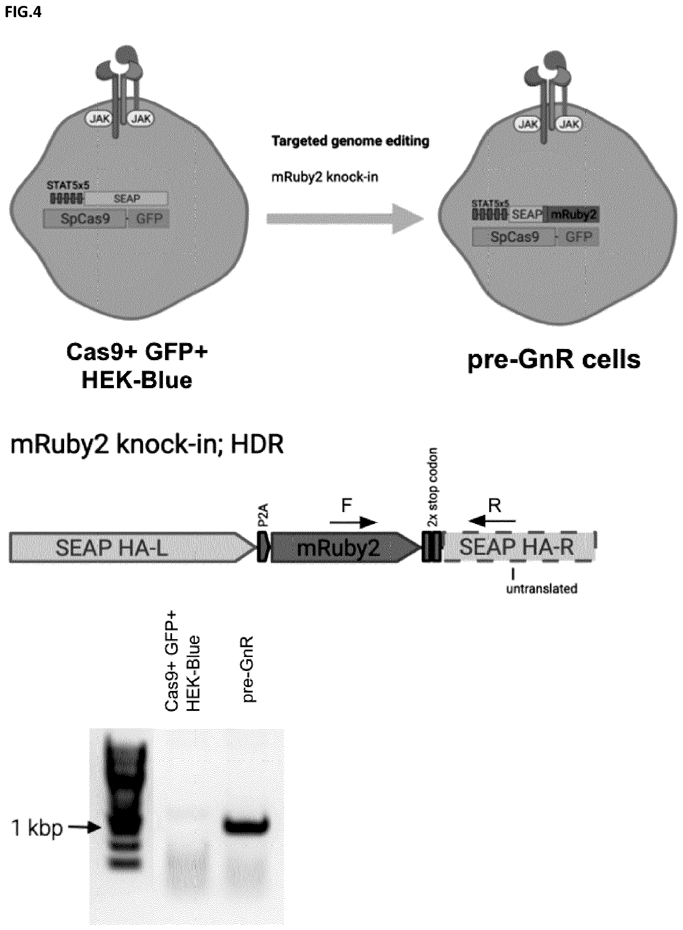

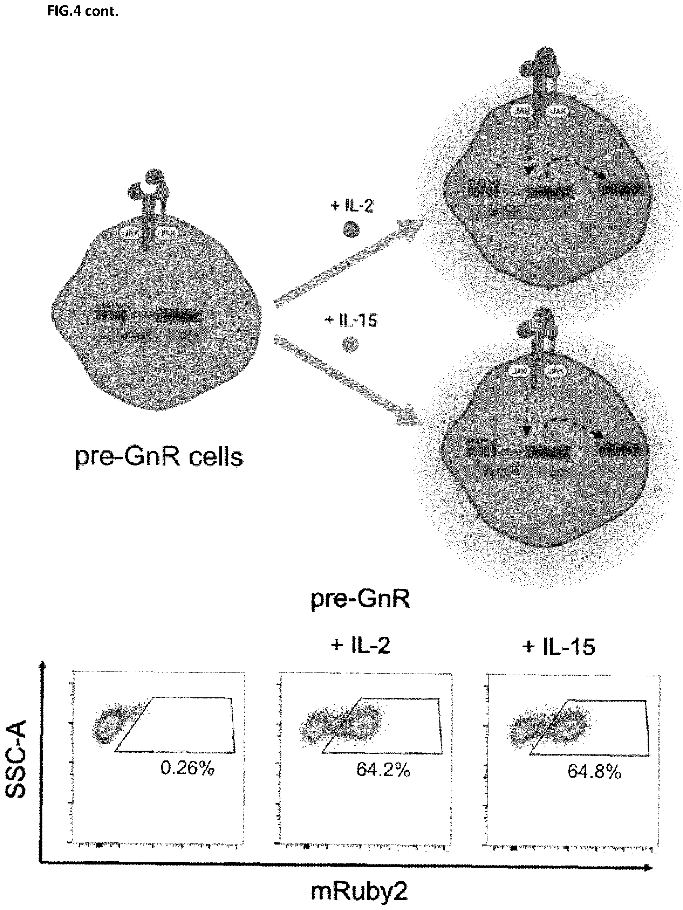

**Figure 4****.** Generation of the pre-GnR reporter cell line. Cas9+ GFP+ HEK-Blue cells were transfected with a gRNA targeting the secreted alkaline phosphatase (SEAP) open reading frame and an HDR template mapping to the central region of SEAP. Transfectants were stimulated with IL-2 on day 7 post-transfection and mRuby2 positive cells were single-cell sorted by FACS. After re-stimulation with IL-2 or IL-15, the clone with the highest mRuby2 response was chosen as the pre-GnR reporter cell line. Integration of the mRuby2 transgene was validated by genomic PCR amplification using a forward primer annealing to SEAP and a reverse primer annealing to mRuby2.

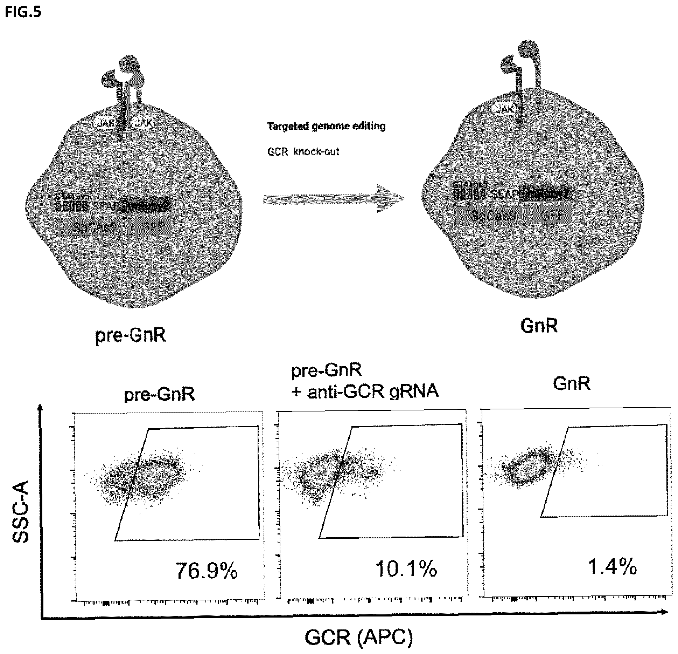

**Figure 5****.** Generation of GnR cells through disruption of endogenous GCR expression. Pre-GnR cells were transfected with a gRNA targeting the GCR ORF. On day 5, transfectants were stained for GCR surface display and single GCR-negative cells were isolated by FACS.

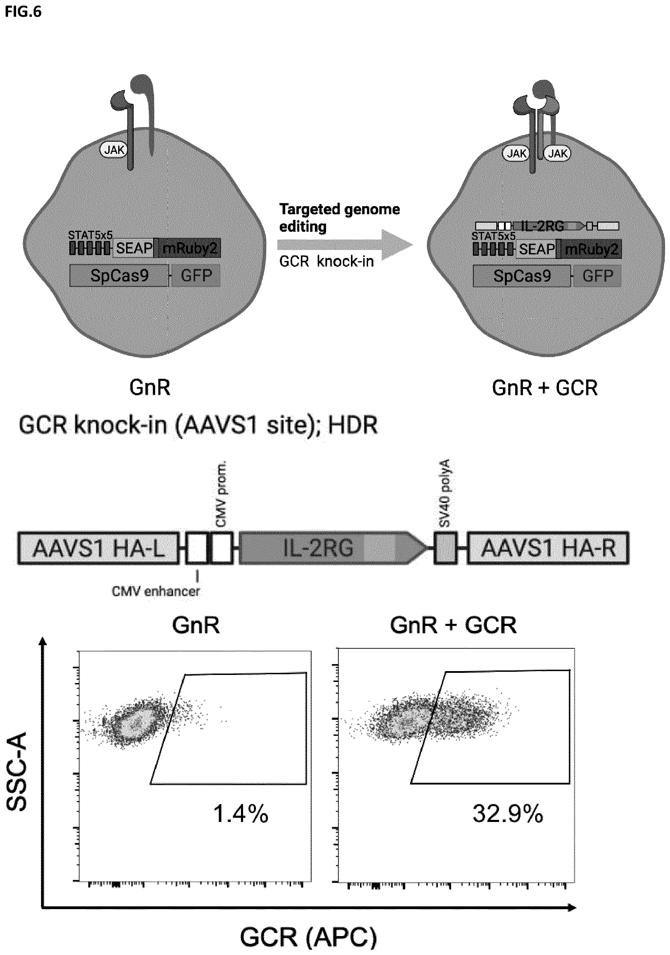

**Figure 6****.** CRISPR-targeted reconstitution of GnR cells with transgenic wild-type GCR. GnR cells were transfected with a gRNA targeting the AAVS1 safe harbour locus and a dsDNA HDR template encoding the complete wild-type GCR open reading frame flanked by homology arms mapping to the AAVS1 locus. On day 7 post-transfection, transfectants were assessed for GCR surface expression by flow cytometry.

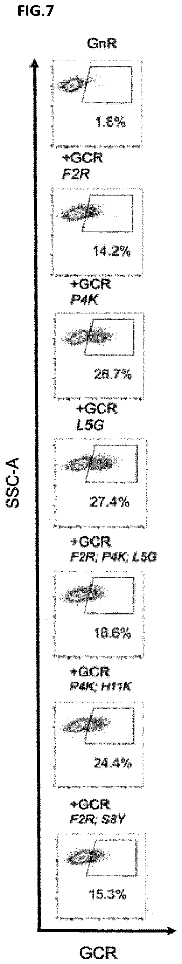

**Figure 7****.** CRISPR-targeted reconstitution of GnR cells with transgenic GCR variants. GnR cells were transfected with a gRNA targeting the AAVS1 locus and HDR templates encoding six GCR variants. On day 7 post-transfection, transfectants were assessed for GCR surface expression by flow cytometry.

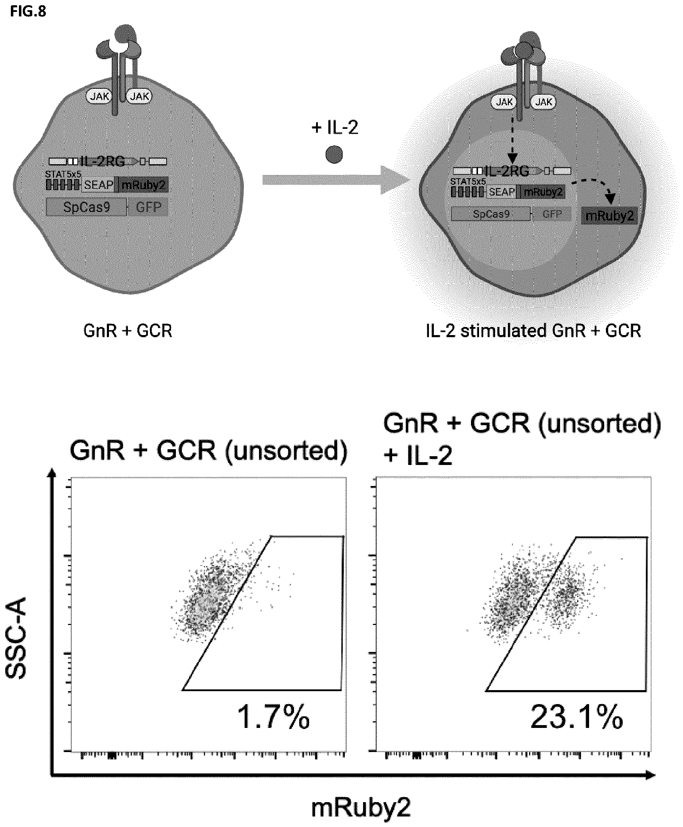

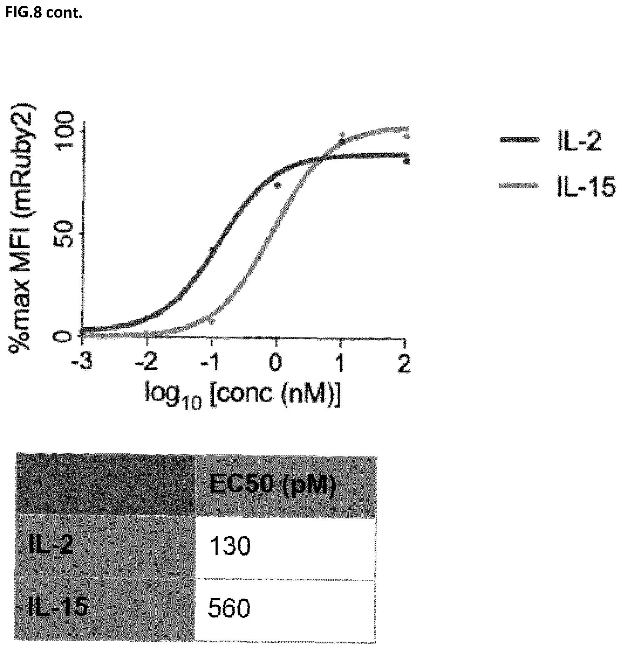

**Figure 8****.** Assessment of GnR platform functionality. Unsorted GnR cells with reconstituted expression of wild type GCR from the AAVS1 locus (GnR + GCR) were stimulated with 5 nM IL-2 for 24 h. Following this, the expression of mRuby2 was assessed by flow cytometry. GnR + GCR cells were stimulated with serially diluted IL-2 or IL-15. mRuby expression was assessed after 24 h by FACS and the EC50 values for IL-2 and IL-15 were calculated based on the response curves.

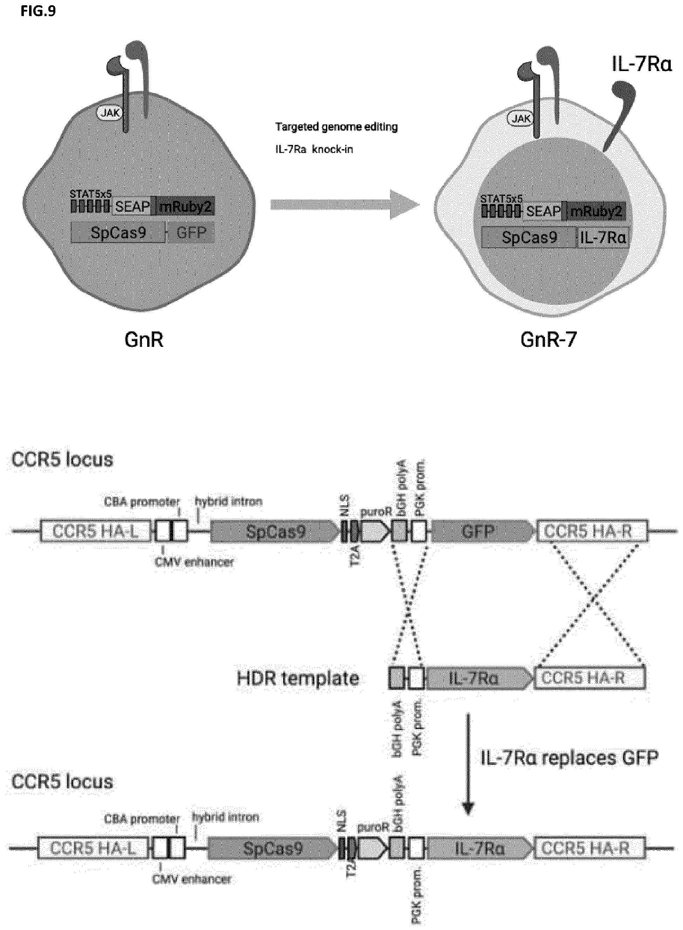

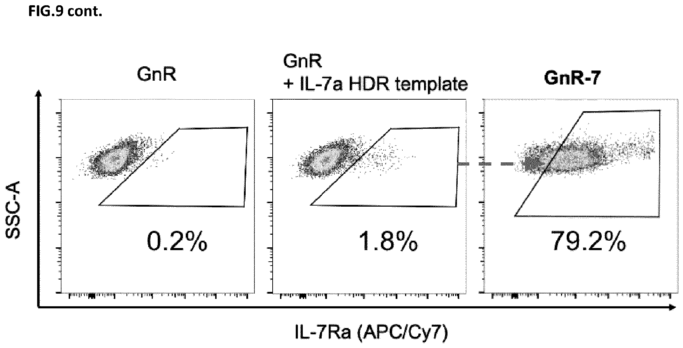

**Figure 9****.** Development of IL-7 responsive GnR cells (GnR-7). GnR cells were transfected with a gRNA targeting the GFP locus and an HDR template containing the complete IL-7 receptor alpha (IL-7Rα) open reading frame mapping to the GFP locus. Transfectants were immunostained for IL-7Rα surface expression and positive single-cell clones were next isolated by FACS. The clone with the most abundant IL-7Ra expression was chosen as the GnR-7 cell line.

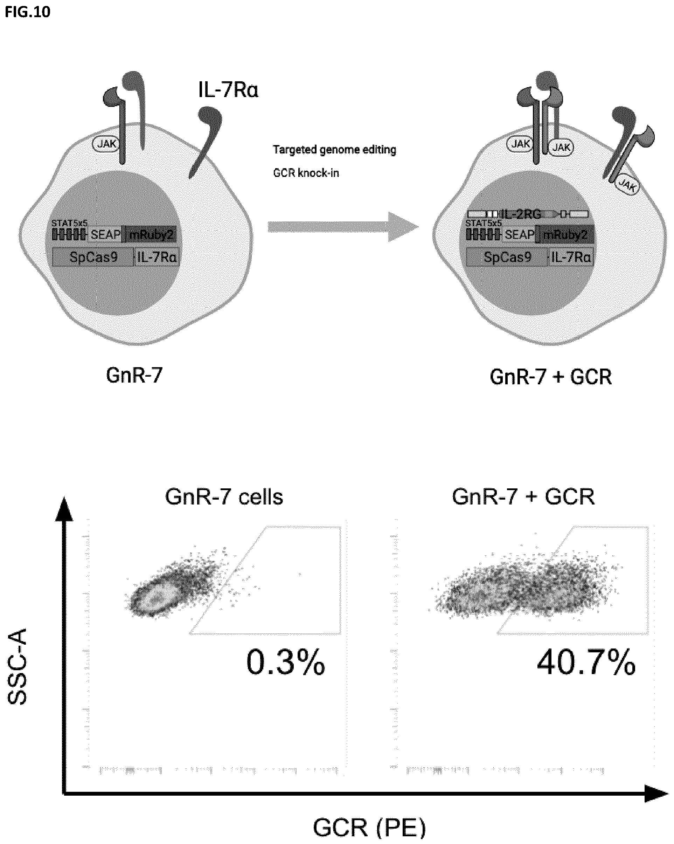

**Figure 10****.** CRISPR-targeted reconstitution of GnR-7 cells with transgenic GCR. GnR-7 cells were transfected with a gRNA targeting the AAVS1 safe harbour locus and an HDR template encoding the complete wild-type GCR open reading frame flanked by homology arms mapping to the AAVS1 locus. On day 7 post-transfection, transfectants were assessed for GCR surface expression by flow cytometry.

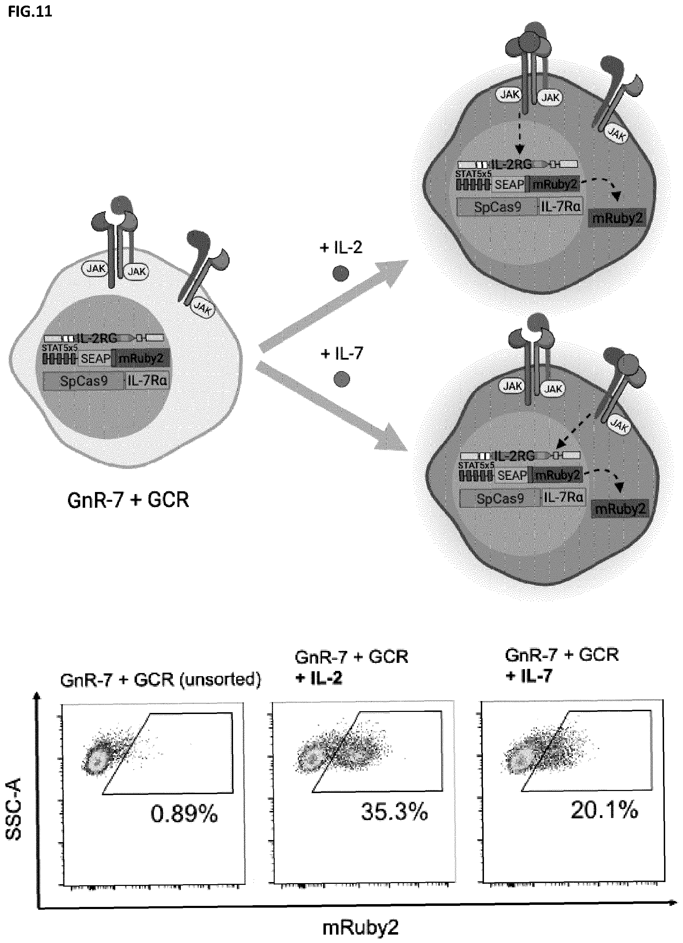

**Figure 11****.** Assessment of GnR-7 platform functionality. Unsorted GnR-7 cells with reconstituted expression of wild-type GCR from the AAVS1 locus (GnR-7 + GCR) were stimulated with 10 nM IL-2 for 24 h. Following this, the expression of mRuby2 was assessed by flow cytometry.

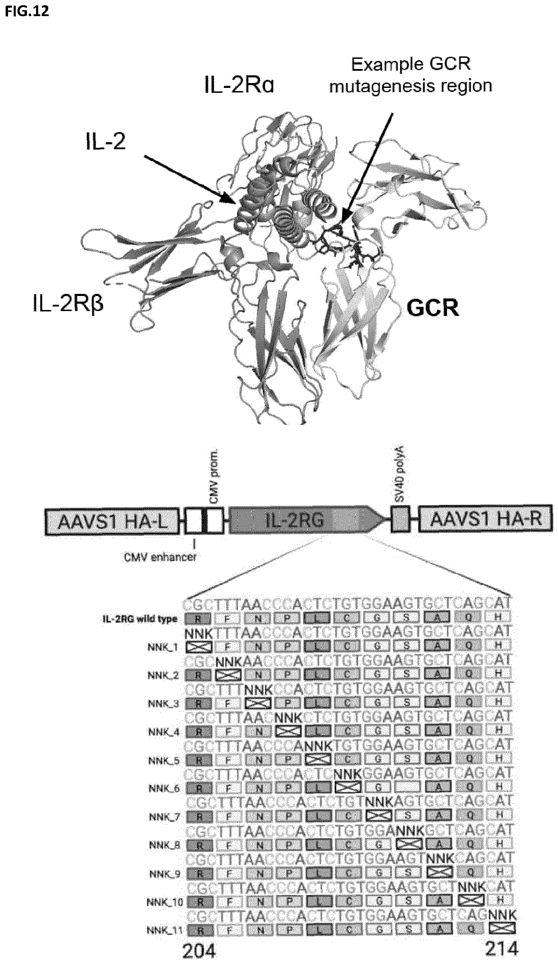

**Figure 12****.** Design of a deep mutational scanning (DMS) library. The crystal structure of the IL-2/IL-2R quaternary complex was analysed in order to identify GCR residues mediating contacts with IL-2. Five contiguous residues forming contacts with IL-2 were identified, namely residues Pro 207 - Ser 211. A single-site saturation mutagenesis library using the NNK codon scheme was designed to target eleven contiguous residues (i.e., Arg 204 - His 214) containing the five highlighted residues of interest (resulting in SEQ ID NO:17 to 40).

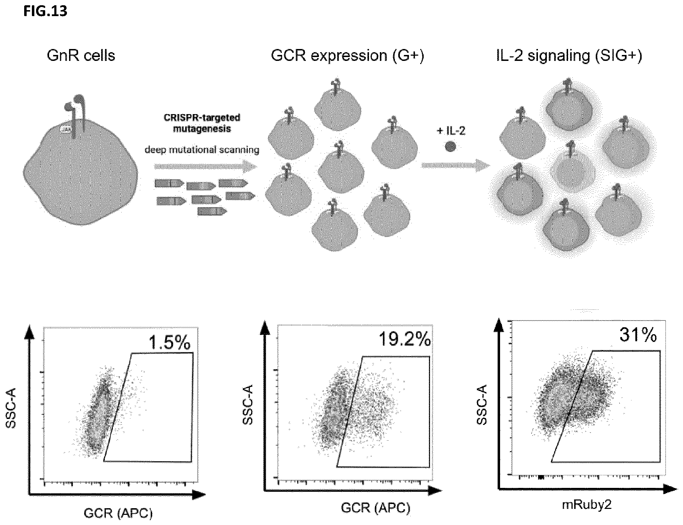

**Figure 13****.** Functional screening of DMS libraries. The GCR DMS library was integrated into the AAVS1 locus of GnR cells via CRISPR-targeted genome editing. On day 7 post-transfection, the cells were immunostained for GCR surface expression GCR and GCR-positive GnR cells were enriched by FACS (G+ fraction). Next, G+ cells were expanded in culture and stimulated with 5 nM IL-2 for 24 h. Activated mRuby2-positive cells were next isolated by FACS (SIG+ fraction).
**Figure 14****.** Validation of deep mutational scanning data. Genomic DNA was extracted from G+ and SIG+ cells and their transgenic GCR locus amplified by PCR for deep sequencing. Sequencing data was then used for calculating the amino acid enrichment ratios of G+ and SIG+ relative to the original plasmid DMS library. Analysis of SIG+ enrichment data identified two single mutations that independently enhanced IL-2-induced signalling - P207K and H214K (of SEQ ID NO:1). P207K and H214K were combined to generate a GCR double mutant (Enhanced 5), which displays enhanced maximal signalling following IL-2 stimulation relative to wild-type GCR. Signalling capacity was calculated based on the proportion of GCR-positive cells expressing mRuby2, and data was normalised to the highest response observed for GnR-GCR cells expressing wild-type GCR.

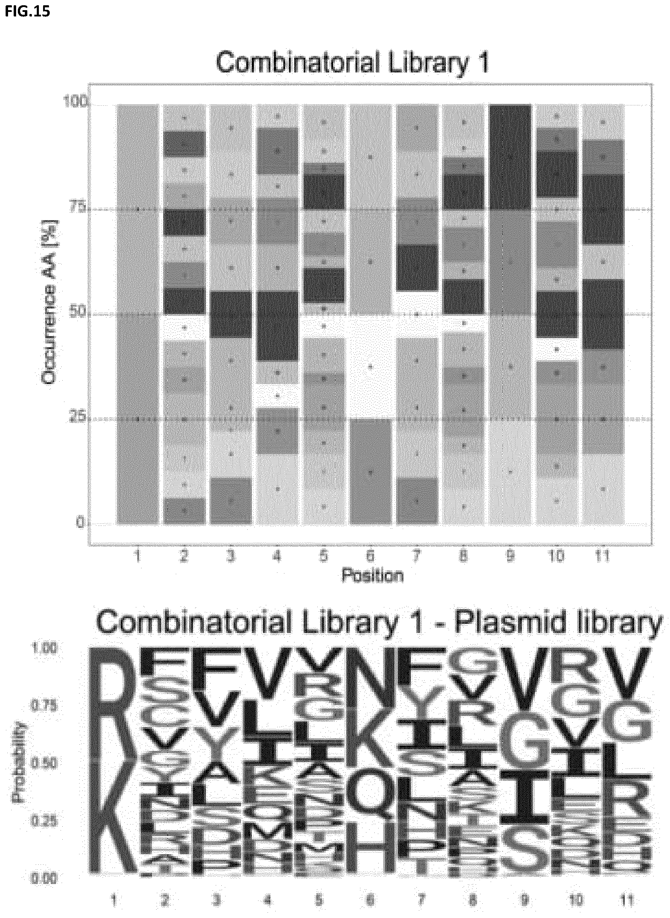

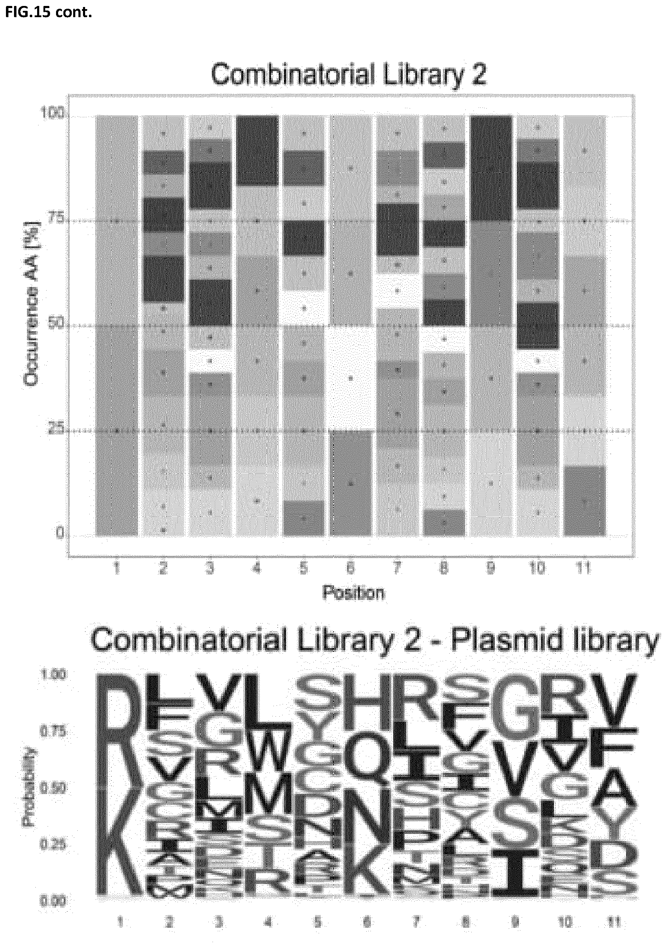

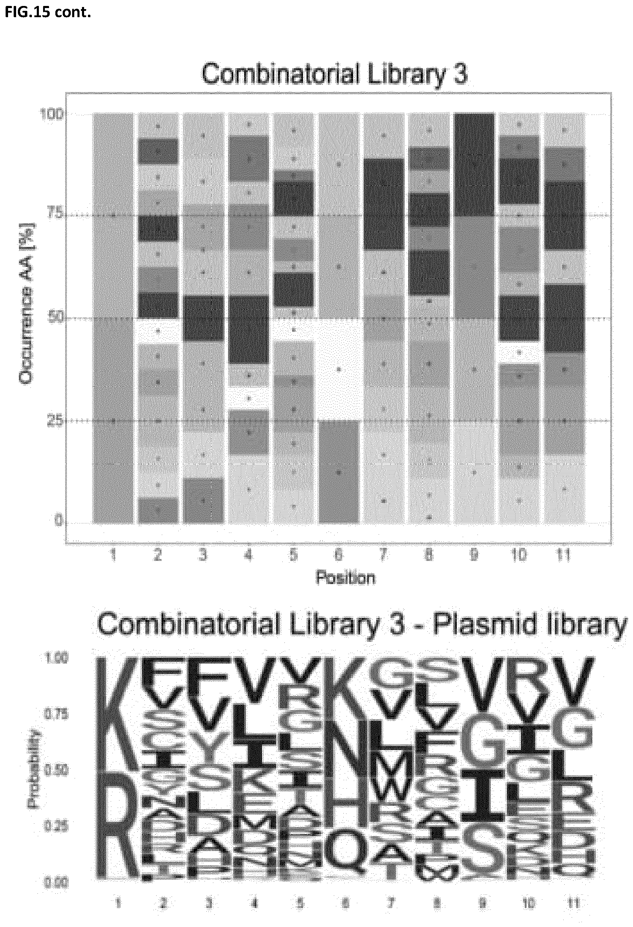

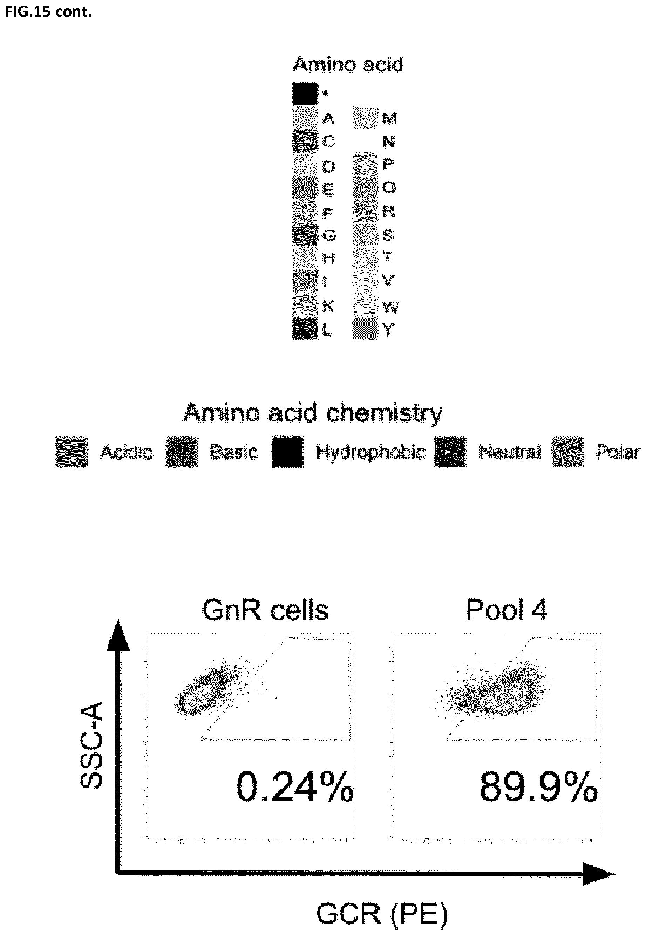

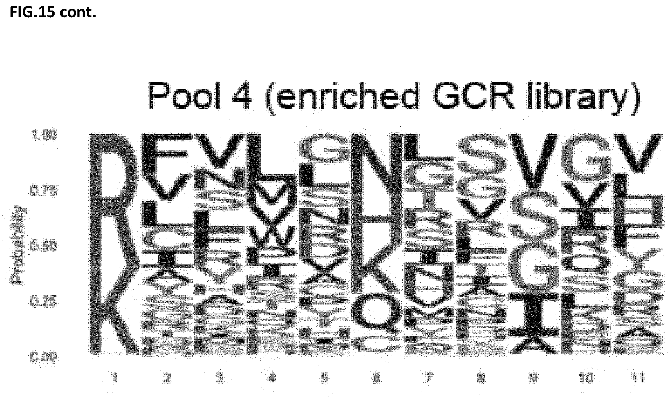

**Figure 15****.** Design and generation of combinatorial mutagenesis libraries. Read frequency enrichment data obtained from DMS of GCR expressibility (G+) was used to computationally design three combinatorial mutagenesis libraries spanning GCR positions Arg 204 - His 214, each with a theoretical diversity of 109 variants. A sequence space optimization algorithm (Mason et al., Nucleic Acids Research, 2018. 46(14), 7436-7449) was applied to identify degenerate codons that mimic the GCR single mutant frequencies found in the G+ fraction. Following assembly into a GCR-encoding plasmid, combinatorial libraries were deep sequenced for experimental validation of their design (data presented as sequence logos). Combinatorial GCR linrarie were each individually integrated into GnR cells by means of CRISPR-targeted genome editing. Following expansion in culture (7 days), transfectants were assessed for GCR surface expression by flow cytometry and combined into a single GnR pool. The combined pool was enriched for GCR surface expression by performing four consecutive rounds of FACS. The resulting GnR pool enriched for GCR-positive cells (i.e., 'Pool 4', > 90% GCR-positive) was subjected to genomic PCR amplification of the GCR transgenic locus for deep sequencing, revealing high levels of sequence diversity (data presented as a sequence logo).

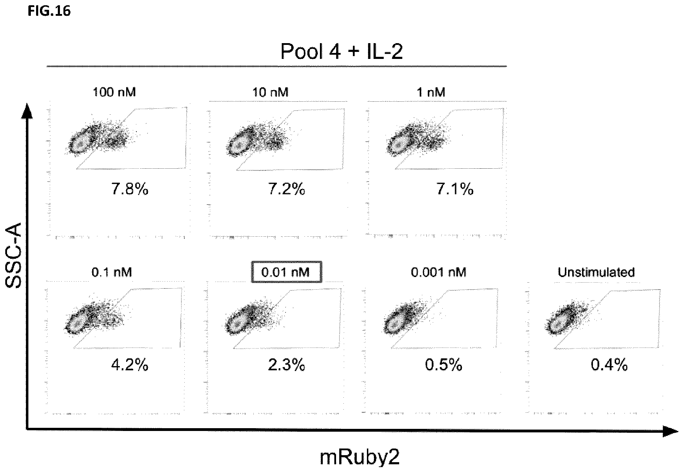

**Figure 16****.** Assessment of IL-2 signalling in a GnR-GCR pool enriched for surface GCR expression. Pool 4 cells with enriched GCR surface expression were stimulated with concentrations of IL-2 ranging from 0.001 nM to 100 nM for 24 hours, followed by flow cytometry assessment of mRuby2 expression.

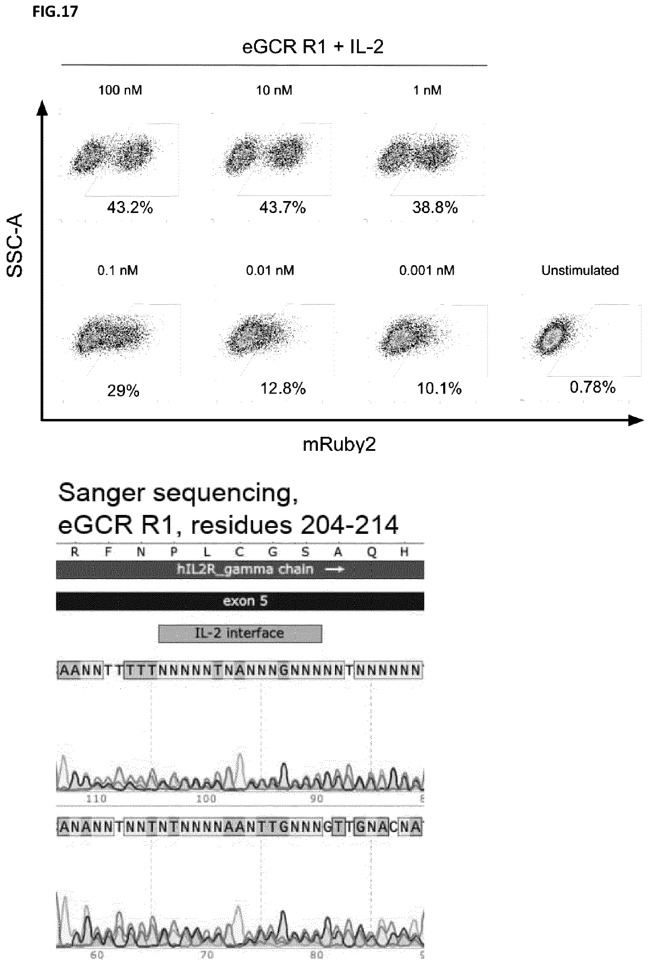

**Figure 17****.** Characterisation of a GnR-GCR pool containing GCR variants with enhanced IL-2 sensitivity. Pool 4 GnR-GCR cells were subjected to overnight stimulation with 0.01 nM IL-2, followed by FACS enrichment of activated mRuby2+ cells, resulting in a new pool of high-sensitivity GnR-GCR cells (i.e, pool eGCR R1). eGCR R1 cells were expanded in culture and similarly stimulated with concentrations of IL-2 ranging from 0.001 nM to 100 nM, revealing superior sensitivity to not only 0.01 nM, but also to 0.001 nM of IL-2. eGCR R1 cells were subjected to genomic PCR amplification of their transgenic GCR locus for Sanger sequencing.

### EXAMPLES

### Example 1: Generation of a reporter cell line with constitutive Cas9 expression

In order to generate the GnR cell line, the inventors started with a derivative of the human endothelial kidney 293 (HEK-293) cell line, namely HEK-Blue^{™} IL-2 (Invivogen, USA), that constitutively expresses all subunits of the high-affinity human trimeric IL-2 receptor (IL-2Rα, IL2Rβ and GCR) as well as IL-2 intracellular signalling pathway components (JAK3 and STAT5). HEK-Blue cells harbour a reporter of IL-2 signalling consisting of five tandem STAT5 response elements driving the expression of secreted embryonic alkaline phosphatase (SEAP) upon IL-2 as well as IL-15 stimulation. Since SEAP is an unsuitable phenotypic marker for high-throughput screening and isolation of activated cells (FACS), the inventors conducted multiple steps of CRISPR-Cas9 genome editing to engineer a cell line with transgenic expression of Cas9 endonuclease and that harbours a fluorescent reporter of IL-2 signalling.

HEK-Blue cells were first engineered to stably express Cas9 in order to increase the efficiency of subsequent CRISPR-Cas9 genome editing steps (**Fig. 2**). To this end, HEK-Blue cells were electroporated with a guide RNA (gRNA) targeting the CCR5 genomic locus in complex with Cas9 recombinant protein (rCas9) and a double-stranded DNA homology-directed repair (HDR) template containing the following elements: (i) left and right homology arms of 924 and 906 bp, respectively mapping to the CCR5 genomic locus; (ii) constitutive chicken beta-actin promoter; (iii) Cas9 encoding gene; (iv) nuclear localization signal; (v) T2A peptide; (vi) puromycin resistance gene; (vii) bGH promoter; (viii) a separate GFP-encoding transgene gene serving as a reporter of HDR (**Fig. 2**). FACS was performed to isolate cells based on GFP expression (**Fig. 2**) and a clonal cell line (i.e., Cas9+ GFP+ HEK-Blue) was derived. CRISPR-targeted knockout of the GFP transgene in the absence of exogenous Cas9 was then used to validate the successful integration, expression and endonuclease activity of transgenic Cas9 in this clone (**Fig. 3**). The resulting cell line expressing Cas9 and GFP was subjected to secondary CRISPR-Cas9 genome editing.

### Example 2: Introduction and characterization of a fluorescent reporter of IL-2 signalling

Cas9+ GFP+ HEK-Blue cells subjected to a second step of CRISPR-Cas9 genome editing in order to replace the SEAP secreted reporter for a fluorescence reporter protein. Accordingly, cells were electroporated with a gRNA targeting the SEAP transgene and an HDR template encoding the following elements: (i) N-terminal region of SEAP open reading frame (ORF); (ii) P2A self-cleaving peptide; (iii) mRuby2 gene; (iv) double stop codon; (v) C-terminal region of SEAP open reading frame (untranslated) **(****Fig. 4**). The resulting transfectants were stimulated with IL-2 and single-cells were isolated by FACS to derive a clone with high sensitivity to IL-2 stimulation (based on mRuby2 reporter expression). The resulting cell line expressing Cas9 and GFP, and harbouring a STAT5-mRuby2 reporter of IL-2 and IL-15 signalling is referred to as pre-GnR cells **(****Fig. 4**). Notably, pre-GnR cells supported high levels of HDR efficiency (30-40% depending on the HDR template, data not shown), a favourable feature for efficient integration of highly diverse GCR mutagenesis libraries in subsequent steps. Additionally, the correct integration of the mRuby2 gene was corroborated by a genomic PCR **(****Fig. 4**).

### Example 3: Disruption of endogenous GCR expression

A final CRISPR-Cas9 genome editing step was performed to facilitate high-throughput functional screens. To this end, the inventors generated a cell line in which the correct genomic integration of GCR mutagenesis libraries could be readily validated by means of a fluorescent readout. Accordingly, the inventors targeted the wild-type (endogenous) GCR for Cas9 NHEJ with the aim of generating a GCR-negative cell line **(****Fig. 5**). To achieve this, pre-GnR cells were electroporated with a GCR-targeting gRNA, expanded in culture, immunostained for GCR expression and subjected to single-cell FACS in order to derive a GCR-negative clone. The resulting cell line expressing Cas9, harbouring a STAT5-mRuby2 reporter of IL-2 signalling, and lacking GCR surface expression, represents the best characterised version of the GnR platform **(****Fig. 5**). This approach enabled the restoration of GCR surface expression upon genomic integration (by Cas9 HDR) of mutagenesis libraries, thereby providing a selectable marker detectable by GCR immunostaining and FACS.

### Example 4: CRISPR-targeted reconstitution of GnR cells with transgenic GCRs

In order to demonstrate the feasibility of GCR reconstitution in GnR cells, the inventors transfected them with an HDR template containing the wild-type GCR gene. The HDR template consisted of: (i) left and right homology arms of 804 and 837 base pairs respectively, mapping to the AAVS1 genomic site; (ii) CMV enhancer; (iii) CMV promoter; (iv) open reading frame of the GCR including a stop codon; and (v) SV40 poly(A) signal **(****Fig. 6**).

The HDR template was integrated into the genomic safe harbour AAVS1 to ensure the GCR expression only originated from the provided transgene, with homology arms targeting the safe harbour AAVS1 site. Following in vitro expansion, transfected cells were analysed by flow cytometry in order to assess GCR surface expression, which revealed successful GCR reconstitution with HDR efficiency of ~40% **(****Fig. 6**). In addition to wild-type GCR, the inventors further validated GCR reconstitution using six GCR mutants, which showed HDR efficiencies ranging from 14-28% **(****Fig. 7**).

### Example 5: Assessment of GnR platform functionality

Following CRISPR-targeted GCR reconstitution, the inventors assessed the activation of GnR-GCR transfectants (~40% GCR-positive, see **Fig. 6**) in the presence of IL-2. Overnight stimulation with 5 nM recombinant human IL-2-Fc led to robust production of mRuby2, with 23% of the transfectant pool (i.e., > 55% of GCR-positive cells) expressing the fluorescent reporter protein **(****Fig. 8**). Thus, our results validate the functionality and correct assembly of reconstituted transgenic GCR as a subunit of the IL-2R. The inventors further characterised the sensitivity of the STAT5-mRuby2 reporter following overnight stimulation in the presence of serially-diluted IL-2 or IL-15, which revealed a titratable mRuby2 response with EC50 values of 130 pM and 560 pM for IL-2 and IL-15, respectively **(****Fig. 8**).

### Example 6: Development of IL-7 and IL-21 responsive GnR cells

Because of the vital roles of both IL-7 and IL-21 in promoting a TSCM phenotype, the inventors set out to engineer derivatives of the GnR platform that could detect IL-7- or IL-21-induced signalling. In order to generate a cell line responsive to IL-7, the complete IL-7Rα subunit open reading frame was integrated into the GnR cells. The HDR template encoding the IL-7Rα ORF contained homology arms mapping to the GFP locus, as this marker had been introduced as a genomic integration marker of the Cas9 gene under a separate promoter and could thus be used as a 'landing pad' for the IL-7Rα transgene. The HDR template contained the following elements: (i) bGH poly(A) signal and PGK promoter, serving as the left homology arm; (ii) IL-7Rα ORF; (iii) right homology arm mapping to CCR5 locus **(****Fig. 9**). Following transfection and expansion in culture, transfected cells were immunostained for the IL7Rα subunit, revealing a 1.8% HDR efficiency **(****Fig. 9**). This was followed by single-cell FACS of IL-7Rα-high cells (also GFP-negative) in order to generate monoclonal cell lines **(****Fig.** 9). After expansion, individual clones were assessed for (i) GCR reconstitution efficiency and (ii) sensitivity to IL-7 stimulation. Accordingly, a wild-type GCR HDR template was generated and transfected into 10 different clonal cell lines for CRISPR-Cas9 genome editing targeted to the AAVS1 locus. After recovery, transfectants were immunostained for both IL-7Rα and GCR followed by separate stimulation with 10 nM IL-7 or 10 nM IL-2. Clone 5 was selected and established as the GnR-7 cell line based on its high rate of GCR reconstitution (41% HDR efficiency; **Fig. 10****)** as well as superior sensitivity to both IL-7 and IL-2 (49% mRuby2+ and 85%mRuby2+ of GCR-expressing cells, respectively) **(****Fig. 11**).

The generation of GnR-21 cells follows a similar path to that of GnR-7, i.e., using the GnR cell line as a starting point for CRISPR-targeted integration of the IL-21R ORF into the AAVS1 locus. An important difference to IL-2 and IL-7 induced signalling is that IL-21 induces preferential phosphorylation of STAT3 over STAT5. Thus, additional genome editing steps to integrate both a STAT3 transgene and a STAT3 response element may be required for the generation of GnR-21 cells.

### Example 7: Application of GCR-Engine for the identification of GCR variants with enhanced responsiveness to IL-2 through high-throughput functional screening

An important first step of GCR-Engine is the profiling of the GCR by deep mutational scanning (DMS), which is performed in order to both validate the GCR engineering workflow and to aid the design of combinatorial mutagenesis libraries that are enriched for productive GCR variants. For this purpose, the inventors first generated a tiled single-site saturation mutagenesis library (i.e., DMS library) in amino acid positions Arg 204 to His 214 of the GCR, a region in which several contacts between the GCR and IL-2/IL-15 occur **(****Fig. 12**). Since this is a single-site library, diversity is simply linear to the number of positions and possible amino acids (e.g., 11 positions × 20 a.a. = 220 GCR variants). The first goal of DMS was to identify the positions and single amino acid mutations that permit surface expression of GCR on GnR cells. The library was integrated into the safe harbour (AAVS1) site via CRISPR-Cas9 in the form of a double-stranded DNA HDR template following the layout illustrated in **Fig. 6**. Upon integration, FACS was applied to isolate the cells with reconstituted GCR surface expression (G+ selection), which were then expanded and subsequently stimulated overnight with 5 nM human IL-2. The following day, IL-2-responsive mRuby2+ cells were enriched via FACS (SIG+ selection) (**Fig. 13**). Both G+ and SIG+ fractions were subjected to genomic PCR amplification of the transgenic GCR locus, and the resulting amplicons deep sequenced (Illumina MiSeq v2 500-cycle kit). Computational analysis of deep sequencing data was performed to determine the read enrichment ratios of specific GCR variants present in G+ and SIG+ fractions relative to the original plasmid library (**Fig. 14**). Read enrichment data obtained from the SIG+ fraction identified two single mutations with predicted enhancement of IL-2-induced signalling, namely P207K and H214K. These mutations were combined to generate a GCR double mutant, which upon IL-2 stimulation displayed enhanced maximal signalling, thus validating our approach for functional GCR engineering (**Fig. 14**). In order to further expand the number of GCR variants that can be functionally screened for desired properties, the information obtained from DMS of GCR expressibility (i.e., G+ fraction) was used to computationally design combinatorial mutagenesis libraries spanning positions 204-214 of the GCR. (**Fig. 15**). To this end, the inventors applied a sequence space optimization algorithm (Mason et al., Nucleic Acids Research, 2018, 46(14), 7436-7449) to identify degenerate codons that mimic the GCR single mutant frequencies found in the G+ fraction. The output of this algorithm was three separate combinatorial libraries with a diversity of 109 each and that include the vast majority of expressible GCR substitutions (**Fig. 15**).

Following computational design, Individual combinatorial libraries were assembled by PCR and cloned into a plasmid encoding the GCR gene flanked by homology arms mapping to the AAVS1 locus. After cloning, double-stranded DNA HDR templates were generated by PCR for CRISPR-targeted reconstitution of GnR cells. For this purpose, 107 GnR cells were transfected with each one of the combinatorial libraries, expanded in culture, and combined in equal amounts to generate a pooled mixture of GnR transfectants. GnR transfectants were then subjected to multiple rounds of FACS to enrich for GCR surface expression. Before each enrichment round, the cells were expanded, and 4×10⁷ cells were immunostained with a fluorescently-labelled anti-CD132 (anti-GCR) antibody. In total, four rounds of FACS were performed to reach an enrichment level of ~90% GCR-positive cells, which the inventors termed 'Pool 4' (**Fig. 15**). Genomic DNA from this enriched population was extracted and the transgenic GCR locus was amplified by PCR and subjected to deep sequencing (Genewiz, US). HDR templates generated from each individual library were also PCR amplified and deep sequenced. Sequencing data was used to build amino acid sequence logo plots, which corroborated the full degeneracy in the region of interest **(****Fig. 15**).

Having obtained a pool of GnR cells (i.e., Pool 4) harbouring a combinatorial mutagenesis library enriched for GCR expression, the inventors next performed stimulation assays using varying concentrations of monomeric human IL-2. In the experiment, Pool 4 cells were stimulated with concentrations of IL-2 ranging from 0.001 nM to 100 nM for 24 hours. Next, the cells were scanned for mRuby2 expression. This experiment demonstrated that a subset of clones responded to the concentration of IL-2 as low as 0.01 nM **(****Fig. 16**). Therefore, this was the concentration chosen to stimulate Pool 4 in an attempt to isolate GCR clones with increased sensitivity to IL-2. Following FACS enrichment of 142,000 mRuby2-positive cells, cells were expanded in culture and re-stimulated to assess their sensitivity to IL-2. The inventors found that the sorted population (renamed to 'eGCR R1', for enhanced GCR Round 1) exhibited a clear enrichment for clones with increased sensitivity to not only 0.01 nM, but also to 0.001 nM of IL-2 **(****Fig. 17**). Sanger sequencing of genomic PCR amplicons revealed degeneracy in the GCR mutagenesis region, thus indicating the presence of non-wild-type GCR sequences with enhanced IL-2 responsiveness in the enriched eGCR R1 pool **(****Fig. 17**).

### Example 8: Application of GCR-Engine for high-throughput engineering and functional screening of GCR variants with tuned signalling output

It has been recently shown that attenuated induction of IL-2/GCR signalling favours the expansion of T cells with a TSCM phenotype, resulting in increased persistence and efficacy of engineered T cells in preclinical tumour models. An important application of GCR-Engine that harnesses its amenability for functional screening of the GCR is the modulation of signalling output in order to identify GCR variants that are fine-tuned to promote TSCM expansion. The starting point for this strategy is the wild-type GCR subunit, or preferably an engineered GCR variant with enhanced responsiveness to a γ_{c} cytokine, such as IL-2 (see Example 9). In order to attenuate GCR signalling, single-site saturation mutagenesis is applied to key regions within the intracellular domain of the GCR. For this purpose deep mutational scanning (DMS) libraries incorporating tiled NNK degenerate codons are designed, assembled by PCR and cloned into a plasmid containing the GCR gene flanked by homology arms mapping to the AAVS1 genomic locus. The key regions targeted by this approach comprise the Box 1 motif mediating interaction with the tyrosine kinase JAK3 (residues Arg 263 - Lys 272), as well as three additional regions containing tyrosine residues that are potential targets of JAK3 phosphorylation (residues Thr 279 - Trp 288, Leu 299 - Cys 322 and Ser 333 - Leu 343), representing 20 possible single amino acid substitutions across 40 positions for a library of 800 GCR variants. After construction, individual plasmid libraries are pooled in equimolar amounts, amplified by PCR for the generation of HDR templates, and transfected into GnR cells for CRISPR-targeted integration directed to the AAVS1 locus. Following transfection, GnR cells are expanded in culture and three rounds of FACS enrichment are applied in order to isolate GnR cells that are positive for GCR surface expression (i.e., expressible GCR variants). This enriched pool of GnR-GCR cells is then stimulated overnight with a range of IL-2 concentrations (0.001 nM - 100 nM) and once again subjected to FACS enrichment, this time based on detection of the mRuby2 fluorescent reporter. Genomic DNA is extracted from GnR-GCR cells originating from each FACS selection and subjected to PCR amplification for deep sequencing of the transgenic GCR locus (i.e., AAVS1 locus). Computational analysis is then applied to assess the enrichment of specific variants across selection steps. By using this strategy, GCR variants that are both expressible and have attenuated GCR signalling relative to the starting GCR can be identified computationally, with the most promising variants selected for experimental validation using primary human T cells.

Accordingly, primary human T cells are reconstituted with selected GCR variants by means of CRISPR-Cas9 genome editing targeted to the first exon of the endogenous GCR gene (X chromosome). In order to facilitate selection by FACS, GCR transgenic constructs incorporate a C-terminal GFP reporter gene preceded by a 2A self-processing peptide. An example workflow for primary T cell phenotyping consists of the following steps: (i) primary human T cells are isolated from PBMC by immunomagnetic enrichment and activated by stimulation with CD3/CD28 magnetic beads that mimic antigen-induced T cell stimulation; (ii) T cells are expanded for three days in the presence of IL-7 and IL-15 in order to minimise T cell exhaustion; (iii) three days post-activation, T cells are edited by CRISPR-Cas9 to introduce candidate engineered GCR transgenes containing a GFP reporter; (iv) seven days post activation, edited primary human T cells are isolated by FACS based on GFP expression; (v) primary human T cells are expanded for an additional 7 days, this time in the presence of the γ_{c} cytokine of interest (e.g., IL-2); (vi) the transcriptomes of primary human T cells expressing GCR variants and the starting GCR (e.g wild-type GCR or highly responsive GCR) are profiled by single-cell sequencing using the 10x Genomics scSeq protocol (Bieberich et al., Front. Immunol., 2021, 12, 701085); (vii) In addition to single-cell transcriptomics, the levels of selected surface proteins, namely CD3, CD8, CD45RO, CCR7, CD45RA, CD62L, CD27, CD28, IL7Rα, and CD95 (Fas) is assessed by means of flow cytometry or CITE-seq (Stoeckius et al., Nat. Methods, 2017, 14, 865-868) in order to determine the proportions of TSCM cells in each sample.

Following the completion of this workflow, computational analysis of single-cell sequencing and flow cytometry data is performed to identify selected GCR variants driving preferential expansion of T cells with stem-like phenotypes. As a final validation step, 1-2 lead GCR variants are integrated alongside a tumour targeting receptor (i.e., a CAR or TCR) by means of CRISPR-Cas9 or lentiviral transduction in order to generate a prototype engineered T cell product, which is used to validate enhanced sensitivity to the γ_{c} cytokine of interest, increased proportions of TSCM cells following manufacturing, and increased efficacy in cell-derived xenograft (CDX) tumour models in immunodeficient NOG mice expressing the human γ_{c} cytokine of interest (Taconic, US).

### Example 9: Application of GCR-Engine for the identification of GCR variants with selective response to common gamma chain cytokines

An important application of GCR-Engine is to engineer GCR variants that display enhanced selectivity. In this context, the engineering of selective GCR variants that (i) promote the development of long-lived TSCM cells (i.e., enhanced T cell persistence) or (ii) respond to inactivated cytokine mutants (i.e., orthogonal cytokine receptors for enhanced safety) are of particular interest. For this purpose, the GCR-Engine workflow is applied to generate GCR mutagenesis libraries targeting the GCR region encompassing residues Arg 204 to His 214 as described in Example 1. Additional regions of interest for targeted mutagenesis are those centred around GCR residues Tyr 103 and Cys 160, which have been highlighted as important contacts to multiple γ_{c} cytokines. After library generation and CRISPR-targeted GCR reconstitution, GnR cells (or any of its derivatives) are enriched for GCR surface expression through multiple rounds of FACS. Enriched pools with high proportions (> 90%) of GCR-positive cells are then used as a starting point for functional screening in response to individual cytokines or cytokine mutants. Examples of functional selections include those following stimulation with wild-type IL-2, IL-7, IL-15 or IL-21, or with inactivated cytokine mutants. If using inactivated IL-2 mutants, inactivating mutations can include but are not limited to those targeting IL-2 residues mediating contacts with IL-2Rα (Arg 38, Lys 43, Glu 61), IL-2Rβ (Asn 88, Val 91) and/or γ_{c} (Leu 18, Gln 22, Thr 123, Gln 126, Ser 127, Ser 130) either as single or as multiple amino acid substitutions.

Functional screening after stimulation with said soluble cytokines is performed on FACS-enriched GnR-GCR cells, in a similar manner to that described in Examples 1 and 2. An important difference to previous examples is that two-way FACS is applied in order to enrich for both mRuby2-positive (i.e., SIG+) and mRuby-negative (i.e., SIG-) fractions. Deep sequencing of the transgenic GCR locus (i.e., the AAVS1 genomic locus) is applied after every selection step in order to identify GCR variants with predicted enhanced responsiveness to each individual cytokine (or cytokine mutant) by computational analysis of read frequency enrichment. Computational analysis is also performed to identify GCR variants that are predicted to have enhanced responsiveness to a particular cytokine (e.g., IL-7 SIG+ fraction) but that are depleted during functional screening with a different cytokine (e.g., IL-2 SIG-fraction); or of GCR variants that are predicted to have enhanced responsiveness to two or more individual cytokines (e.g., IL-7 SIG+ fraction and IL-15 SIG+ fraction). In order to expand the limits of experimental GCR screening, sequencing data from SIG+ and SIG- fractions is encoded (e.g., one-hot encoding) and used to train supervised machine learning models (e.g. SVM, Random Forest, neural networks) that can perform classification for each individual cytokine, thus enabling functional prediction of GCR variants in silico. For example, fully trained machine learning models will be able to predict based on the protein sequence of a GCR, its classification towards activation of a given cytokine. In this context, in silico libraries of GCR variants such as the ones described in Example 8 (diversity ~ 1 × 10⁹) are used as input for each of these machine learning models (in a sequential or multi-classification manner), so that candidate GCR variants with desired functional properties are identified. Examples of desirable GCR variants resulting from this application of GCR-Engine include but are not limited to:
a. GCR variants with enhanced responsiveness to both IL-7 and IL-15;
b. GCR variants with enhanced responsiveness to IL-15 but reduced response to IL-2;
c. GCR variants with enhanced responsiveness to IL-7 but reduced response to IL-2;
d. GCR variants with enhanced responsiveness to IL-21 but reduced response to IL-2;
e. GCR variants with restored response to an inactivated IL-2 mutant but reduced response to wild-type IL-2 (i.e., an orthogonal cytokine-cytokine receptor pair)

### Materials and methods

### Cell Culture

GnR and GnR-7 were cultured in DMEM medium (ATCC, #30-2002) with 10 % FBS (gibco, #16000-044) and 1 % Penicillin-Streptomycin (Gibco, #15140-122). For prolonged storage, cells were frozen in FBS (Gibco, #10270106) supplemented with DMSO (final concentration 10%) and stored in liquid nitrogen.

### CRISPR-Cas9 Genome Editing

HDR templates were generated by PCR amplification of GCR cassettes flanked by AAVS1 safe harbour site homology arms. Cell transfection was performed using electroporation (SF Cell Line Solution Box, Lonza, #V4XC-2024) with the "HEK-293" protocol on the 4D-Nucleofector (Lonza). For genome editing, 200 µM tracrRNA and 200 µM crRNA were mixed at equimolar concentration to form the gRNA complex. For HDR, 1 µg PCR-amplified DNA constructs and 8 µL gRNA were used per transfection. After one week, HDR efficiency was assessed by flow cytometry. Transfections were either bulk or single cell sorted, expanded and characterised by flow cytometry, PCR, Sanger or next-generation sequencing.

### IL-2 stimulation assay

GnR cells expressing GCR libraries were stimulated for 24 hours at a density of 5 × 10⁵ / mL with either 5nM or serially diluted (0.001-100nM) monomeric human IL-2 (R&D Systems, #202-IL-050). The cells were washed with ice-cold FACS buffer (PBS supplemented with 2% FBS) and resuspended in 180 uL FACS buffer before scanning for mRuby2 expression by flow cytometry.

### Molecular cloning of GCR gene constructs

For GCR cloning, GCR inserts and pMT2-PL vector containing AAVS1 safe harbour homology arms and the wild-type GCR open reading frame under control of the CMV promoter were digested with Ncol and SaIl for 1 h at 37°C. Vector 5' ends were dephosphorylated by addition of 1 µL calf intestinal phosphatase (CIP) for a further 30 min. The digested vector and inserts were gel-purified and (ZymoClean Gel DNA Recovery Kit, Zymo Research, #D4001) and ligated using T4 ligase (NEB, #M0202F) at a molar ratio 3:1 insert-to-vector for 2h at RT. Ligation mixes were transformed into NEB5α bacteria (NEB, #C2988J) according to the manufacturer's instructions. Positive bacterial clones were selected by colony PCR using the Q5^{®} High-Fidelity 2X Master Mix (NEB, #M0492S)

### Generation of deep mutational scanning (DMS) libraries

For library construction, ssDNA oligonucleotides containing a 20 nt complementary overlap were designed and purchased as (i) a pool of plus strand ssDNA containing tiled NNK degenerate codons at GCR positions 204 to 214 (IDT oPool), and (ii) a minus strand ssDNA strand containing wild-type GCR codons (IDT ultramer). 200 pmol of each oPool and ultramer were mixed and subjected to PCR amplification in the presence of external primers using the following conditions: 95°C for 3 min, 98°C for 20 s, 70°C for 15 s, 72°C for 10 min. The resulting 270 bp dsDNA product was gel-purified (Zymogen, #D4002) and cloned into the pMT2-PL plasmid as described above. The ligation products were dialysed in nuclease-free water for 30 minutes using 0.025 µm mixed cellulose esters (MCE) membrane filter discs (MF-Millipore Merck). 100 µL of the dialysed DNA was transformed into the electrocompetent ElectroMAX DH10B T1 Phage-Resistant Competent Cells (Invitrogen, #12033015) cells according to the manufacturer's instructions. Each transformation mix was plated on ampicillin LB agar in Square Bioassay dishes (Corning, #431111). 1/100 and 1/1000 dilutions of the transformations were also prepared and plated on ampicillin plates to determine the number of transformants the following day. Colonies from the Square Bioassay plates were collected by adding 15 mL of LB media to each plate and resuspending. Colonies from each library were pooled together in a single tube and centrifuged at 4817 g for 20 min. The supernatant was discarded and the plasmid DNA was isolated following the Maxiprep protocol (QIAGEN, # 12162).

### Screening of DMS libraries

DMS library HDR templates and AAVS1 gRNA were used to transfect 10⁶ GnR cells. The cells with recovered GCR surface expression were enriched by FACS (G+ library) and recovered for 7 days. Subsequently, the G+ cells were stimulated with human IL-2 at 5 nM for 24h. Following this, the stimulated (mRuby2+) cells were enriched via FACS (SIG+ library). Both G+ and SIG+ fractions were subjected to genomic PCR of the transgenic GCR locus and the resulting amplicons were deep sequenced. Next, sequencing read enrichment ratios were calculated for G+ and SIG+ fractions relative to the original plasmid library.

### Design of combinatorial mutagenesis libraries

Degenerate codons reflecting the combined frequencies of amino acids in GCR residues 204 - 214 observed in the G+ DMS selection were determined as previously described (Mason et al., Nucleic Acids Research, 2018, 46(14), 7436-7449). Residues identified to have an expression enrichment equal or greater than 1.2 in the DMS experiment were normalised according to their enrichment ratios, converted to theoretical frequencies and taken as the target amino acid frequencies. For each position, degenerate codons approximating the corresponding amino acid frequency distribution and diversity were selected. For library construction, ssDNA oligonucleotides containing a 20 nt complementary overlap were designed and purchased as custom ultramers. The reverse ultramer encoded exclusively wild-type GCR codons, while the forward ultramer contained both wild-type and library degenerate codons. Furthermore, the ultramers contained 20 bp overlaps (at their 5' ends) to the pMT2-PL-GCR vector linearized with SaIl and Ncol restriction enzymes. A splice by overlap extension (SOE)-PCR strategy was designed to bridge the gap in the GCR gene created by the restriction digestion, amplify the whole vector and allow it to self-ligate (which happens spontaneously). For each library, a 500 µL reaction was performed: 250 µL of Q5^{®} High-Fidelity 2X Master Mix were added together with 25 µL of the forward and reverse ultramers (both at 1 µM), 300 ng of plasmid DNA and 597.7 µL of nuclease-free water. PCR products of correct size (i.e. 5899 kb) were gel-purified and dialysed in nuclease-free water for 30 minutes using 0.025 µm mixed cellulose esters (MCE) membrane filter discs (MF-Millipore Merck). 100 µL of the dialysed DNA was transformed into the electrocompetent ElectroMAX DH10B T1 Phage-Resistant Competent Cells (Invitrogen, #12033015) cells according to the manufacturer's instructions. Quantification of bacterial transformants, purification of plasmid library and generation of HDR templates was performed as described for DMS libraries.

### Screening of combinatorial mutagenesis libraries

Combinatorial library HDR templates (10 µg) and AAVS1 gRNA (10 nmol) were used to transfect 10⁷ GnR or GnR-7 cells using the 4D-Nucleofector LV unit (Lonza, #AAF-1002L). GnR/GnR-7 cells with restored GCR surface expression were bulk-sorted (enrichment 1) on day 7 post transfection. Enrichment 1 cells were expanded and resorted for GCR surface expression in three more rounds of enrichment to obtain the GCR surface expression level ≈ 90% (Pool 4). 1.4×10⁷ of Pool 4 cells were next cultured in 96-well plates at a density of 7.5×10⁴ cells per well in the presence of IL-2 at 0.001 nM. After 24 h stimulation, the cells were pooled and sorted for mRuby2 expression (ROUND 1) by FACS. Next, the cells were recovered for 7 days, restimulated with 0.001 nM IL-2 and another round of sorting for the low concentration IL-2 sensitive variants was performed (ROUND 2). The genomic DNA from bulk-sorted ROUND 1 and ROUND 2 cells was extracted and amplified via PCR and the amplicons were deep sequenced.

### crRNA sequences

CRISPR-RNA (crRNA) reagents targeting the CCR5 (5'-TGACATCAATTATTATACAT-3'; SEQ ID NO:14), eGFP (5'-CAACTACAAGACCCGCGCCG-3'; SEQ ID NO:15), AAVS1 (5'-GGGGCCACTAGGGACAGGAT-3'; SEQ ID NO:16) loci were purchased from IDT. The sequences above reflect the DNA template used to design the crRNA constructs.

### Genotyping of GnR-GCR clones

Genomic DNA was extracted using the QuickExtract solution kit (Lucigen, #0905T). Genomic and cDNA PCRs were performed using the Q5^{®} High-Fidelity 2X Master Mix (NEB, #M0492S).

### Flow Cytometry and Cell Sorting

For flow cytometric analysis of surface antigens, cells were washed with FACS buffer and stained with appropriate fluorophore-conjugated antibodies (all BioLegend: CD132-PE (#338606), CD122-BV421 (#339010), CD25-PE (#356103), CD127-APC/Cy7 (#351348), for 20 min at 4°C. Cells were washed twice in the FACS buffer, and fluorescence was measured using the Cytoflex S (Beckman Coulter) flow cytometer. FACS was performed either on the Aria III instrument (BD Biosciences) or FACSMelody (BD Bioscience).

## Claims

1. A cell line for identifying engineered common gamma chain (γ_{c}) receptors, the cell line comprising:
a) a reporter system comprising a polynucleotide encoding a fluorescent marker, wherein the polynucleotide encoding the fluorescent marker is transcriptionally linked to one or more STAT response elements;
b) at least one polynucleotide encoding a STAT protein, wherein the STAT protein activates the STAT response element comprised in the reporter system; and
c) at least one polynucleotide encoding a JAK protein;
wherein said cell line does not encode a functional common gamma chain.

2. The cell line according to claim 1, wherein the cell line further comprises at least one cytokine receptor subunit, wherein the at least one cytokine receptor subunit is selected from the group consisting of: IL2Rα, IL2Rβ, IL4Rα, IL7Rα, IL9Rα, IL15Rα and IL21Rα.

3. The cell line according to claim 1 or 2, wherein the polynucleotide encoding the fluorescent marker is transcriptionally linked to at least 2, 3, 4, or 5 STAT response elements.

4. The cell line according to any one of claims 1 to 3, wherein the cell line further comprises a polynucleotide encoding a sequence-specific endonuclease, in particular wherein the sequence-specific endonuclease is a CRISPR-associated (Cas) protein, in particular wherein the CRISPR-associated (Cas) protein is Cas9.

5. The cell line according to any one of claims 1 to 4, wherein the cell line is a mammalian cell line, in particular wherein the mammalian cell line is a human cell line, in particular wherein the human cell line is derived from HEK-293.

6. A method for obtaining an engineered common gamma chain (γ_{c}), the method comprising the steps of:
a) introducing a plurality of polynucleotides encoding common gamma chain (γ_{c}) variants into the cell line according to any one of claims 1 to 5;
b) contacting the cells obtained in step (a) with a cytokine;
c) isolating cell(s) expressing a polynucleotide encoding an engineered common gamma chain (γ_{c}) variant based on the expression level of the polynucleotide encoding the fluorescent marker; and
d) obtaining the engineered common gamma chain (γ_{c}) variant encoded by the cell(s) isolated in step (c);
preferably wherein obtaining the engineered common gamma chain (γ_{c}) variant comprises the steps of:
i) sequencing the polynucleotides encoding the engineered common gamma chain (γ_{c}) variants of the cells isolated in step (c); and
ii) identifying a nucleotide sequence encoding an engineered common gamma chain (γ_{c}) variant based on the sequencing results obtained in step (i) and/or identifying one or more mutations in an engineered common gamma chain (γ_{c}) variant based on the sequencing results obtained in step (i);
in particular wherein the nucleotide sequence encoding the engineered common gamma chain (γ_{c}) variant and/or the one or more mutations in an engineered common gamma chain (γ_{c}) variant is/are identified with a classification algorithm.

7. A method for obtaining an engineered common gamma chain (γ_{c}) having reduced or enhanced cytokine responsiveness, the method comprising the steps of:
a) introducing a polynucleotide encoding a common gamma chain (γ_{c}) variant into a first cell of a cell line according to any one of claims 1 to 5; and introducing a polynucleotide encoding a reference common gamma chain (γ_{c}) into a second cell of a cell line according to any one of claims 1 to 5;
b) contacting the cells obtained in step (a) with a cytokine;
c) identifying the common gamma chain (γ_{c}) variant as an engineered common gamma chain (γ_{c}) variant having enhanced responsiveness to the cytokine, if the expression of the polynucleotide encoding the fluorescent marker is higher in the first cell encoding said common gamma chain (γ_{c}) variant compared to the second cell encoding the reference common gamma chain (γ_{c}); or
identifying the common gamma chain (γ_{c}) variant as having reduced responsiveness to the cytokine, if the expression of the polynucleotide encoding the fluorescent marker is lower in the first cell encoding said common gamma chain (γ_{c}) variant compared to the second cell encoding the reference common gamma chain (γ_{c}); and
d) obtaining an engineered common gamma chain (γ_{c}) having reduced or enhanced cytokine responsiveness based on the identification in step (c).

8. A method for obtaining an engineered common gamma chain (γ_{c}) for selective cytokine activation, the method comprising the steps of:
a) introducing a plurality of polynucleotides encoding common gamma chain (γ_{c}) variants into a plurality of cells according to any one of claim 1 to 5;
b) contacting the cells obtained in step (a) with a first cytokine;
c) isolating cells based on the expression of the polynucleotide encoding the fluorescent marker in response to the first cytokine;
d) contacting the cells isolated in step (c) with a second cytokine;
e) isolating cells based on the expression of the polynucleotide encoding the fluorescent marker in response to the second cytokine; and
f) obtaining an engineered common gamma chain (γ_{c}) for selective cytokine activation from the cells isolated in step (e).

9. A method for obtaining a chimeric common gamma chain (γ_{c}) receptor comprising a cytoplasmic domain of a common gamma chain (γ_{c}) receptor, in particular wherein the polynucleotides encoding the chimeric common gamma chain (γ_{c}) receptor candidates encode amino acid residues Glu 262 to Thr 347 of SEQ ID NO:1, and an extracellular domain of a receptor protein, the method comprising the steps of:
a) introducing a plurality of polynucleotides encoding chimeric common gamma chain (γ_{c}) receptor candidates into a plurality of cells according to any one of claims 1 to 5;
b) contacting the cells obtained in step (a) with a ligand of the receptor protein;
c) isolating cells expressing the polynucleotide encoding the fluorescent marker in response to the ligand of the receptor protein; and
d) obtaining a chimeric common gamma chain (γ_{c}) receptor from the cells isolated in the (c).

10. The method according to any one of claim 6 to 9, wherein obtaining the engineered or chimeric common gamma chain (γ_{c}) comprises a step of isolating cells by fluorescence-activated cell sorting (FACS) based on the expression of the polynucleotide encoding the fluorescent marker.

11. The method according to claim 10, wherein the nucleotide sequence encoding the engineered or chimeric common gamma chain (γ_{c}) is identified by sequencing, in particular by deep sequencing.

12. The method according to claim 11, wherein a classification algorithm is used to identify mutations that contribute to the phenotype of the engineered or chimeric common gamma chain (γ_{c}).

13. The method according to any one of claims 5 to 12, wherein the polynucleotide encoding the engineered or chimeric common gamma chain (γ_{c}) is comprised in a library of polynucleotides encoding common gamma chain (γ_{c}) variants or chimeric common gamma chain (γ_{c}) receptor candidates, in particular wherein the library has been obtained by site-directed mutagenesis, in particular wherein
i) at least one amino residue in position His66 to His214 of SEQ ID NO:1 has been subjected to site directed mutagenesis, in particular wherein at least one amino acid residue in position Tyr103 to Cys160 and/or Arg204 to His214 of SEQ ID NO:1 has been subjected to site directed mutagenesis; and/or
ii) at least one amino residue in position Arg263 to Lys272, Thr279 to Trp288, Leu299 to Cys322 and/or Ser333 to Leu343 of SEQ ID NO:1 has been subjected to site directed mutagenesis.

14. The method according to any one of claims 5 to 13, wherein the polynucleotide encoding the engineered or chimeric common gamma chain (γ_{c}) is integrated into the genome of the first and second cell, respectively, by means of genome editing, in particular wherein genome editing involves a sequence-specific endonuclease, in particular wherein the sequence-specific endonuclease is a CRISPR-associated (Cas) protein, in particular wherein the CRISPR-associated (Cas) protein is Cas9.

15. The method according to any one of claims 5 to 14, wherein the cytokine is IL-2, IL-4, IL-7, IL-9, IL-15 or IL-21, or a variant thereof.
